# EUROPEAN PATENT APPLICATION

(11) **EP 4 681 721 A1**
(43) Date of publication of application: **21.01.2026**
(21) Application number: 24774897.3
(22) Date of filing: 18.03.2024
(51) Int. Cl.: A61K 35/763, A61K 38/20, A61P 35/00, A61P 37/04, C12N 7/01, C12N 15/12, C12N 15/24, C12N 15/62, C12N 15/869

(54) **IL-12-EXPRESSING TRANSGENIC HERPES SIMPLEX VIRUS**

(30) Priority: 17.03.2023 JP 2023043594
(71) Applicant: Todo, Tomoki, Koto-ku Tokyo 135-0034 (JP)
(72) Inventor: FUKUHARA, Hiroshi, Mitaka-shi, Tokyo 181-8611 (JP); TODO, Tomoki, Tokyo 135-0034 (JP)
(74) Representative: Hoffmann Eitle
(86) International application number: PCT/JP2024/010478
(87) International publication number: WO 2024/195761

(57) **Abstract**

An object of the present invention is to develop a novel armed oncolytic herpes simplex virus (HSV) using G47Δ as the backbone. There is provided a pharmaceutical composition comprising a recombinant herpes simplex virus (HSV) with interleukin 12 (IL-12) expression, the recombinant HSV having a gene encoding a fusion polypeptide in which the 35 kDa light chain (p35) and the 40 kDa heavy chain (p40) of IL-12 are linked via two or more elastin motifs, and having the following characteristics (a) to (c):
(a) the ICP6 gene is deleted or inactivated; (b) the γ34.5 gene is deleted or inactivated; and (c) the α47 gene is deleted or inactivated.

## Description

### Technical Field

The present invention relates to an oncolytic herpes virus. More specifically, the present invention relates to a function-added oncolytic herpes virus that enhances therapeutic effect of viral therapy.

### Background Art

Oncolytic herpes simplex virus type 1 (HSV-1) is an effective tool for the treatment of cancer (Non-patent documents 1 and 2). The first oncolytic HSV-1 having a deletion in the thymidine kinase gene was reported in 1991 (Non-patent document 3). Since then, various oncolytic HSV-1s have been validated in clinical trials. T-VEC (Talimogene laherparepvec), an oncolytic HSV-1 with mutations in the γ34.5 and α47 genes, was approved as a drug for inoperable melanoma in the United States and Europe in 2015 (Non-patent document 4). G207, one of the oncolytic HSV-1 viruses first used in clinical trials, has deletions in both copies of the γ34.5 gene and a lacZ insertion that inactivates the ICP6 gene (Non-patent document 5). G47Δ was constructed by further introducing a deletion into the US11 promoter overlapping with the α47 gene of the G207 genome (Non-patent document 6, and Patent document 1). The efficacies of G47Δ against a variety of cancers have been demonstrated (Non-patent documents 7 to 16). Safety of G47Δ has been verified in multiple clinical trials conducted in Japan, including those for glioblastoma (UMIN000002661, Non-patent document 17), castration-resistant prostate cancer (UMIN 000010463), metastatic prostate cancer (jRCTs033210603), olfactory neuroblastoma (UMIN000011636, jRCTs03180325), and malignant pleural mesothelioma (UMIN000034063, jRCTs03180326). Recently, a phase II clinical trial for glioblastoma (UMIN000015995) led to the approval of G47Δ as a new drug for malignant glioma in Japan (Non-patent document 18).

As the next generation of oncolytic HSV-1, function-added G47Δ incorporating various exogenous genes into the basic backbone of G47Δ, for example, a vascular endothelial growth factor (VEGF)-expressing and swelling-suppressive oncolytic virus incorporating a gene encoding a VEGF antagonist (Patent document 2), is under development.

By the way, interleukin-12 (IL-12) is a promising tool for cancer therapy, and several types of mouse IL-12 have been confirmed to be expressed by oncolytic HSV-1 (Non-patent documents 19 to 22). In general, tumor formation and growth are highly dependent on the host's inability to elicit strong anti-tumor immune responses and formation of new blood vessels to feed the tumor. IL-12 can target these two processes. IL-12 is a cytokine that activates a variety of immune cells, including NK cells and T cells. In fact, IFNγ is induced and secreted by these cells.

Functional IL-12, p70, is a heterodimeric glycoprotein composed of two subunits, a 35 kDa light chain (p35) and a 40 kDa heavy chain (p40) (Non-patent documents 23 and 24). The p70-IL-12Rβ1 complex binds to another second p70, and the resulting complex preforms a binding site with high affinity for IL-12Rβ2. The interaction between p40 and IL-12Rβ1 is required to stabilize the p70-IL-12Rβ1 complex (Non-patent document 25). The production of biologically active IL-12 requires simultaneous transfection of genes for these two subunits into mammalian cells (Non-patent documents 26 and 27).

### Prior Art References

### Patent documents

Patent document 1: Japanese Patent No. 4212897
Patent document 2: WO2019/189643

### Non-patent documents

Non-patent document 1: Kirn, D., Martuza, R.L. & Zwiebel, J. Replication-selective virotherapy for cancer: Biological principles, risk management and future directions. Nat. Med. 7, 781-787 (2001).
Non-patent document 2: Fukuhara, H., Ino, Y. & Todo, T. Oncolytic virus therapy: A new era of cancer treatment at dawn. Cancer Sci. 107, 1373-1379 (2016).
Non-patent document 3: Martuza, R.L., Malick, A., Markert, J.M., Ruffner, K.L. & Coen, D.M. Experimental therapy of human glioma by means of a genetically engineered virus mutant. Science. 252, 854-856 (1991).
Non-patent document 4: Coffin R. Interview with Robert Coffin, inventor of T-VEC: the first oncolytic immunotherapy approved for the treatment of cancer. Immunotherapy. 8, 103-106 (2016).
Non-patent document 5: Mineta, T., Rabkin, S.D., Yazaki, T., Hunter, W.D. & Martuza, R.L. Attenuated multi-mutated herpes simplex virus-1 for the treatment of malignant gliomas. Nat. Med. 1, 938-943 (1995).
Non-patent document 6: Todo, T., Martuza, R.L., Rabkin, S.D. & Johnson, P.A. Oncolytic herpes simplex virus vector with enhanced MHC class I presentation and tumor cell killing. Proc. Natl. Acad. Sci. U S A. 98, 6396-6401 (2001).
Non-patent document 7: Sugawara, K., et al. Efficacy of a third-generation oncolytic herpes virus G47Δ in advanced stage models of human gastric cancer. Mol. Ther. Oncolytics. 17, 205-215 (2020).
Non-patent document 8: Yamada, T., Tateishi, R., Iwai, M., Koike, K. & Todo, T. Neoadjuvant use of oncolytic herpes virus G47Δ enhances the antitumor efficacy of radiofrequency ablation. Mol. Ther. Oncolytics. 21, 612-623 (2020).
Non-patent document 9: Fukuhara, H., Martuza, R.L., Rabkin, S.D., Ito, Y. & Todo, T. Oncolytic herpes simplex virus vector G47delta in combination with androgen ablation for the treatment of human prostate adenocarcinoma. Clin. Cancer Res. 11, 7886-7890 (2005).
Non-patent document 10: Zeng, W.G., et al. An oncolytic herpes simplex virus vector, G47Δ, synergizes with paclitaxel in the treatment of breast cancer. Oncol. Rep. 29, 2355-2361 (2013).
Non-patent document 11: Liu, T.C., Castelo-Branco, P., Rabkin, S.D. & Martuza, R.L. Trichostatin A and oncolytic HSV combination therapy shows enhanced antitumoral and antiangiogenic effects. Mol. Ther. 16, 1041-1047 (2008).
Non-patent document 12: Liu, T.C., et al. Oncolytic HSV armed with platelet factor 4, an antiangiogenic agent, shows enhanced efficacy. Mol. Ther. 14, 789-797 (2006).
Non-patent document 13: Liu, T.C., et al. Dominant-negative fibroblast growth factor receptor expression enhances antitumoral potency of oncolytic herpes simplex virus in neural tumors. Clin. Cancer. Res. 12, 6791-6799 (2006).
Non-patent document 14: Uchihashi, T., et al. Oncolytic herpes virus G47Δ injected into tongue cancer swiftly traffics in lymphatics and suppresses metastasis. Mol. Ther. Oncolytics. 22, 388-389 (2021).
Non-patent document 15: Sugawara, K., et al. Oncolytic herpes virus G47Δ works synergistically with CTLA-4 inhibition through dynamic intratumoral immune modulation. Mol. Ther. Oncolytics. 22, 129-142 (2021).
Non-patent document 16: Yajima, S., et al. Efficacy and safety of a third-generation oncolytic herpes virus G47Δ in models of human esophageal carcinoma. Mol. Ther. Oncolytics. 23, 402-411 (2021).
Non-patent document 17: Todo, T., et al. A phase I/II clinical trial of triple-mutated oncolytic herpes virus G47Δ in patients with progressive glioblastoma Nat. Commun. 13: 4119 (2022).
Non-patent document 18: Todo, T., et al. Intratumoral oncolytic herpes virus G47Δ for residual or recurrent glioblastoma: a phase 2 trial. Nat. Med. 28: 1630-1639 (2022).
Non-patent document 19: Wong, R.J., et al. Cytokine gene transfer enhances herpes oncolytic therapy in murine squamous cell carcinoma. Hum. Gene Ther. 12, 253-265 (2001).
Non-patent document 20: Ino, Y., Saeki, Y., Fukuhara, H. & Todo, T. Triple combination of oncolytic herpes simplex virus-1 vectors armed with interleukin-12, interleukin-18, or soluble B7-1 results in enhanced antitumor efficacy. Clin. Cancer Res. 12, 643-652 (2006).
Non-patent document 21: Cheema, T.A., et al. Multifaceted oncolytic virus therapy for glioblastoma in an immunocompetent cancer stem cell model. Natl. Acad. Sci. U S A. 110, 12006-12011 (2013).
Non-patent document 22: Parker, J.N., et al. Engineered herpes simplex virus expressing IL-12 in the treatment of experimental murine brain tumors. Proc. Natl. Acad. Sci. U S A. 97, 2208-2213 (2000).
Non-patent document 23: Gubler, U., et al. Coexpression of two distinct genes is required to generate secreted bioactive cytotoxic lymphocyte maturation factor. Proc. Natl. Acad. Sci. U S A. 88, 4143-4147 (1991).
Non-patent document 24: Wolf, S.F., et al. Cloning of cDNA for natural killer cell stimulatory factor, a heterodimeric cytokine with multiple biologic effects on T and natural killer cells. J. Immunol. 146, 3074-3081 (1991).
Non-patent document 25: Yoon, C., et al. Charged residues dominate a unique interlocking topography in the heterodimeric cytokine interleukin-12. EMBO J. 19, 3530-3541 (2000).
Non-patent document 26: Kobayashi, M., et al. Identification and purification of natural killer cell stimulatory factor (NKSF), a cytokine with multiple biologic effects on human lymphocytes. J. Exp. Med. 170, 827-845 (1989).
Non-patent document 27: Stern, A.S., et al. Purification to homogeneity and partial characterization of cytotoxic lymphocyte maturation factor from human B-lymphoblastoid cells. Proc. Natl. Acad. Sci. U S A. 87, 6808-6812 (1990).
Non-patent document 28: D'Andrea, A., et al. Production of natural killer cell stimulatory factor (interleukin 12) by peripheral blood mononuclear cells. J. Exp. Med. 176, 1387-1398 (1992).
Non-patent document 29: Mattner, F., et al. The IL-12 subunit p40 specifically inhibits effects of the IL-12 heterodimer. Eur. J. Immunol. 23, 2202-2208 (1993).
Non-patent document 30: York, I.A., et al. A cytosolic herpes simplex virus protein inhibits antigen presentation to CD8+ T lymphocytes. Cell. 77, 525-535 (1994).

### Summary of the Invention

### Object to be achieved by the invention

An object of the present invention is to develop a novel armed oncolytic herpes simplex virus (HSV) based on G47Δ as the backbone. Since HSV elicits specific anti-tumor immunity, it can be expected to make IL-12 function as an effective payload and enhance the efficacy thereof.

### Means for achieving the object

The inventors of the present invention prepared the following two types of mouse IL-12 expression cassettes using a very early cytomegalovirus (CMV) promoter:
1) a fusion peptide-expressing type, in which the p35 and p40 subunit genes are linked as one gene in the same reading frame, and which expresses a fusion peptide comprising p40 and p35, and
2) a co-expression type, in which the p35 and p40 subunit genes are separated by an internal ribosome entry site (IRES) sequence, resulting in co-expression of two separate proteins, the p40 and p35 subunits. In this type, the p40 gene was placed close to the promoter because the p35 subunit is considered not to be secreted without the presence of the p40 subunit, although transcripts of the p40 subunit are present in many cell species (Non-patent document 28).

For a fusion protein of two subunits, the key requirement is that the expressed molecule maintains a functional tertiary structure. Therefore, it was devised that two bovine elastin motifs were inserted between the p35 and p40 genes so that the two subunits are folded and interact with each other. On the other hand, IRES ensures that the two subunits expressed separately form a molecule with the function of IL-12 intact, while maintaining the tertiary structure. However, IRES may lead to heterogeneous expression of the two subunit genes and overexpression of the p40 gene close to the promoter. Excess free p40 subunits may inhibit the IL-12 activity in mouse models (Non-patent document 29).

The α47 gene product binds to a transporter associated with antigen presentation (TAP) and downregulates the expression of major histocompatibility complex (MHC) class I molecules, thereby evading detection of infected cells by host immune surveillance (Non-patent document 30). G47Δ, an α47-deficient HSV-1, prevents the downregulation of MHC class I, providing enhanced immunostimulatory ability (Non-patent document 6). Therefore, it is suitable as a backbone for expression of immunostimulatory genes in humans. In the present study, the two different murine IL-12 expression cassettes were each inserted into the deleted ICP6 locus of the G47Δ backbone to create two types of IL-12-expressing oncolytic HSV-1, T-mfIL12 and T-mIL12-IRES. The difference in effect between them should reflect the method employed to express mouse IL-12. In vitro and in vivo evaluation using mouse prostate cancer and neuroblastoma cells revealed that the fusion IL-12, T-mfIL12, expressed higher levels of functional IL-12 and exhibited higher antitumor activity than T-mIL12-IRES.

The present invention provides the followings.
[1] A pharmaceutical composition comprising a recombinant herpes simplex virus (HSV) with interleukin 12 (IL-12) expression, the recombinant HSV comprising a gene encoding a fusion polypeptide in which the 35 kDa light chain (p35) and the 40 kDa heavy chain (p40) of IL-12 are linked via two or more elastin motifs, and having the following characteristics (a) to (c):
   (a) the ICP6 gene is deleted or inactivated,
   (b) the γ34.5 gene is deleted or inactivated, and
   (c) the α47 gene is deleted or inactivated.
[2] The pharmaceutical composition according to 1, wherein the fusion polypeptide comprises p40, two or more elastin motifs, and p35 linked in this order from the N-terminal side.
[3] The pharmaceutical composition according to 1 or 2, wherein the gene encoding the fusion polypeptide is inserted into the ICP6 locus.
[4] The pharmaceutical composition according to any one of 1 to 3, wherein the gene encoding the fusion peptide is any of the following polynucleotides:
   (i) a polynucleotide consisting of the sequence of SEQ ID NO: 1;
   (ii) a polynucleotide consisting of a sequence having a sequence identity of 90% or higher to the sequence of SEQ ID NO: 1 and encoding a polypeptide capable of forming an active IL-12;
   (iii) a polynucleotide encoding a polypeptide consisting of the sequence of SEQ ID NO: 2;
   (iv) a polynucleotide encoding a polypeptide consisting of a sequence having a sequence identity of 90% or higher to the sequence of SEQ ID NO: 2 and capable of forming an active IL-12;
   (v) a polynucleotide consisting of the sequence of SEQ ID NO: 3;
   (vi) a polynucleotide consisting of a sequence having a sequence identity of 90% or higher to the sequence of SEQ ID NO: 3 and encoding a polypeptide capable of forming an active IL-12;
   (vii) a polynucleotide encoding a polypeptide consisting of the sequence of SEQ ID NO: 4; and
   (viii) a polynucleotide encoding a polypeptide consisting of a sequence having a sequence identity of 90% or higher to the sequence of SEQ ID NO: 4 and capable of forming an active IL-12.
[5] The pharmaceutical composition according to any one of 1 to 4, wherein the recombinant HSV is derived from G47Δ.
[6] The pharmaceutical composition according to any one of 1 to 5, which is used for enhancing the effect of IL-12 in a treatment of a tumor; the recombinant HSV defined in any one of 1 to 5 or a pharmaceutical composition comprising the same, which is for use in a method for enhancing the effect of IL-12 in a treatment of a tumor; use of the recombinant HSV with IL-12 expression defined in any one of 1 to 5 in manufacture of a pharmaceutical composition for enhancing the effect of IL-12 in a treatment of a tumor; a method for enhancing the effect of IL-12 in a treatment of a tumor, which comprises the step of administering the recombinant HSV with IL-12 expression defined in any one of 1 to 5 or a pharmaceutical composition comprising the same to a subject; or use of the recombinant HSV with IL-12 expression defined in any one of 1 to 5 or a pharmaceutical composition comprising the same, which is for enhancing the effect of IL-12 in a treatment of a tumor.
[7] The pharmaceutical composition according to any one of 1 to 6, which is for local injection; or the composition, use in manufacture, method, or use according to 6, wherein the pharmaceutical composition or the recombinant HSV with IL-12 expression is for local injection.
[8] A method for enhancing the effect of IL-12 in a treatment of a tumor, the method comprising administering the pharmaceutical composition comprising a recombinant HSV with IL-12 expression according to any one of 1 to 7 to a subject in need of the enhancement.
[9] A pharmaceutical composition comprising a recombinant HSV with IL-12 expression, the recombinant HSV comprising a gene encoding IL-12, and having the characteristics (a) to (c) mentioned above, which induces IFNγ production through IL-12 expression with no decrease of the replication ability of the recombinant HSV with IL-12 expression; a pharmaceutical composition comprising a recombinant HSV with IL-12 expression, the recombinant HSV comprising a gene encoding IL-12, and having the characteristics (a) to (c) mentioned above for use in a method for treating a tumor, which induces IFNγ production through IL-12 expression with no decrease of the replication ability of the recombinant HSV with IL-12 expression; use of a recombinant HSV with IL-12 expression, the recombinant HSV comprising a gene encoding IL-12, and having the characteristics (a) to (c) mentioned above in manufacture of a pharmaceutical composition for treating a tumor, wherein the composition induces IFNγ production through IL-12 expression with no decrease of the replication ability of the recombinant HSV with IL-12 expression; a method for treating a tumor, the method comprising administering a recombinant HSV with IL-12 expression , the recombinant HSV comprising a gene encoding IL-12, and having the characteristics (a) to (c) mentioned above or a pharmaceutical composition comprising the same to a subject, and wherein IFNγ production is induced through IL-12 expression with no decrease of the replication ability of the recombinant HSV with IL-12 expression; or a pharmaceutical composition comprising a recombinant HSV with IL-12 expression, the recombinant HSV comprising a gene encoding IL-12, and having the characteristics (a) to (c) mentioned above for use in a method for treating a tumor, which induces IFNγ production through IL-12 expression with no decrease of the replication ability of the recombinant HSV with IL-12 expression.
[10] A pharmaceutical composition for intravenous administration, comprising a recombinant HSV with IL-12 expression, the recombinant HSV comprising a gene encoding IL-12, and having the characteristics (a) to (c) mentioned above; a recombinant HSV with IL-12 expression, the recombinant HSV comprising a gene encoding IL-12, and having the characteristics (a) to (c) mentioned above or a pharmaceutical composition comprising the same, which is for use in a method for treating a tumor by intravenous administration; use of a recombinant HSV with IL-12 expression, the recombinant HSV comprising a gene encoding IL-12, and having the characteristics (a) to (c) mentioned above in manufacture of a pharmaceutical composition for intravenous administration for treating a tumor; a method for treating a tumor, the method comprising intravenously administering a recombinant HSV with IL-12 expression, the recombinant HSV comprising a gene encoding IL-12, and having the characteristics (a) to (c) mentioned above or a pharmaceutical composition comprising the same to a subject; or use of a pharmaceutical composition for intravenous administration containing an IL-12-expressing type recombinant type HSV, the recombinant HSV comprising a gene encoding IL-12, and having the characteristics (a) to (c) mentioned above for treating a tumor.
[11] A pharmaceutical composition for intratumoral administration, comprising a recombinant HSV with IL-12 expression, the recombinant HSV comprising a gene encoding IL-12, and having the characteristics (a) to (c) mentioned above, which elicits systemic anti-tumor immunity, and thereby attains at least one of suppression of metastasis and suppression of growth of a tumor in the absence of intratumoral administration; a recombinant HSV with IL-12 expression, the recombinant HSV comprising a gene encoding IL-12, and having the characteristics (a) to (c) mentioned above or a pharmaceutical composition comprising the same, which is for use in a method for treating a tumor in which intratumoral administration of the HSV or pharmaceutical composition elicits systemic anti-tumor immunity, and thereby attains at least one of suppression of metastasis and suppression of growth of a tumor in the absence of intratumoral administration; use of a recombinant HSV with IL-12 expression, the recombinant HSV comprising a gene encoding IL-12, and having the characteristics (a) to (c) mentioned above in manufacture of a pharmaceutical composition for intratumoral administration, which elicits systemic anti-tumor immunity, and thereby attains at least one of suppression of metastasis and suppression of growth of a tumor in the absence of intratumoral administration; a method for treating a tumor, the method comprising intratumorally administering a recombinant HSV with IL-12 expression, the recombinant HSV comprising a gene encoding IL-12, and having the characteristics (a) to (c) mentioned above or a pharmaceutical composition comprising the same to a subject, and wherein intratumoral administration of the HSV or pharmaceutical composition elicits systemic anti-tumor immunity, and thereby attains at least one of suppression of metastasis and suppression of growth of a tumor in the absence of intratumoral administration; or use of a recombinant HSV with IL-12 expression, the recombinant HSV comprising a gene encoding IL-12, and having the characteristics (a) to (c) mentioned above or a pharmaceutical composition for intratumoral administration containing the same for treating a tumor, wherein the HSV or pharmaceutical composition elicits systemic anti-tumor immunity, and thereby attains at least one of suppression of metastasis and suppression of growth of a tumor in the absence of intratumoral administration.
[12] A pharmaceutical composition for treating a tumor requiring increased infiltration of immune cells, comprising a recombinant HSV with IL-12 expression, the recombinant HSV comprising a gene encoding IL-12, and having the characteristics (a) to (c) mentioned above; a recombinant HSV with IL-12 expression, the recombinant HSV comprising a gene encoding IL-12, and having the characteristics (a) to (c) mentioned above or a pharmaceutical composition comprising the same, which is for use in a method for treating a tumor requiring increased infiltration of immune cells; use of a recombinant HSV with IL-12 expression, the recombinant HSV comprising a gene encoding IL-12, and having the characteristics (a) to (c) mentioned above in manufacture of a pharmaceutical composition for treating a tumor requiring increased infiltration of immune cells; a method for treating a tumor requiring increased infiltration of immune cells, the method comprising administering a recombinant HSV with IL-12 expression, the recombinant HSV comprising a gene encoding IL-12, and having the characteristics (a) to (c) mentioned above or a pharmaceutical composition comprising the same to a subject; or use of a recombinant HSV with IL-12 expression, the recombinant HSV comprising a gene encoding IL-12, and having the characteristics (a) to (c) mentioned above or a pharmaceutical composition comprising the same for treating a tumor requiring increased infiltration of immune cells.
[13] The pharmaceutical composition, use in manufacture, method, or use according to any one of 9 to 12, wherein the recombinant HSV is the recombinant HSV defined in any one of 1 to 5.

### Effects of the Invention

The recombinant HSV with IL-12 expression provided by the present invention can express active IL-12 (maintaining functional tertiary structure) upon viral replication when administered to tumor cells.

In addition, it can also achieve the following effects.
- The effect of combination of systemic administration of IL-12 and viral therapy can be achieved by administering only one type of virus.
- It exhibits enhanced therapeutic effect for both tumors with intratumoral administration (treated tumor) and distant tumors in the absence of intratumoral administration (untreated tumor) compared with a virus that does not express IL-12.
- Expressed IL-12 has the same function as IL-12 administered as a protein.
- Expressed IL-12 can be highly effective at lower levels compared with IL-12 administered as a protein.
- Expressed IL-12 stays at to the local site of tumor and is less likely to be released into the bloodstream, thus less likely to cause systemic side effects.
- Combination with oncolytic HSV-1 with soluble B7-1 expression may be expected to further enhance the therapeutic effect.
- Administration of IL-12 as a virus-expressed IL-12 may more effectively act on local and systemic anti-tumor immunity compared with mixed administration of protein IL-12 and non-expressing oncolytic virus.

### Brief Description of the Drawings

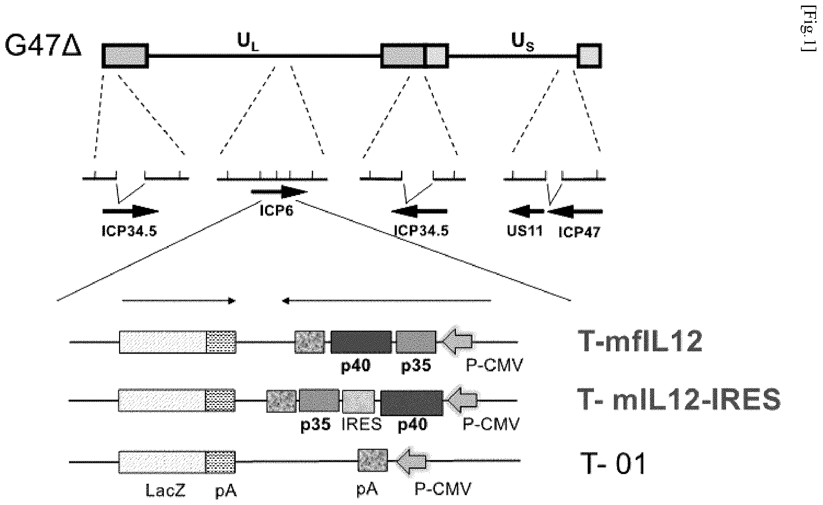

[Fig. 1] Structures of T-mfIL12 and T-mIL12-IRES. The boxes (top line) represent the DNA structure of HSV-1. The structure is composed of the long (U_{L}) and short (U_{S}) strands of the native sequence and inverted repeats flanking each of U_{L} and U_{S}. The oncolytic HSV-1 is based on G47Δ as the backbone and contains a deletion of approximately 1.0 kb in both copies of the γ34.5 gene, a deletion of approximately 0.3 kb in the α47 gene, and a deletion of approximately 0.9 kb in the ICP6 gene. The lacZ gene and the cytomegalovirus (CMV) promoter-regulated interleukin-12 (IL-12) gene are oriented in the opposite direction and inserted into the deleted ICP6 locus of G47Δ. T-mfIL12 comprises a cassette containing the p35 and p40 genes of mouse IL-12 linked via two bovine elastin motifs. The mouse IL-12 is expressed as a single fusion peptide and folded for activation. T-mIL12-IRES comprises a cassette in which the p40 and p35 genes of mouse IL-12 are separated by an internal ribosomal entry site (IRES) sequence derived from encephalomyocarditis virus (EMCV). The p40 and p35 subunits are efficiently co-expressed to form active mouse IL-12. T-01 contains an empty cassette with no transgene.

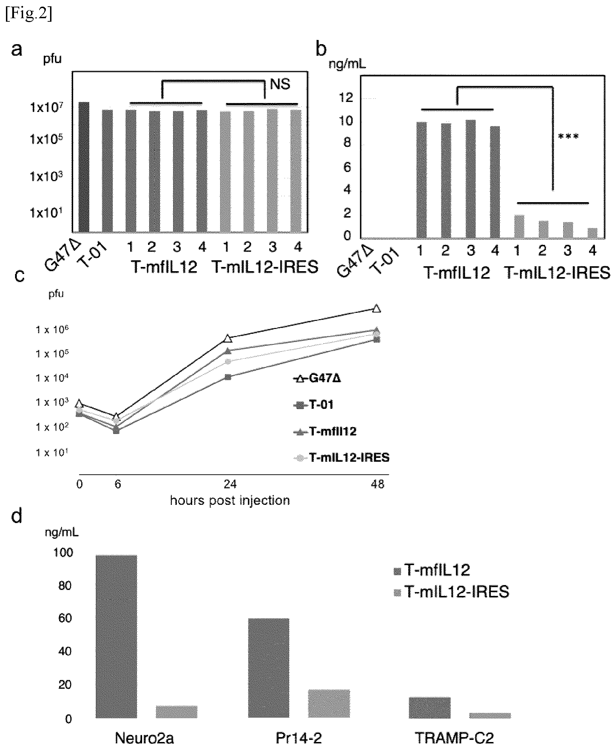

[Fig. 2] In vitro replication abilities of T-mfIL12 and T-mIL12-IRES and mouse IL-12 expression. a. In vitro replication assay: Vero cells were infected with G47Δ, T-01, T-mflL12 candidates (4 clones) or T-mIL12-IRES candidates (4 clones) at a multiplicity of infection (MOI) of 0.01. The progeny viruses were collected at 48 hours after the infection and counted by plaque assay. T-mfIL12 and T-mIL12-IRES did not show significant difference in replication ability (p=0.736, t-test). b. In vitro expression of mouse IL-12: Vero cells were infected with G47Δ, T-01, T-mflL12 candidates (4 clones) or T-mIL12-IRES candidates (4 clones) at an MOI of 1 and the amount of secreted mouse interleukin 12 (IL-12) (p70) was determined by enzyme-linked immunosorbent assay (ELISA). T-mfIL12 provided significantly higher expression amounts of p70IL-12 than T-mIL12-IRES (p<0.001, t-test). c. The time course of viral yields: Vero cells were infected with G47Δ, T-01, T-mfIL12 or T-mIL12-IRES at an MOI of 0.01, and progeny viruses were collected at 0, 6, 24, and 48 hours after the infection and counted by plaque assay. The viral yield in time course of T-mflL 12 was comparable to that of T-mIL12-IRES. d. In vitro mouse IL-12 expression in mouse tumor cell lines: Neuro2a, PR14-2 or TRAMP-C2 cells were infected with T-mfIL12 or T-mIL12-IRES at an MOI of 1 and the amount of secreted mouse IL-12 (p70) was determined by ELISA. T-mfIL12 provided expression of p70IL-12 at higher amounts than T-mIL12-IRES in all the three cell lines. All the assays were performed in duplicate. Bars, SD. ***, p<0.001; NS, not significant.

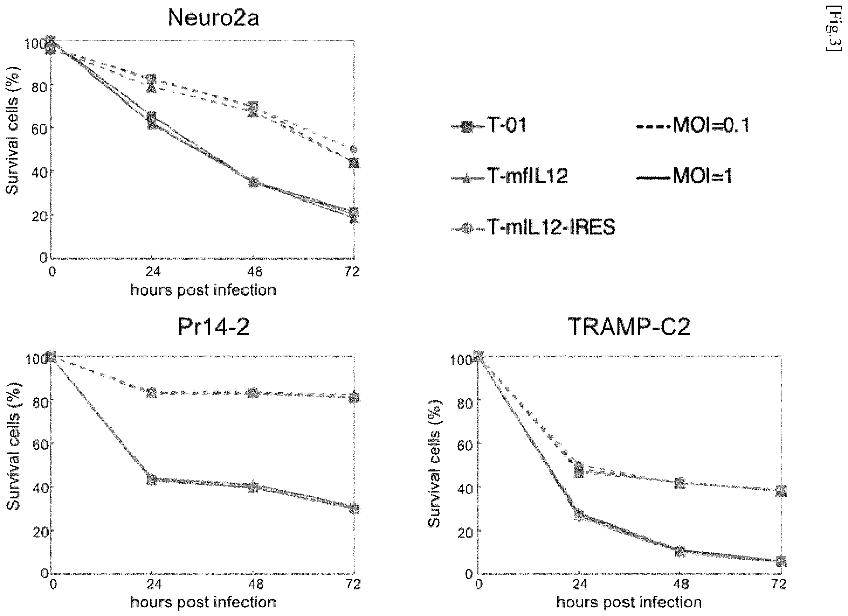

[Fig. 3] In vitro cytotoxic effects of T-mfIL12 and T-mIL12-IRES. In all the three mouse cancer cell lines, both T-mfIL12 and T-mIL12-IRES showed cytotoxic effects comparable to that of the control virus T-01 at both multiplicity of infection (MOI) = 0.1 (dotted line) and MOI = 1 (solid line). The number of viable cells was counted daily and expressed as a percentage based on that of the mock-infected control. The results are means of triplicate wells. Bar, SD.

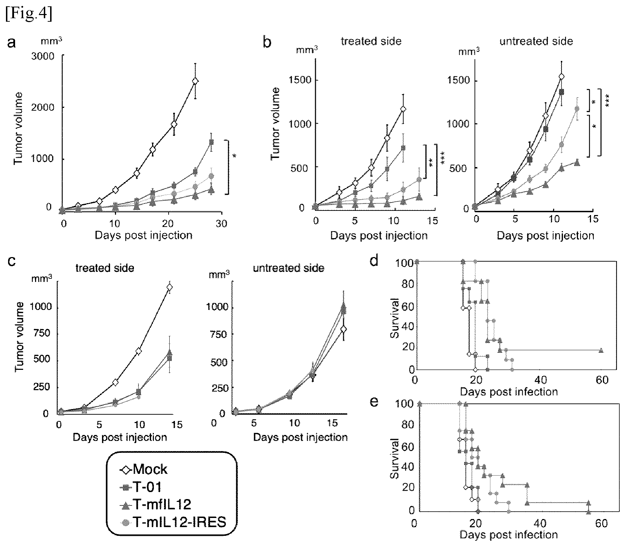

[Fig. 4] In vivo efficacy of T-mfIL12 and T-mIL12-IRES. a, b, and c. Assessment by subcutaneous tumor growth inhibition. a. Male C57BL/6 mice with unilateral subcutaneous TRAMP-C2 tumor (prostate cancer) were intratumorally inoculated with T-01, T-mfIL12, T-mIL12-IRES (5 x 10⁶ pfu) or mock on days 0 and 3 (n=6). T-mflL12 showed the highest efficacy. All the three viruses were significantly more effective than the mock (p<0.001 for all, significance is not shown in the graph). b. Female A/J mice with bilateral subcutaneous Neuro2a tumors (neuroblastoma) were intratumorally inoculated with T-01, T-mfIL12, T-mIL12-IRES (5 x 10⁴ pfu) or mock only in the left tumor on days 0 and 3 (n=9 to 12). T-mfIL12 showed the highest efficacy in both the treated and untreated sides. All the three viruses were significantly more effective than the mock on the treated side (p=0.001 for T-01, p<0.001 for both T-mfIL12 and T-mIL12-IRES), and T-mfIL12 and T-mIL12-IRES were more effective than the mock (p<0.001 for both viruses) on the untreated side (significant difference is not shown in the graphs). c. NOG mice with bilateral Neuro2a subcutaneous tumors were intratumorally inoculated with T-01, T-mfIL12, T-mIL12-IRES (1 x 10⁶ pfu) or mock only in the left tumor on days 0 and 3 (n=10). T-01, T-mfIL12, and T-mIL12-IRES showed no difference in effect on the treated side. The effect of IL-12 expression was completely abolished on both treated and untreated sides for both T-mfIL12 and T-mIL12-IRES. Tumor volume = length x width x height x 0.52. The results are mean values. The bars indicate standard error of the mean (SEM). *, p<0.05; **, p<0.01; ***, p<0.001; Two-way ANOVA with Bonferroni's multiple comparisons test. d and e. Evaluation based on survival rate. Female A/J mice with bilateral subcutaneous Neuro2a tumors were treated by intratumoral inoculation of 5 x 10⁵ pfu (d) or 5 x 10⁴ pfu (e) of T-01, T-mfIL12 or T-mIL12-IRES, or mock in the left tumor on days 0 and 3. The mice were euthanized when the maximum diameter of one of the bilateral tumors reached 22 mm. Both T-mflL12 and T-mIL12-IRES significantly prolonged survival of the carcinoma-bearing mice compared with T-01 at both doses (log-rank test). Although one or two of the mice administered with T-mfIL12 showed long-term survival at both doses, there was no significant difference between T-mflL12 and T-mIL12-IRES.

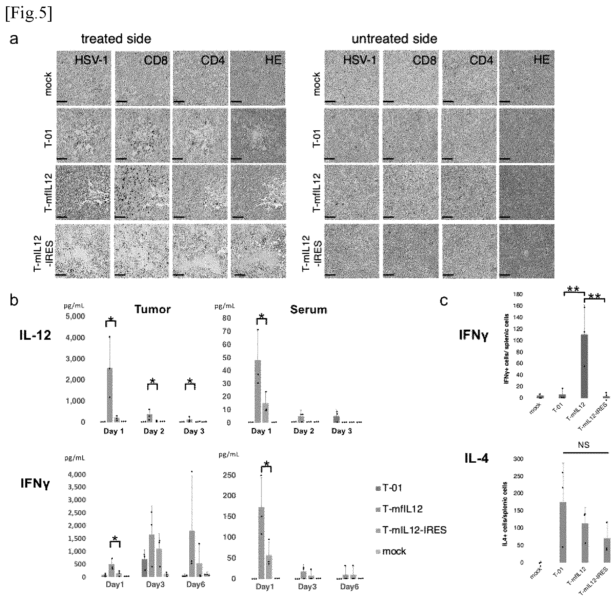

[Fig. 5] Immune response by T-mfIL12 and T-mIL12-IRES. a. Immunohistochemistry. Bilateral Neuro2a subcutaneous tumors were created in A/J mice, only the left tumor was inoculated with T-mfIL12, T-mIL12-IRES, T-01 (2 x 10⁵ pfu) or mock on days 0 and 3, and the tumors were harvested on day 6 (n=3 per group). Within the tumors, increased infiltration of CD4⁺ and CD8⁺ lymphocytes was observed on both the treated and untreated sides for all the three viruses, which was most pronounced for T-mfIL12 (Fig. 5, a). HSV-1-positive cells were observed within the tumors on the treated side for all the viruses but not on the untreated side. HE, hematoxylinandeosin; scale bar, 100 µm. b. In vivo levels of interleukin-12 (IL-12) and interferon γ (IFNγ). In unilateral Neuro2a subcutaneous tumor models, established tumors were inoculated with T-01, T-mfIL12, T-mIL12-IRES (2 x 10⁶ pfu) or mock, serum and tumor samples were collected on days 1, 3 and 6 (n=3 per group), and mouse IL-12 and IFNγ levels were measured by ELISA. The intratumoral IL-12 levels of the T-mfIL12-inoculated mice were significantly higher than those of the T-mIL12-IRES-inoculated mice at all time points (p=0.018, p=0.016, and p=0.046 for days 1, 3 and 6, respectively). The IL-12 levels detected in serum were significantly lower than those in the tumors. In correlation with intratumoral IL-12, the serum IL-12 levels of the T-mflL 12-inoculated mice were higher than those of the T-mIL12-IRES-inoculated mice on day 1 (p=0.047). The IFNγ levels of the T-mfIL12-inoculated mice were also significantly higher than those of the T-mIL12-IRES-inoculated mice in both tumor and serum on day 1 (p=0.014 and p=0.027 for tumor and serum, respectively). c. Specific immune responses to Neuro2a cells. In the unilateral Neuro2a subcutaneous tumor models, established tumors were inoculated with T-01, T-mfIL12, T-mIL12-IRES (5 x 10⁴ pfu) or mock on days 0 and 3, and the spleens were extracted on day 6. ELISpot assay revealed that IFNγ-releasing cells stimulated by the Neuro2a cells were significantly more abundant in the splenocytes of the T-mflL 12-treated mice compared with the splenocytes from the T-01 and T-mIL12-IRES-treated mice (p=0.005 and p=0.004 for T-01 and T-mIL12-IRES, respectively). There was no significant difference in the number of IL-4-releasing splenocytes among the three virus-administered groups. The number of IFNγ- or IL-4-releasing cells specific for Neuro2a cells was calculated by subtracting the number of SaI/N responding spots from the number of Neuro2a responding spots. For b and c, the graphs represent mean values. The dots represent the respective data. Bar, SD; *, p<0.05; **,p<0.01; NS, no significant difference; one-way ANOVA with Tukey's multiple comparison.

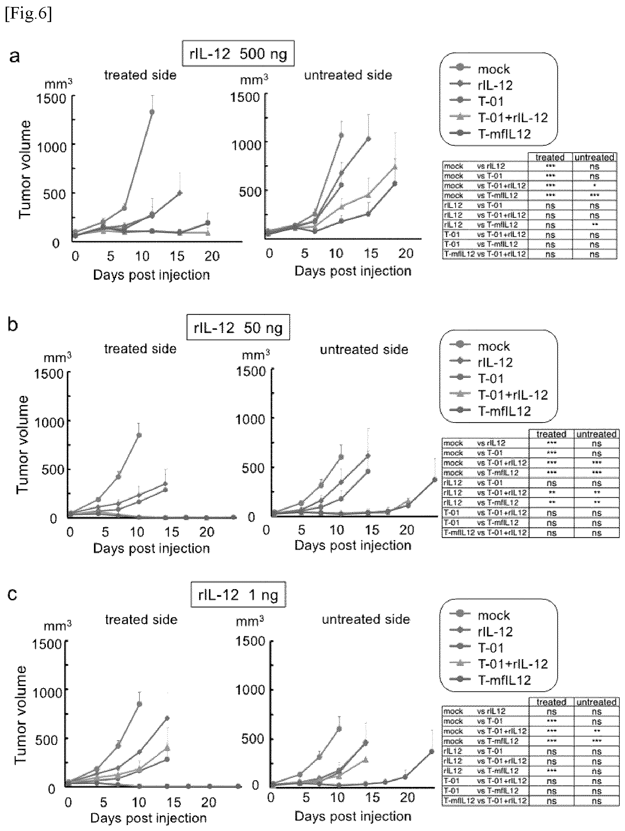

[Fig. 6] Comparison of in vivo efficacy of direct intratumoral injection with T-mflL 12 and recombinant interleukin-12 (rIL-12). In bilateral Neuro2a subcutaneous tumor models, only the left tumors were inoculated with rIL-12, T-01 (5 x 10⁴ pfu) with and without rIL-12, T-mfIL12 (5 x 10⁴ pfu), or mock on days 0 and 4 (n=10 per group). rIL-12 was inoculated at three different doses: 500 ng (a), 50 ng (b), and 1 ng (c). The dose of 50 ng corresponds to the IL-12 level in the tumors treated with 2 x 10⁶ pfu of T-mfIL12, and 1 ng corresponds to the IL-12 level in the tumors treated with 5 x 10⁴ pfu of T-mfIL12. a. When rIL-12 was used at the dose of 500 ng, rIL-12, T-01, T-01+rIL-12 and T-mfIL12 were significantly more effective compared with the mock (p<0.001, versus mock for all) on the treated side. On the untreated side, while rIL-12 alone showed no significant anti-tumor effect compared with the mock, T-01+rIL-12 and T-mflL12 were significantly more effective compared with the mock (p=0.039 and p<0.001 versus mock, respectively). Furthermore, on the untreated side, T-mflL12 was significantly more effective compared with rIL-12 alone (p=0.003), while T-01+rIL-12 did not show significant difference compared with rIL-12 alone. b. The dose of 50 ng of rIL-12 produced results similar to those of a. c. When rIL-12 was used at the dose of 1 ng, rIL-12 alone showed no significant effect on both the treated side and untreated side, and on the treated side, T-mfIL12 showed significantly higher effect compared with rIL-12 alone, but T-01+rIL-12 did not show significant difference compared with rIL-12 alone. Tumor volume = length x width x height x 0.52. The results are represented by means. Bar, standard error of the mean (SEM); *, p<0.05; **, p<0.01; ***, p<0.001; ns, no significant difference; two-way ANOVA with Bonferroni's multiple comparisons test.

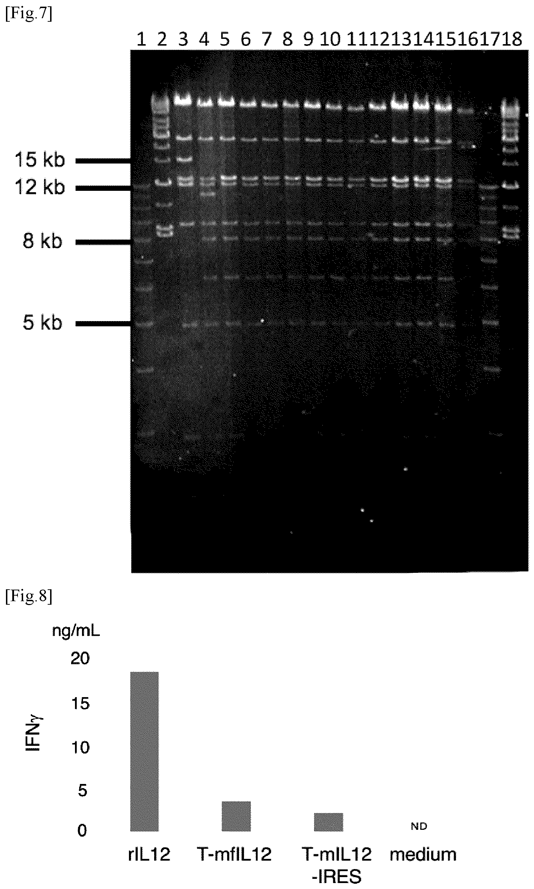

[Fig. 7] Representative gel electrophoresis confirming the structure of the T-BAC/Vec9 plasmid obtained after Cre recombination. DNAs of T-BAC (lane 3), T-BAC/Vec9-empty (lane 4), Nos. 1 to 6 of T-BAC/Vec9-fused-mIL12 (lanes 5 to 10) and Nos. 1 to 6 of T-BAC/Vec9-IRES-mIL12 (lanes 11 to 16) were digested with HindIII and separated by electrophoresis at 2.5 V/cm for 18 hors on a 0.6% (w/v) agarose gel in 1 x TB-EDTA buffer. All the bands were as expected: 15 kb for T-BAC (lane 3), 11 kb, 8 kb, and 6.2 kb for T-BAC/Vec9-empty (lane 4), and 8 kb and 6.2 kb for Nos. 1 to 6 of T-BAC/Vec9-fused-mIL12 (lanes 5 to 10) and Nos. 1 to 6 of T-BAC/Vec9-IRES-mIL12 (lanes 11 to 16). Lanes 1, 2, 17, and 18 are kb-markers.
[Fig. 8] Interferon γ release from splenocytes stimulated with virus-expressed IL-12. Supernatants of Vero cells infected with T-mfIL12 or T-mIL12-IRES at a multiplicity of infection (MOI) = 1 were collected at 48 hours after the infection. The splenocytes were administered with the supernatant, recombinant mouse IL-12 or DMEM (medium) for 48 hours and interferon-γ (IFNγ) levels were measured (n=2). IL-12 expressed by T-mfIL12 and T-mIL12-IRES both stimulated the splenocytes. ND, not detected.

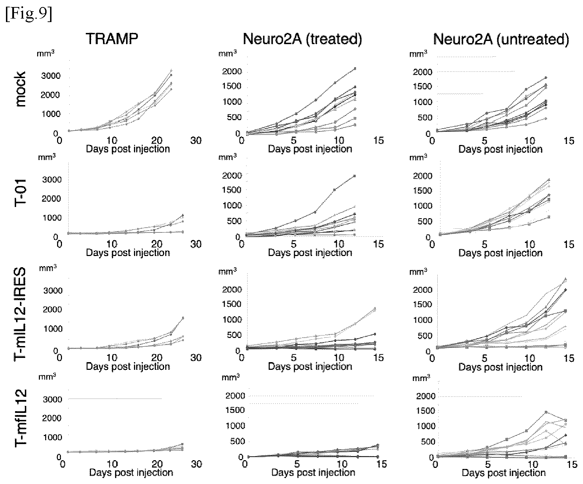

[Fig. 9] Tumor growth curves of individual animals used in the experiments of which results are shown in Fig. 4a and Fig. 4b. In the unilateral subcutaneous TRAMP-C2 tumor models, T-01, T-mfIL12, T-mIL12-IRES (5x 10⁶ pfu) or mock was intratumorally inoculated on days 0 and 3. In the bilateral subcutaneous Neuro2a models, T-01, T-mfIL12, T-mIL12-IRES (5 x 10⁴ pfu) or mock was inoculated to only the left tumors on days 0 and 3.

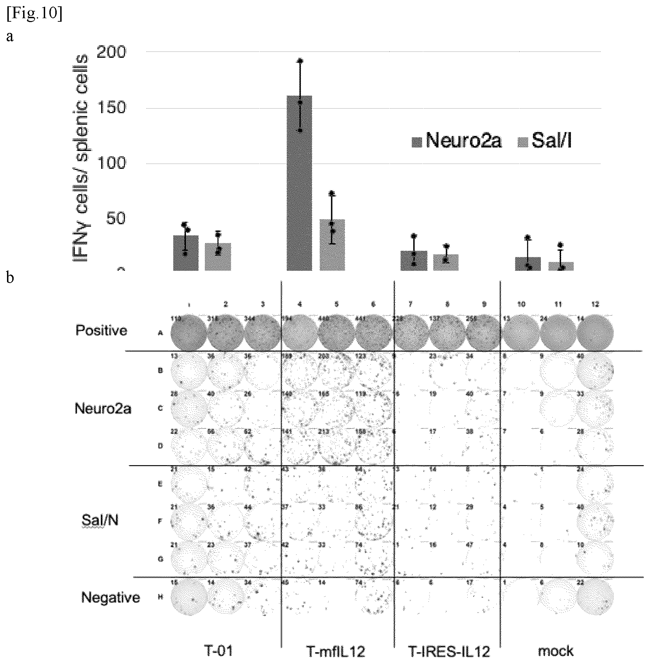

[Fig. 10] ELISpot assay for interferon γ (IFNγ). Established subcutaneous Neuro2a tumors were inoculated with T-01, T-mfIL12, T-mIL12-IRES (5 x 10⁴ pfu) or mock on days 0 and 3, and the spleens were harvested on day 6 (n=3). The splenocytes were stimulated with Neuro2a cells, SaI/N cells, ConA (positive control), or no stimulating agent (negative control). a. Graph showing the mean values. The dots represent the data from individual mice. b. Photograph of wells showing the results of ELISpot assay. Bar, SD.

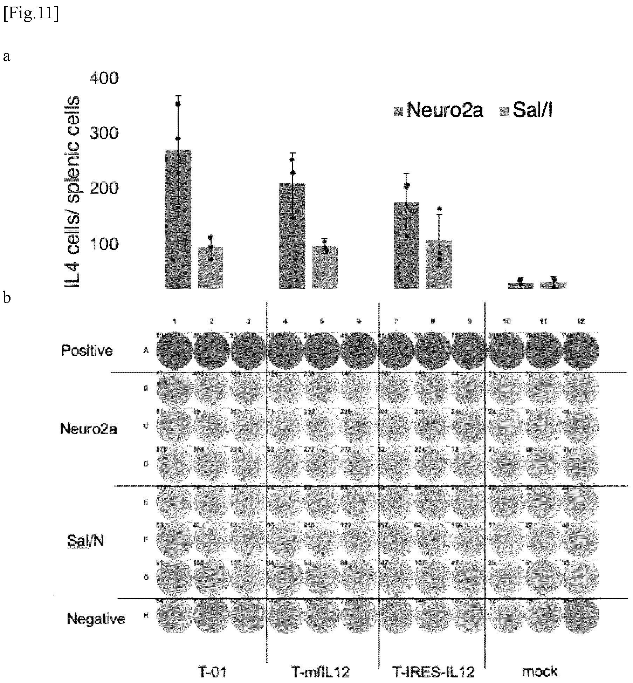

[Fig. 11] ELISpot assay for interleukin-4 (IL-4). Established Neuro2a subcutaneous tumors were inoculated with T-01, T-mfIL12, T-mIL12-IRES (5 x 10⁴ pfu) or mock on days 0 and 3, and the spleens were extracted on day 6. The splenocytes were stimulated with Neuro2a cells, SaI/N cells, ConA (positive control), or no stimulating agent (negative control). a. The dots represent the data from individual mice. b. Photograph of wells showing the results of the ELISpot assay. Bar, SD

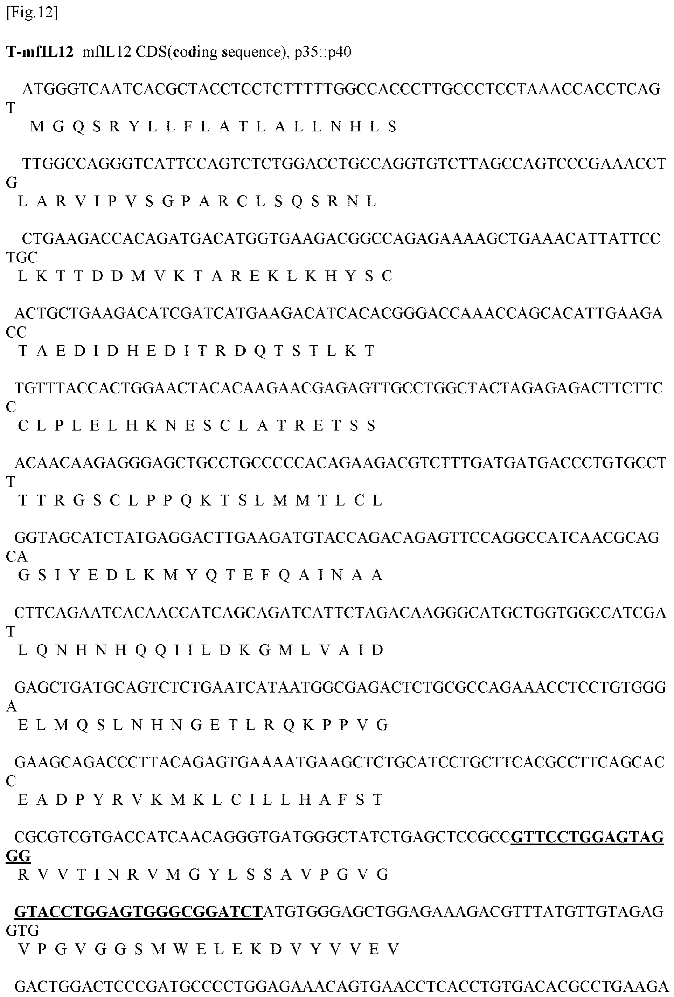

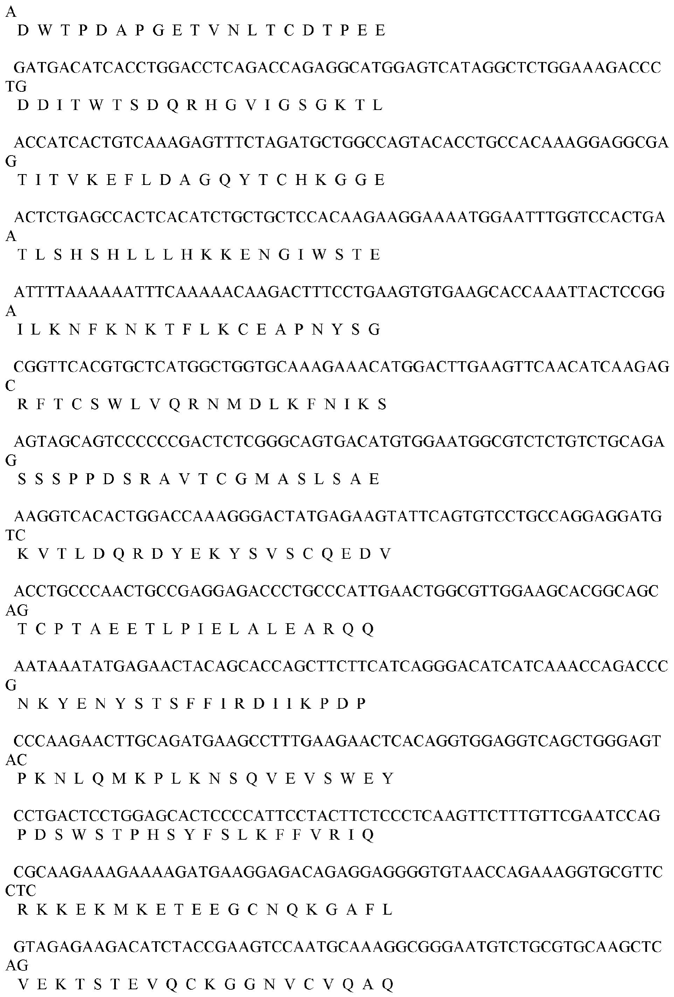

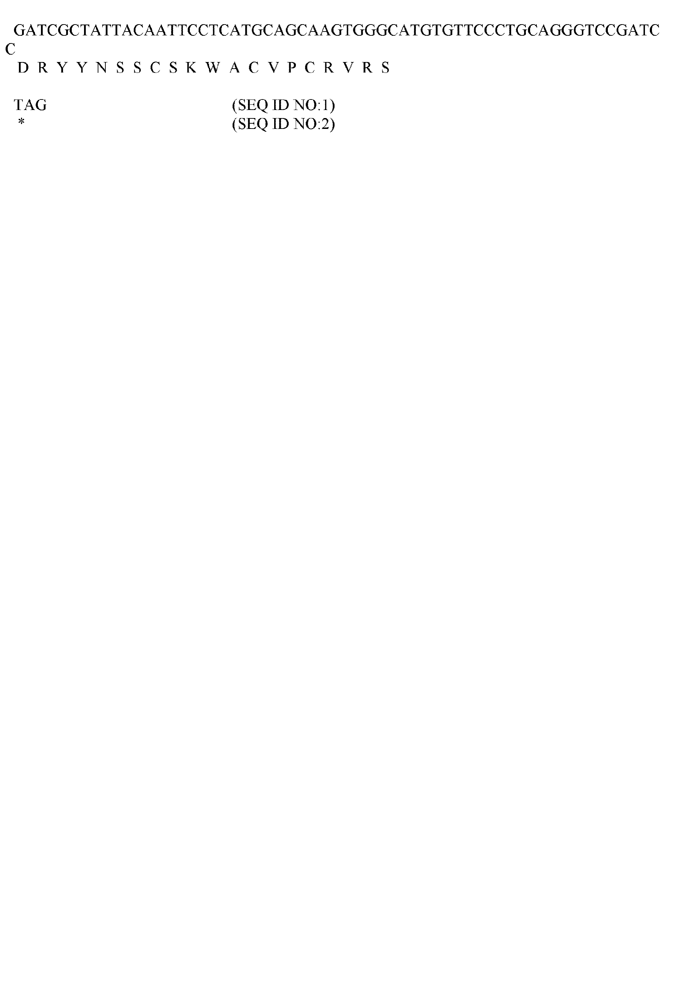

[Fig. 12] Nucleotide sequence data of the inserted nucleotides of T-mflL12. The underlined part of the nucleotides in T-mfIL12 corresponds to the bovine elastin motif. In T-mIL12-IRES, the underlined part corresponds to the IRES sequence.

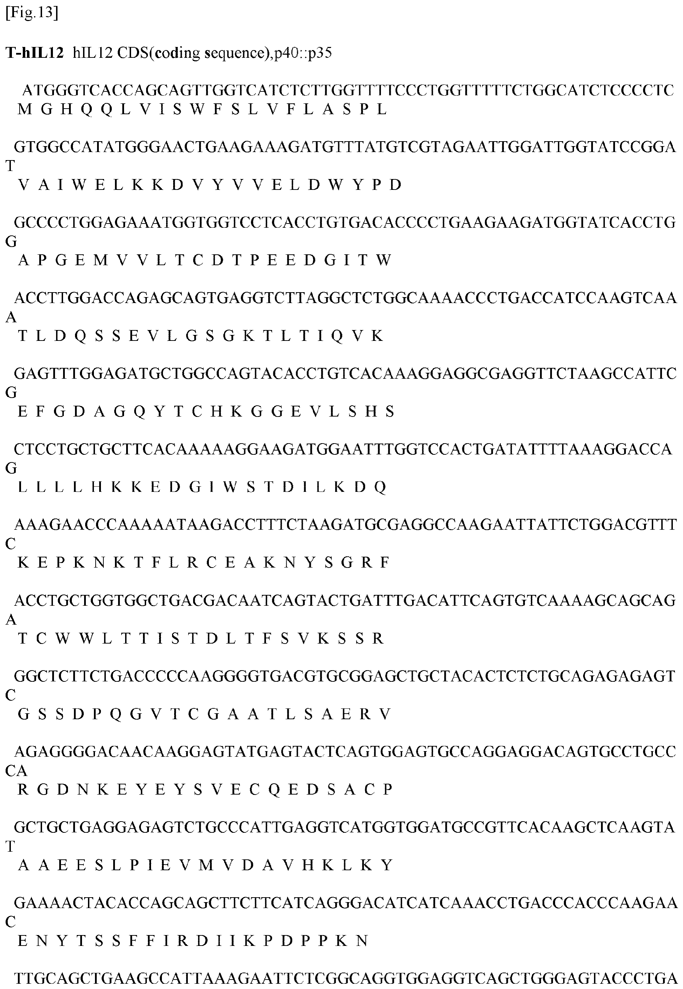

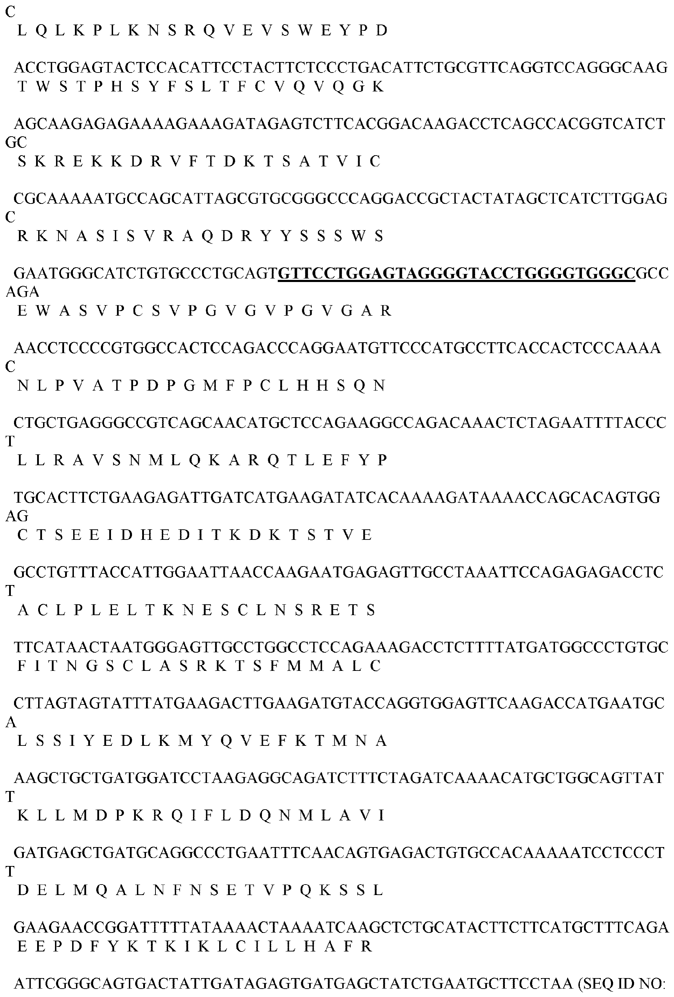

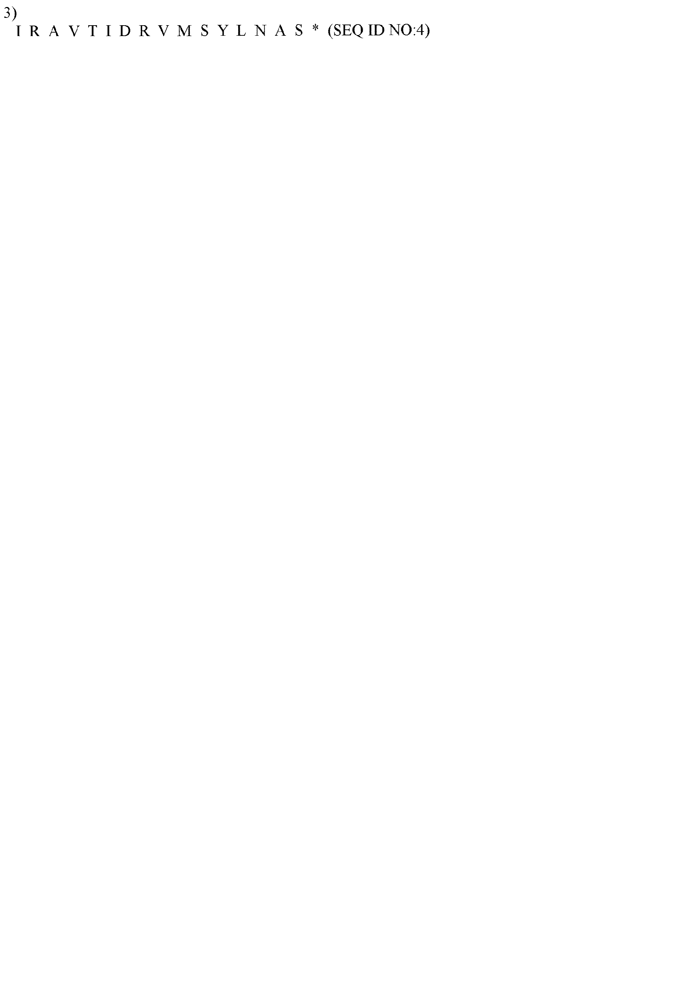

[Fig. 13] Nucleotide sequence data of the inserted nucleotides of T-hIL12. The underlined part corresponds to the bovine elastin motif.

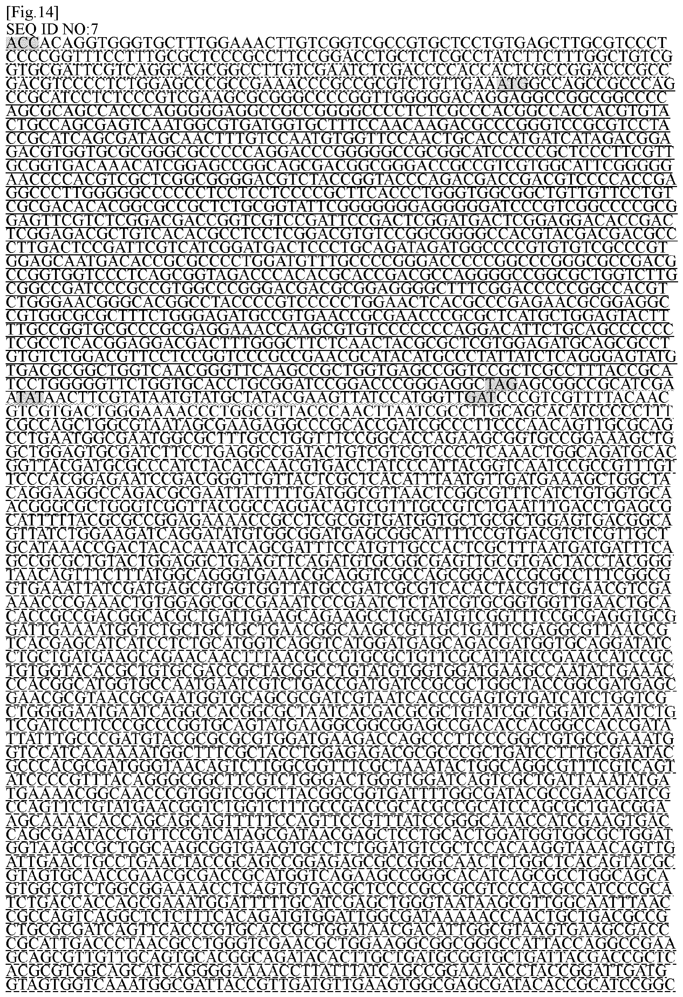

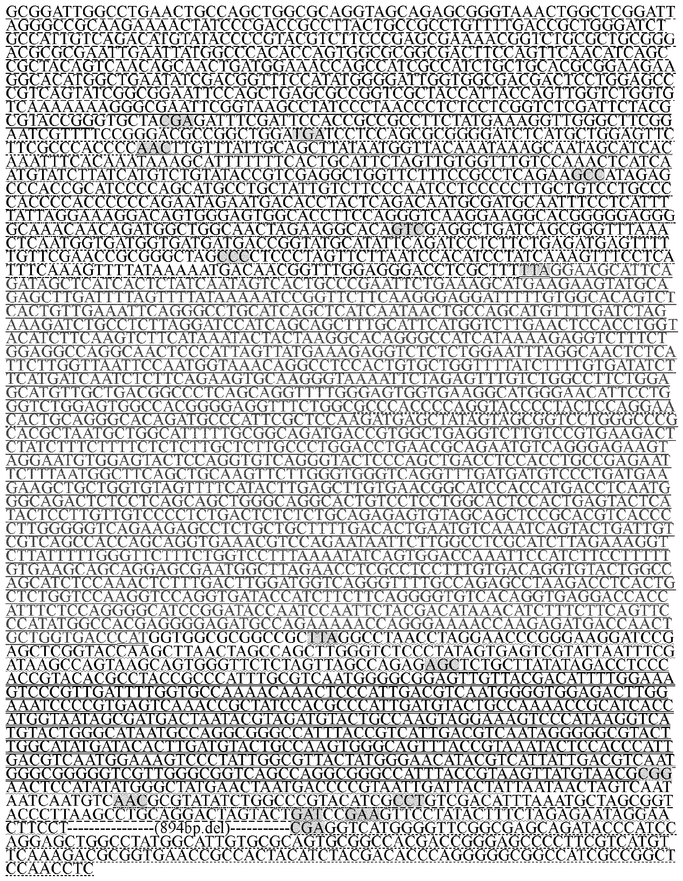

[Fig. 14] Nucleotide sequence of the fusion polypeptide insertion site and upstream and downstream parts thereof.

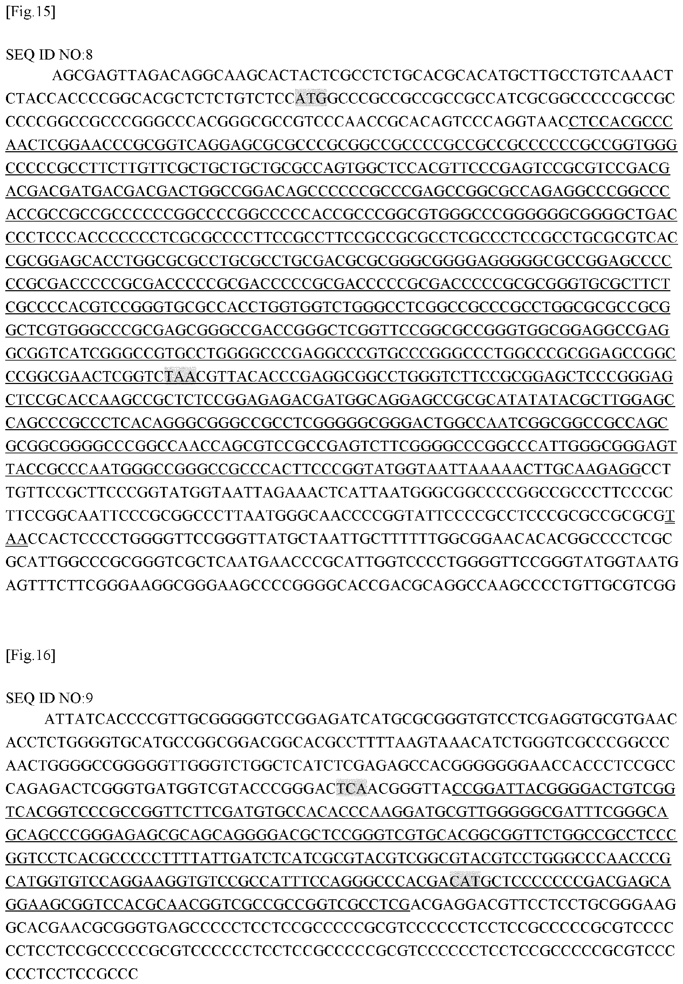

[Fig. 15] Nucleotide sequence of the γ34.5 gene deletion site and upstream and downstream parts thereof (in the TR_{L} region).
[Fig. 16] Nucleotide sequence of the α47 gene deletion site and upstream and downstream parts thereof.

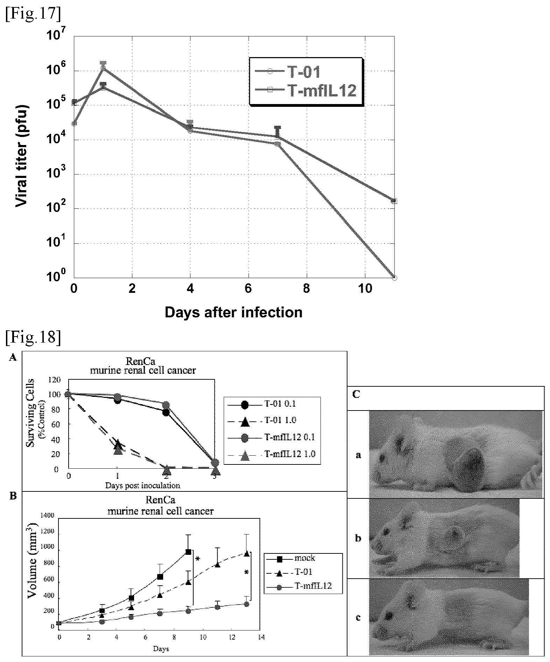

[Fig. 17] Study of virus replication ability in unilateral subcutaneous Neuro2a tumors of A/J mice. 5 x 10⁶ each of Neuro2a cells dispersed in 50 µL DMEM were subcutaneously injected into the left lateral flanks of A/J mice to create unilateral subcutaneous tumors. Five days later, a single dose of 1.0 x 10⁶ pfu/20 µL T-01 (n=3 for each measurement day) or T-mfIL12 (n=3 for each measurement day) was administered into the tumors. The mice were euthanized on the day of administration and 1, 4, 7, and 11 days after the administration, and the tumor portions were excised, weighed, and homogenized in PBS. Portions of the supernatants were titrated to determine the viral titer. I Bar, standard error.

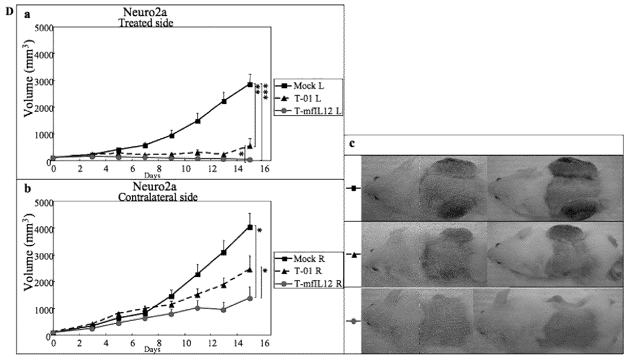

[Fig. 18] Experiments for in vitro cell-killing effect and comparison of T-01 virus and T-mfIL12 virus in subcutaneous tumor model. A. 2.0 x 10⁵ of RenCa cells were inoculated on a 6-well plate and infected with T-01 virus or T-mfIL12 virus at an MOI of 1.0 or 0.1. The cells were cultured at 34.5°C, and the numbers of viable cells were measured every 24 hours until day 4, and evaluated on the basis of the percentages based on the number of viable cells in the mock infection group. In the in vitro experimental system, the T-01 virus and T-mflL12 virus showed comparable cell-killing effects. B and C. BALB/c mice were subcutaneously injected with 1.0 x10⁵ each of RenCa cells into the left flanks to create subcutaneous tumors, and when the largest tumor diameter reached about 5 mm (day 0), 2 x 10⁵ pfu of T-01 virus or T-mfIL12 virus was intratumorally injected on day 0 and day 3 (n=8/group). In the T-mflL12 virus-administered group, tumor growth was significantly suppressed compared with the mock and T-01 virus-administered groups (p=0.0072 and p=0.0202, respectively). 0.001≤P<0.05 was marked with *. While the planned intratumoral dose of T-01 virus or T-mfIL12 virus was 2 x 10⁵ pfu, the actual doses were found to be 0.6 x 10⁵ and 0.5 x 10⁵ pfu, respectively, as a result of titration. C. Typical examples of mice on day 9 during the RenCa subcutaneous tumor model experiment. a, Mock-administered group; b, T-01 virus-administered group; and c, T-mflL12 virus-administered group. In the T-mflL12 virus-administered group, tumors disappeared in 3 out of 8 mice.

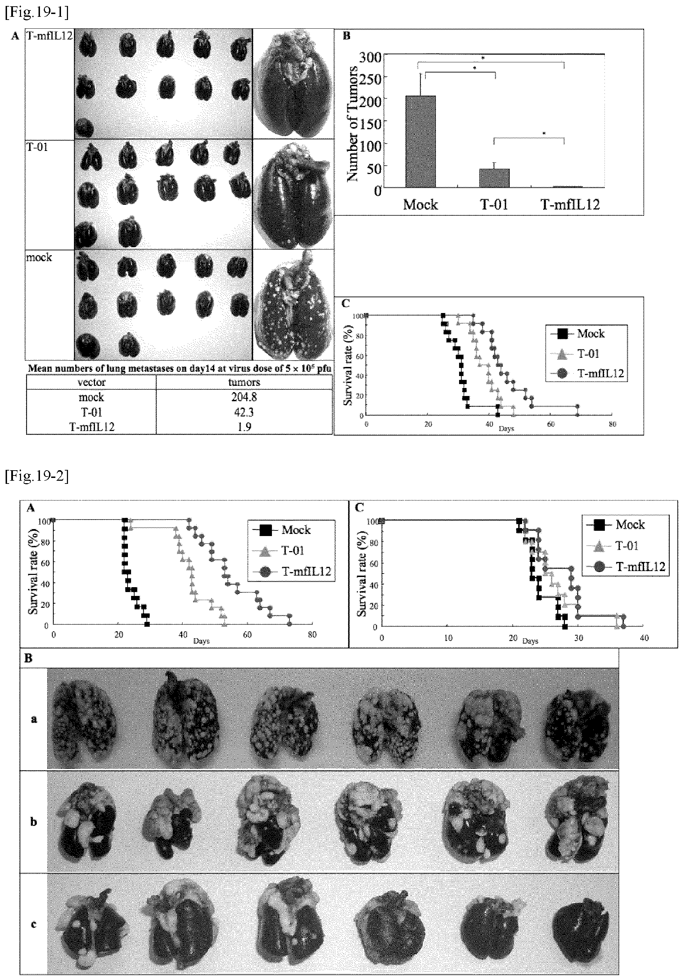

[Fig. 19-1] Intravenous administration experiments of T-01 virus and T-mfIL12 virus (5 x 10⁵ pfu) in RenCa lung metastasis model. BALB/c mice were intravenously administered with 2.0 x 10⁵ RenCa cells through the tail vein (day 0) and intravenously administered with 5 x 10⁵ pfu of T-01 virus or T-mfIL12 virus on days 1, 3 and 5. A and B. The number of lung metastases was evaluated on day 14 (n=11/group). The means of the numbers of metastases were 205, 42, and 1.9 in the mock, T-01 virus, and T-mfIL12 virus-administered groups, respectively, and the numbers of metastases significantly decreased in the T-01 and T-mfIL12 virus-administered groups compared with the mock-administered group (p=0.0210 and p=0.0017, respectively). The number of metastases significantly decreased in the T-mfIL12 virus-administered group also compared with the T-01 virus-administered group (p=0.0159). 0.001≤P<0.05 was marked with *. The actual doses of T-01 virus and T-mfIL12 virus were found to be 2.1 x 10⁵ and 5.9 x 10⁵ pfu, respectively, as a result of titration. C. Survival time was observed by using the same protocol (n=12/group). The T-01 and T-mfIL12 viruses significantly prolonged mouse survival time compared with the mock (p=0.0016 and p<0.0001), and the T-mflL12 virus significantly prolonged mouse survival time also compared with the T-01 virus (p=0.0154). The actual doses of the T-01 virus and T-mflL12 virus were found to be 5.6 x 10⁵ and 5.0 x 10⁵ pfu, respectively, as a result of titration.
[Fig. 19-2] Intravenous administration experiments of T-01 virus and T-mfIL12 virus in RenCa lung metastasis model (5 x 10⁶ pfu). BALB/c mice were intravenously administered with 2.0 x 10⁵ of RenCa cells through the tail vein (day 0). A and B. The mice were intravenously administered with 5 x 10⁵ pfu of the T-01 virus or T-mflL12 virus on days 1, 3, and 5, and the survival time was observed (n=12/group). The T-01 and T-mfIL12 viruses significantly prolonged the survival time of the mice compared with the mock (p<0.0001 and p<0.0001), and the T-mfIL12 virus significantly prolonged the survival time of the mice also compared with the T-01 virus (p<0.0003). The actual doses of the T-01 virus and T-mfIL12 virus were found to be 3.3 x 10⁶ and 3.1 x 10⁶ pfu, respectively, as a result of titration. B. Lungs extracted at death. a. Mock-administered group; b. T-01 virus-administered group; and c. T-mfIL12 virus-administered group. C. When the start date of the treatment was delayed until day 9, 11, and 13, only the T-mfIL12 virus significantly prolonged the survival time of the mice (p=0.0054, n=10/ group) compared with the mock. The actual doses of the T-01 virus and T-mflL12 virus were found to be 4.6 x 10⁶ and 5.2 x 10⁶ pfu, respectively, as a result of titration.

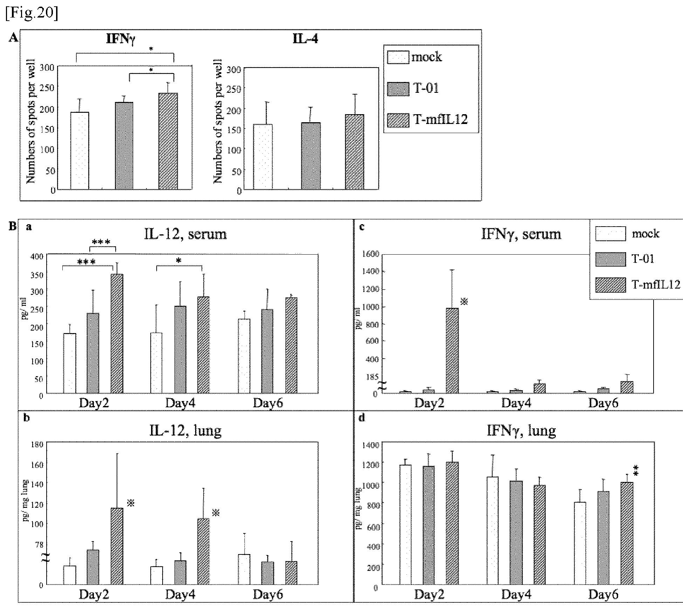

[Fig. 20] Cytokine secretion activity test for intravenous administration of T-01 virus and T-mfIL12 virus in RenCa lung metastasis model. BALB/c mice were intravenously administered with 2.0 x 10⁵ of RenCa cells through the tail vein (day 0), and intravenously administered with 5 x 10⁶ pfu of T-01 virus or T-mfIL12 virus on days 1, 3 and 5, and the spleens were extracted on day 14. The extracted spleens were made into single cell suspensions, and 2 x 10⁵ each of the cells were inoculated in wells of a 96-well plate, and mIFNγ and mIL-4 secretion activities were measured by ELISpot assay. For each group, the measurement was independently performed in triplicate. For mIFNγ and mIL-4, the incubation time was 24 and 48 hours. RenCa cells were used for antigen stimulation. Under the RenCa antigen stimulation, increased IFNγ secretion was observed in the T-01 virus and T-mfIL12 virus-administered groups compared with the mock-administered group (p=0.0726 and p=0.0112), and a significant difference was observed for T-mfIL12 virus. The T-mflL12 virus-administered group showed significantly enhanced secretion also compared with the T-01 virus-administered group (p=0.0251). No significant difference was observed in either group with respect to IL-4 secretion activity. 0.001≤P<0.05 was marked with *.

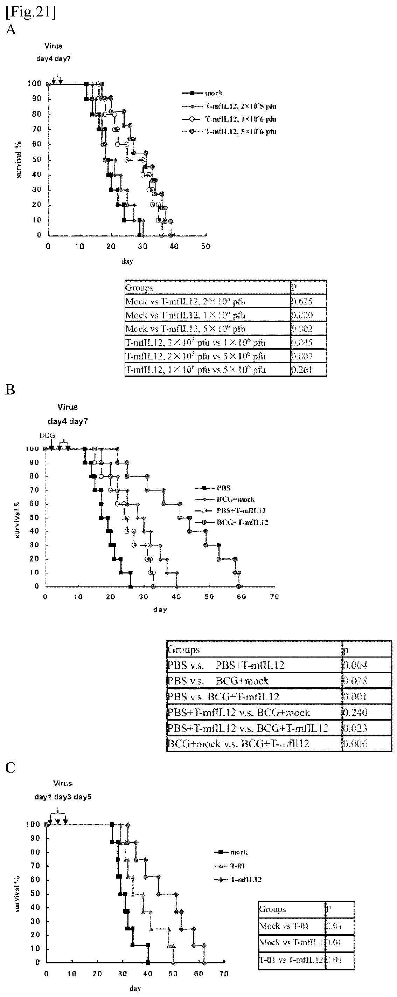

[Fig. 21] A. Survival rate analysis. MB49 cells (5.0 x 10⁵) were inoculated into the bladders of female C57BL/6 mice, and T-mfIL12 was intravesically administered at three different doses (2.0 x 10⁵, 1.0 x 10⁶ and 5.0 x 10⁶ pfu) on days 4 and 7 after the tumor inoculation. Survival rate data were analyzed by using the log-rank test (n=10 per group). As a result, T-mfIL12 showed statistically significant differences (P<0.05 and P<0.01) at the medium and high doses (1 x 10⁶ and 5 x 10⁶ pfu) compared with the control. However, it did not show any effect at the low dose (P=0.625). B. Survival rate analysis. Female C57BL/6 mice were intravesically inoculated with MB49 cells (5.0 x 10⁵), and intravesically administered with PBS or BCG (1.35 mg) on day 1 and mock or T-mfIL12 (1.0 x 10⁶ pfu) on days 4 and 7. The survival data were analyzed by using the log-rank test (n=10 per group). Significant and strong antitumor effects (P<0.05, log-rank test) were observed in all the treatment groups (BCG+mock, PBS+T-mflL12, and BCG+T-mfIL12). The combination treatment showed significantly higher anti-tumor effect compared with the other groups. About 30% of the mice in the combination treatment group survived for 50 days (P<0.01, log-rank test). C. Survival analysis. Female C57BL/6 mice were injected with 1.0 x 10⁵ of MB49 cells via the tail veins and administered with mock, T-01, or T-mfIL12 (5.0 x 10⁶ pfu) on days 1, 3 and 5. Kaplan-Meier survival analysis showed that the two treatment groups showed significant differences compared with the control (log-rank test, P=0.04 and P<0.01, respectively), and a significant difference was also observed between T-01 and T-mfIL12 treatments (log-rank test, P<0.05). Thus, improvement of survival rate by the T-mfIL12 treatment was demonstrated.

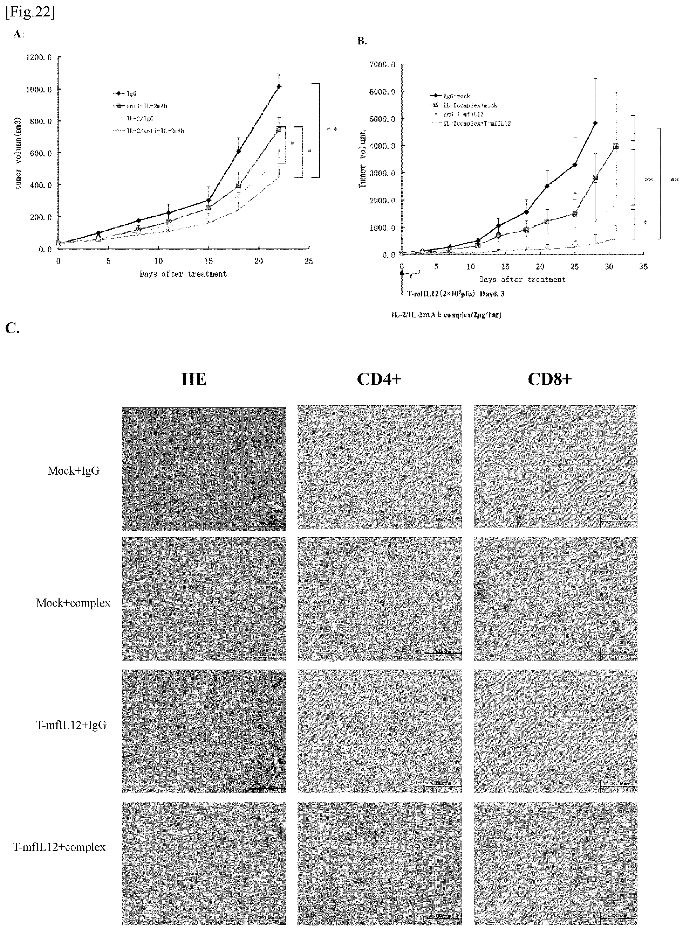

[Fig. 22] A: Inhibition of tumor growth by IL-2/anti-IL-2 mAb complex. BALB/c mice with established RenCa subcutaneous tumors were treated with IgG, anti-IL2 mAb (1 mg), IL2 (2 µg) or IL-2/anti-IL-2 mAb (2 µg/1 mg) complex on day 5 (n=10 per group). Tumor growth was inhibited by the IL-2/anti-IL-2 mAb complex compared with anti-IL2 mAb (P<0.05; bar, ±SDp; days 18 to 23) or control treatment (P<0.01; bar, ±SD; days 12 to 23). *, P<0.05; **, P<0.01. B: Inhibition of tumor growth by combination therapy. BALB/c mice with established RenCa subcutaneous tumors were treated with IgG+mock, IL-2/anti-IL-2 mAb complex (2 µg/1 mg)+mock, IgG+T-mfIL12 (1.0 x 10⁶ pfu) or IL-2/anti-IL-2 mAb (2 µg/1 mg) complex+T-mfIL12 (1 x 10⁶ pfu) on days 0 and 3 (n=10 per group). Subcutaneous RenCa tumors were significantly smaller in mice of the combination treatment group. Statistical significance was observed at this time point (P<0.0001; bar, SD). *, P<0.05; **, P<0.01. C: Histology and immunohistochemistry of tumors produced by RenCa subcutaneous tumors obtained from the indicated treatment groups. H&E staining of primary tumor tissues derived from RenCa cells of BALB/c mice after treatment with T-mfIL12 and/or IL-2/anti-IL-2 mAb for 2 weeks. Areas showing reduced cellularity and necrosis were seen in the combination treatment group. Immunohistochemistry of the tumors treated with the combination therapy also showed increased CD4⁺ (blue dots) and CD8⁺ (blue dots) T lymphocyte infiltration.

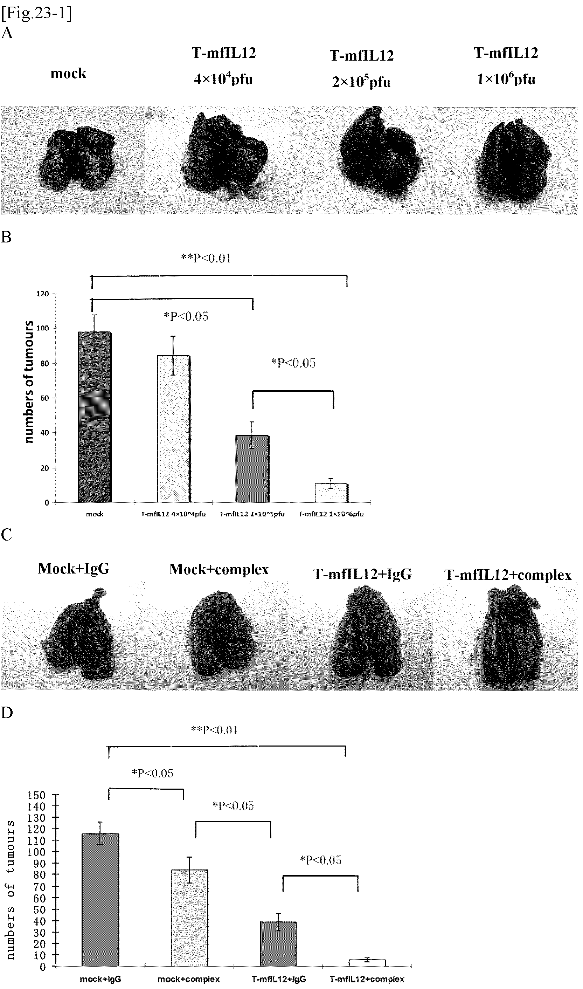

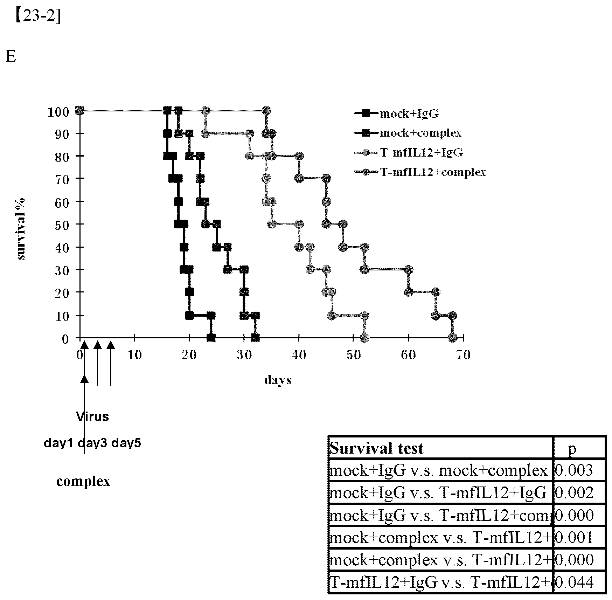

[Fig. 23] A: Representative views of actual lungs. B: The antitumor effect of T-mfIL12 dose-dependently inhibited tumor growth in mouse RCC lung metastasis lesions. BALB/c mice received intravenous injection of RenCa cells on day 0. T-mflL12 was administered via tail veins on days 1, 3 and 5 (n=10). On day 14, the animals were sacrificed and the number of lung metastases was counted. The mean number of lung surface nodules in the mock-treated control was significantly greater than those of 2.0 x 10⁵ and 1.0 x 10⁶ pfu T-mfIL12-treated animals (P<0.05 and P<0.01, respectively; t test; bar, ±SD). No surface nodules were observed in some of the animals treated with 1.0 x 10⁶ pfu of T-mfIL12,. *, P<0.05; **, P<0.01. C: Representative views of actual lungs. D: Anti-tumor immunity is enhanced by simultaneous use of T-mfIL12 and IL-2/anti-IL2 mAb complex. Mice were intravenously inoculated with RenCa cells on day 0. On day 1, the tumor-bearing mice were treated with 2.0 x 10⁵ pfu of T-mfIL12 and 2 µg/1 mg of IL-2/anti-IL2 mAb complex, as indicated. T-mfIL12 was repeatedly administered on days 3 and 5. Lung metastasis was assessed on day 14. There are shown representative images of the lung at 14 days after the RenCa cell inoculation (top). RenCa cell nodules in the lungs were counted on day 14 (bottom). The combination of T-mfIL12 and IL-2/anti-IL-2 mAb complex was effective and significantly reduced the number of nodules in Balb/c mice bearing RenCa cells in the lungs, while T-mfIL12 or IL-2/anti-IL-2 mAb complex alone slightly reduced the number of nodules. *, P<0.05; **, P< 0.01 (t-test, bar ±SD). E: Effect of combination treatment on survival rate was examined by using a RenCa cell lung metastasis model. Carcinoma-bearing mice were administered with 2 x 10⁵ pfu of T-mfIL12 or mock on days 1, 3, and 5. The mice were intraperitoneally injected with 2 µg/1 mg of IL-2/anti-IL2 mAb complex on day 1. Survival rate analysis was performed by plotting according to the Kaplan-Meier method, and statistical differences were determined by using the log-rank test. The median survival time of the control animals treated with the mock and IgG was 18 days, and 100% died on day 24. In contrast, the median survival times for the groups of the treatments with the combination, T-mfIL12 alone, and IL-2/anti-IL-2 mAb complex alone were 49, 38, and 25 days, respectively (log-rank test, P< 0.001). Significant and strong antitumor effects (P<0.05, log-rank test) were observed in all the treatment groups (complex+mock, IgG+T-mfIL12, and complex+T-mfIL12).

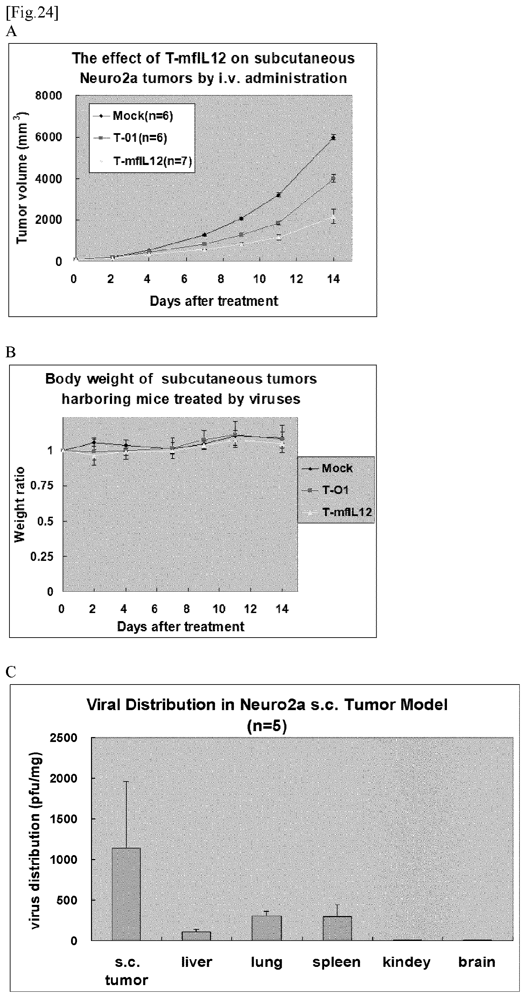

[Fig. 24] A: Effect of intravenous administration of T-mfIL12 on subcutaneous Neuro2a. Intravenous administration of oncolytic HSV-1 showed significant antitumor effect, which was further enhanced by IL-12 (days 7 to 14; mock vs T-01 and T-01 vs TmfIL12, p<0.001; SNK; bar, SEM). B. Body weight of subcutaneous tumor-bearing mice administered with virus. No weight loss was observed in all three groups after the intravenous administration, but a slight weight loss was observed on day 2 in the T-mfIL12-administered group (p<0.05; SNK; bar, SD). C: Virus distribution in the Neuro2a subcutaneous tumor model. Real-time PCR showed that the amount of viral DNA in subcutaneous tumor samples was higher than in normal samples (bar, SEM).

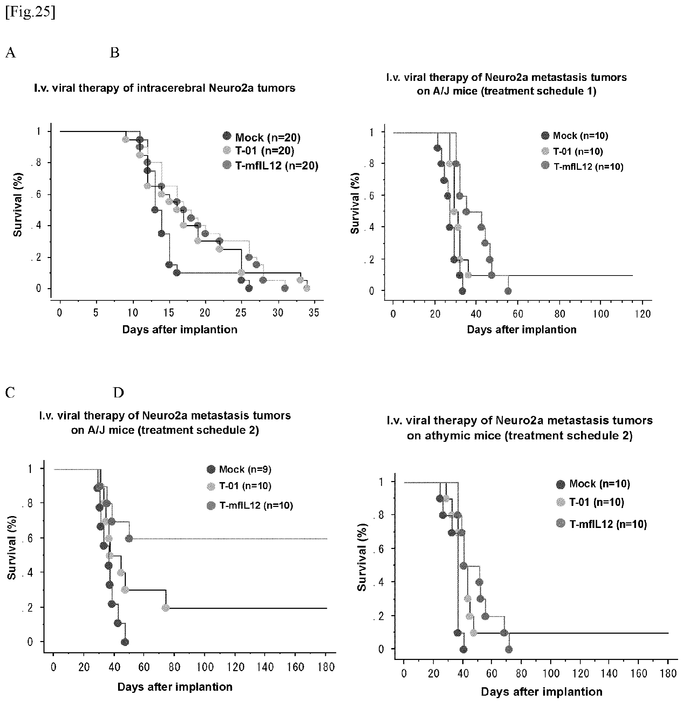

[Fig. 25] A: Virus intravenous administration therapy of Neuro2a tumors in the brain. Mice were intravenously administered with virus vectors on days 5, 7, and 9 after tumor cell inoculation. As a result, survival time of the mice administered with T-mflL12 was significantly prolonged compared with the mice administered with mock (p<0.05, Breslow-Gehan-Wilcoxon). B: Virus intravenous administration therapy for Neuro2a metastatic tumors in A/J mice (treatment schedule 1). Mice were intravenously administered with virus vectors on days 1, 4, and 7 after intravenous inoculation of tumor cells. The survival times of the mice administered with T-mfIL12 or T-01 were significantly prolonged compared with the mice administered with the mock (T-mfIL12 vs T-01, p<0.05; T-mfIL12 vs mock, p< 0.001; T-01 vs mock, p<0.05, respectively, Breslow-Gehan-Wilcoxon). C: Virus intravenous administration therapy for Neuro2a metastatic tumors in A/J mice (treatment schedule 2). Mice were intravenously administered with virus on days 1, 3, and 5 after tumor cell inoculation. The survival times of the T-mfIL12-treated mice were significantly prolonged compared with the mock-treated mice (p<0.01, Breslow-Gehan-Wilcoxon), and amazing survival times of 180 days or longer were observed in 2/10 of the T-01-treated mice and 6/10 of the T-mfIL12-treated mice. D: Virus intravenous administration therapy for Neuro2a metastatic tumors in nude mice (treatment schedule 2). Nude mice without T cells were intravenously administered with virus on days 1, 3 and 5 after tumor cell inoculation. The survival times of the mice administered with T-01 or T-mfIL 12 were significantly prolonged compared with the mice administered with the mock (p<0.05 and p<0.005, respectively; Breslow-Gehan-Wilcoxon).

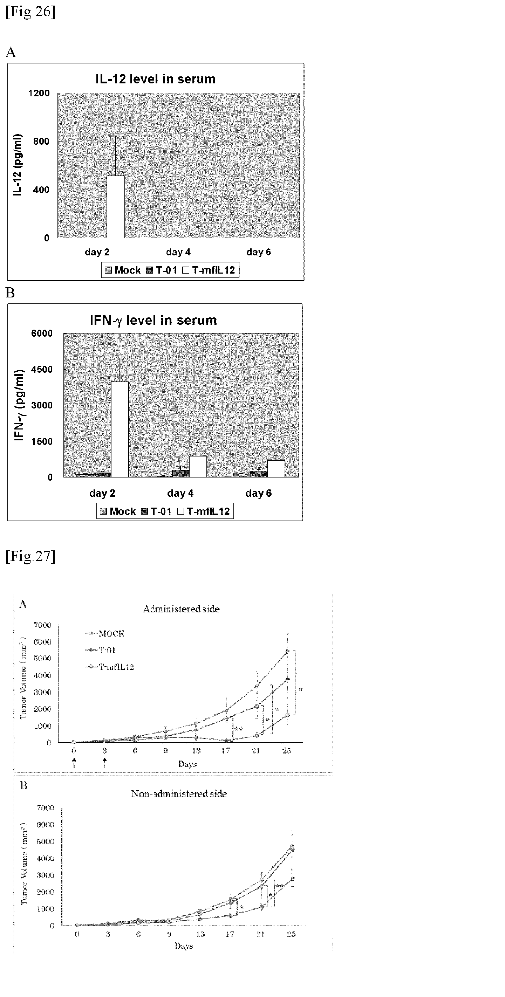

[Fig. 26] A: IL-12 levels in serum. ELISA was performed for serum interleukin (IL)-12 in A/J mouse Neuro2a metastatic tumor models intravenously administered with three different doses of mock, T-01, or T-mfIL12 (n=3). IL-12 levels were higher only on day 2 in the mice administered with T-mfIL12. In contrast, IL-12 levels were undetectable in the mice administered with the mock and T-01 at all time points (bar, SEM). B: Serum IFNγ levels. ELISA was performed for serum IFNγ in A/J mouse Neuro2a metastatic tumor models intravenously administered with three different doses of mock, T-01, or T-mfIL12 (n=3). The evaluated IFNγ levels initially decreased from day 2 to day 6. There were significant differences in IFNγ expression on day 6 among the three groups (T-mfIl12 vs T-01, p=0.019; T-01 vs mock, p=0.030; T-mfIL12 vs mock, p=0.007; t test; bar, SD).
[Fig. 27] Subcutaneous tumor treatment experiment using DBA/2 mouse bilateral subcutaneous tumor model. DBA/2 mice were subcutaneously injected with 1.0 x 10⁶ cells/mouse of mouse malignant melanoma cell line Clone M3 into the right and left lateral flanks to create subcutaneous tumors. The day on which the maximum tumor diameter reached approximately 5 mm was defined as day 0, and on day 0 and day 3, T-01 or T-mfIL12 was directly injected into the tumors in the left flanks at a dose of 4.0 x 10⁴ pfu/20 µL (mock, n=14; T-01, n=7; T-mfIL12, n=8). The tumors were measured twice a week and evaluated by calculating tumor volume as maximum diameter x shortest diameter x thickness (mm³). Error bar, SEM; *, p<0.05; **, p<0.01; ↑, virus administration date.

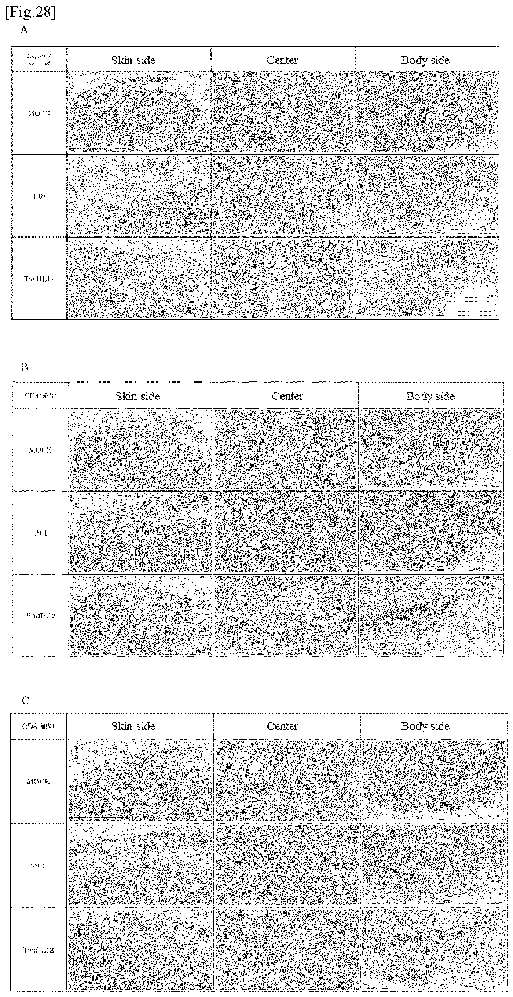

[Fig. 28] Immunohistochemical staining (A, negative control; B, CD4⁺ cells; C, CD8⁺ cells). Frozen sections were prepared from excised subcutaneous tumors and immunohistochemical staining was performed by using Rat Anti-Mouse CD4 antibody (B) and Rat Anti-Mouse CD8 antibody (C) as the primary antibody. The negative control was 2% BSA/PBS (A). Size bar, 1 mm.

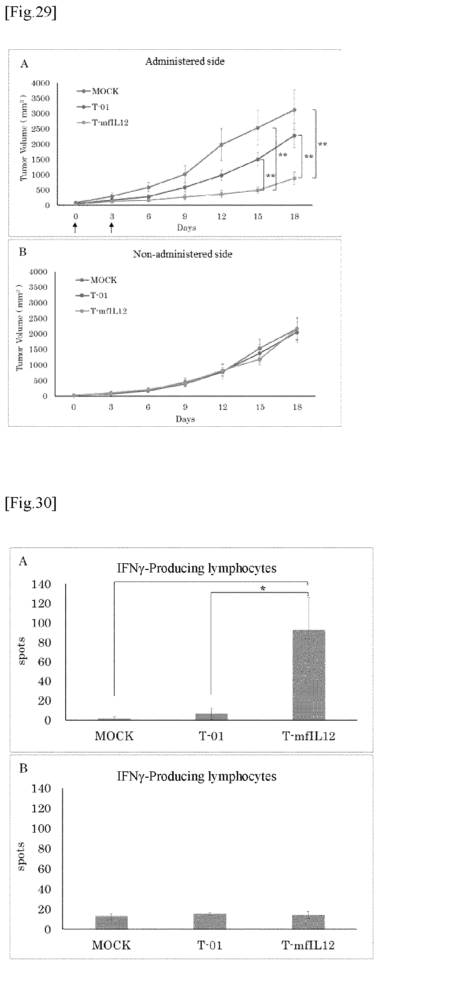

[Fig. 29] Treatment experiment using nude mouse bilateral subcutaneous tumor model. Nude mice were subcutaneously injected with 1.0 x 10⁶ cells/each mouse of malignant melanoma cell line Clone M3 into the right and left flanks to create subcutaneous tumors. The day on which the maximum tumor diameter reached approximately 5 mm was defined as day 0, and on days 0 and day 3, 4.0 x 10⁴ pfu/20 µL of T-01 or T-mflL12 was injected directly into the tumor in the left flank of each mouse (mock, n=10; T-01, n=8; T-mfIL12, n=9). Tumors were measured twice a week and evaluated by calculating tumor volume as maximal diameter x short diameter x thickness (mm³). Error bar, SEM; **, p<0.01; ↑, date of virus administration.
[Fig. 30] Analysis of IFNγ- or IL-4-producing splenic lymphocytes by ELISpot method. In a DBA/2 mouse Clone M3 subcutaneous tumor model, the day on which the maximum tumor diameter reached approximately 5 mm was defined as day 0, and on days 0 and day 3, 4.0 x 10⁴ pfu/20 µL of T-01 or T-mfIL12 was injected directly into the tumor of each mouse. On day 14, the mice were euthanized (n=3 for each group), and isolated splenic lymphocytes were used to measure IFNγ- and IL-4-producing lymphocytes by the ELISpot method. Clone M3 cells, a cell line derived from DBA/2 mice, were used for antigen stimulation. Error bar, SEM; *, p<0.05; **, p<0.01.

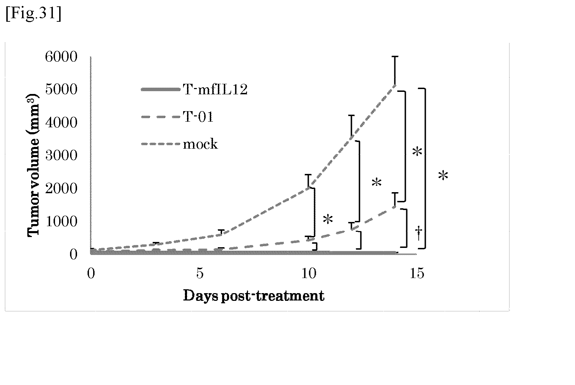

[Fig. 31] An F9 model was created by subcutaneously injecting a cell suspension of 5.0 x 10⁵ of F9, mouse NSGCT cells, in a total volume of 100 µL consisting of 50 µL DMEM medium and 50 µL BD Matrigel, into each of 129 male mice on the back. When the tumor diameter reached around 5 mm, 4.0 x 10⁴ pfu of T-01, 4.0 x 10⁴ pfu of T-mfIL12, or mock was intratumorally administered twice on day 0 and day 3. Significant tumor suppression effects were observed in both the T-01-treated group and the T-mflL 12-treated group compared with the mock-treated group, and the T-mfIL12-treated group showed significant tumor suppression also compared with the T-01-treated group (*, P<0.01; †. P<0.05). It was demonstrated that the antitumor effect of T-01 is further enhanced by IL-12 expression.

### Modes for Carrying out the Invention

The present invention relates to a recombinant herpes simplex virus (HSV) with IL-12 expression .

### (IL-12-expressing type)

The present invention relates to a recombinant HSV that expresses IL-12.

In one embodiment, the recombinant HSV is incorporated with a gene encoding interleukin 12 (IL-12). The gene to be incorporated may be configured in any way as long as it is capable of expressing IL-12 in the active form thereof (maintaining the functional tertiary structure). In one preferred embodiment, a gene encoding a fusion polypeptide in which the 35 kDa light chain (p35) and the 40 kDa heavy chain (p40) of IL-12 are linked via two or more elastin motifs is incorporated.

In one embodiment, the animal species from which p35 and p40 are derived is not particularly limited and can be any animal, such as human, mouse, rat, etc., but preferably, when administration to humans is intended, they are preferably derived from humans.

In one embodiment, the fusion polypeptide comprises p35, two or more elastin motifs, and p40, or p40, two or more elastin motifs, and p35, in this order from the N-terminus side. The number of elastin motifs is not particularly limited as long as the two subunits can be linked together, and active (maintaining functional tertiary structure) IL-12 can be expressed, and it may be, for example, 2, 3, 4, or 5. The number of elastin motifs is preferably 2. The linkage between the two subunits may contain several other amino acids in addition to repeats of the elastin motif (typically, VPGVG).

In one embodiment, the gene encoding the fusion polypeptide is any of the following polynucleotides:
(i) a polynucleotide consisting of the sequence of SEQ ID NO: 1;
(ii) a polynucleotide consisting of a sequence having a sequence identity of 90% or higher to the sequence of SEQ ID NO: 1 and encoding a polypeptide capable of forming active IL-12;
(iii) a polynucleotide encoding a polypeptide consisting of the sequence of SEQ ID NO: 2;
(iv) a polynucleotide encoding a polypeptide consisting of a sequence having a sequence identity of 90% or higher to the sequence of SEQ ID NO: 2 and capable of forming an active IL-12;
(v) a polynucleotide consisting of the sequence of SEQ ID NO: 3;
(vi) a polynucleotide consisting of a sequence having a sequence identity of 80% or higher, 90% or higher, 95% or higher, 97% or higher, 98% or higher, 99% or higher, 99.5% or higher, 99.7% or higher, 99.8% or higher, or 99.9% or higher to the sequence of SEQ ID NO: 3 and encoding a polypeptide capable of forming active IL-12;
(vii) a polynucleotide encoding a polypeptide consisting of the sequence of SEQ ID NO: 4; and
(viii) a polynucleotide encoding a polypeptide consisting of a sequence having a sequence identity of 80% or higher, 90% or higher, 95% or higher, 97% or higher, 98% or higher, 99% or higher, 99.5% or higher, 99.7% or higher, 99.8% or higher, or 99.9% or higher to the sequence of SEQ ID NO: 4 and capable of forming active IL-12.

Alternatively, in one embodiment, the fusion polypeptide is any of the following polypeptides:
(ix) a polypeptide encoded by a polynucleotide consisting of the sequence of SEQ ID NO: 1;
(x) a polypeptide encoded by a polynucleotide consisting of a sequence having a sequence identity of 90% or higher to the sequence of SEQ ID NO: 1 and capable of forming active IL-12;
(xi) a polypeptide consisting of the sequence of SEQ ID NO: 2;
(xii) a polypeptide consisting of a sequence having a sequence identity of 90% or higher to the sequence of SEQ ID NO: 2 and capable of forming active IL-12;
(xiii) a polypeptide encoded by a polynucleotide consisting of the sequence of SEQ ID NO: 3;
(xiv) a polypeptide encoded by a polynucleotide consisting of a sequence having a sequence identity of 80% or higher, 90% or higher, 95% or higher, 97% or higher, 98% or higher, 99% or higher, 99.5% or higher, 99.7% or higher, 99.8% or higher, or 99.9% or higher to the sequence of SEQ ID NO: 3 and capable of forming active IL-12;
(xv) a polypeptide consisting of the sequence of SEQ ID NO: 4; and (xvi) a polypeptide consisting of a sequence having a sequence identity of 80% or higher, 90% or higher, 95% or higher, 97% or higher, 98% or higher, 99% or higher, 99.5% or higher, 99.7% or higher, 99.8% or higher, or 99.9% or higher to the sequence of SEQ ID NO: 4 and capable of forming active IL-12.

The nucleotide and amino acid sequences for the polypeptide comprising p35 and p40 derived from mouse fused with two elastin motifs, which was used in the examples described below, are shown in Sequence Listing and Fig. 12.

The nucleotide and amino acid sequences for the polypeptide comprising p40 and p35 derived from human fused with two elastin motifs, which was used in the examples described below, are shown in Sequence Listing and Fig. 13.

An amino acid sequence having a high sequence identity can be expressed as an amino acid sequence derived from the original sequence by deletion, substitution, or addition of one or more amino acids. When an amino acid sequence derived by deletion, substitution, or addition of one or more amino acids is mentioned, the number of amino acids to be deleted, replaced or added is not particularly limited as long as the protein consisting of the amino acid sequence has the desired binding ability, but it may be, for example, 1 to 100, 1 to 50, 1 to 40, 1 to 30, 1 to 20, 1 to 10, 1 to 9, 1 to 5, 1 to 3, or 1 or 2. The position of the deletion, substitution or addition may be at the end or in middle of the peptide, and may consist of one or more positions. The amino acids may be natural amino acids, as well as derivatives thereof, artificial amino acids, and non-natural amino acids. In one embodiment, the amino acids are preferably natural amino acids.

For inserting a gene encoding a fusion polypeptide into HSV, known methods can be used. For example, DNA encoding IL-12 can be inserted into an expression vector by a known method (e.g., methods using restriction enzymes etc.), and the expression vector can be transfected into a recombinant HSV. The expression vector can further contain a promoter, which regulates the expression of the target gene, replication origin, selection marker gene, etc. The promoter and replication origin can be appropriately selected according to the types of recombinant HSV and vector into which the gene is introduced.

Genetic recombination in viruses with a large genome size, such as HSV, can be performed by using the T-BAC system described in Fukuhara, H. et al. (2005). Cancer Research, 65, 10663-10668 and WO2005/103237, or a similar system. When the T-BAC system is used for HSV, HSV capable of expressing the target IL-12 in tumor cells can conveniently created by a method comprising the first step of inserting a BAC plasmid having the loxP site, FRT site, and at least one expression cassette of a marker gene inserted between the loxP and FRT sites into the HSV genome, the second step of preparing a shuttle vector inserted with at least one type of expression cassette of a gene encoding a target protein, at least one type of marker gene, loxP site, and FRT site and inserting the shuttle vector into the loxP site of the HSV genome using Cre recombinase, and the third step of co-infecting a host with the HSV genome and a vector capable of expressing Flp recombinase, excising the region between the FRT sites on the genome, and producing the target recombinant HSV.

### (Genetic recombinant HSV)

In one embodiment, a gene encoding the fusion polypeptide is introduced into the recombinant HSV. Recombinant HSV means that it has been subjected to genetic recombination for use in treatments of tumors in addition to incorporation of a gene encoding IL-12.

In one embodiment, the recombinant HSV is a virus that can be used to treat tumors, and is not particularly limited so long as it has an ability to infect tumor cells, selectively replicate in the tumor cells, destroy the tumor cells in the process of viral replication, infect other surrounding tumor cells, and further replicate, and known viruses can be used. Many of such viruses are mutants of naturally occurring viruses that have been genetically modified to increase tumor selectivity. If necessary, they may be attenuated and modified (e.g., incorporation of suicide genes) to enhance antitumor activity.

For example, a recombinant HSV that can be used in one embodiment is a mutant of HSV-1 or HSV-2.

In one embodiment, the recombinant HSV is a mutant of HSV-1. HSV-1 is classified as an enveloped double-stranded DNA virus and has the following characteristics advantageous for oncolytic therapy: 1) it is infectious to all types of cells in human; 2) the life cycle and the genome sequence of the virus have been elucidated; 3) the functions of the majority of the viral genes have become clear and genetic manipulation can be applied; and 4) the viral genome is large (about 152 kb), and therefore a large gene or multiple genes can be incorporated. HSV-1 further has the following advantages suitable for clinical application: 5) it can kill all cells at a relatively low multiplicity of infection (MOI); 6) there are antiviral drugs to suppress proliferation thereof; 7) anti-HSV-1 antibodies in blood do not affect the spread of the virus infection from cell to cell, and therefore repeated administration thereof is possible; 8) preclinical evaluation of safety and effects can be performed in animals thanks to to the existence of mice and monkeys that are sensitive to HSV-1; and 9) the viral DNA is not incorporated into the genome of the host cell and is present outside the chromosome.

The genome of HSV-1 consists of two unique sequence regions: 82% of long unique region (U_{L}) and 12% of short unique region (U_{S}), as well as terminal repeat (TR) and inverted repeat (IR) locating at each end of U_{L} and U_{S} (Table 1, Todo, T. (2008). Frontiers in Bioscience a Journal and Virtual Library 13, 2060-2064). Since each of the two regions L and S can take two directions independently, the HSV-1 genomic DNA has four isomers. This genome contains, in total, 84 genes encoded unidirectionally in R_{L}1 and R_{L}2 on TR_{L}, U_{L}1 to U_{L}56 on U_{L}, R_{S}1 on TR_{S}, and U_{S}1 to U_{S}12 on U_{S}; about half of which are genes unnecessary for viral growth, and deletion of these non-essential gene moieties can reduce pathogenicity and allow gene transduction (Carson, J. et al. (2010). Drugs of the Future 35, 183-195).

**[Table 1]**

| 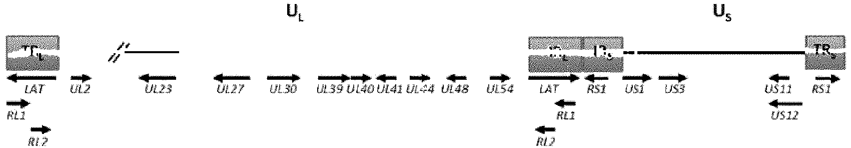 U_L ; U_S TR_L ; IR_L ; IR_S ; TR_S LAT ; UL2 ; UL23 ; UL27 ; UL30 ; UL39 ; UL40 ; UL41 ; UL44 ; UL48 ; UL54 ; LAT ; RS1 ; US1 ; US3 ; US11 ; RS1 RL1 ; RL1 ; US12 RL2 ; RL2 | | | |
|---|---|---|---|
| Gene | Essential (E) or non-essential (N) | Protein | Function |
| *LAT* | | LAT | Only gene that is expressed during latent infection |
| *RL1* | N | ICP34.5 | Neuropathogenicity, suppressing apoptosis |
| *RL2** | N | ICP0 | Promoting transcription |
| *UL2* | E | UNG | Encoding uracil-DNA glycosylase required for DNA synthesis |
| *UL23* | N | ICP36 (Thymidine kinase) | Encoding thymidine kinase required for DNA synthesis |
| *UL27* | E | gB | Invasion into cells |
| *UL30* | E | DNA polymerase | Catalytic subunit of DNA polymerase |
| *UL39* | N | ICP6 | Encoding large subunit of ribonucleotide reductase (RR) required for DNA synthesis |
| *UL40* | | | Encoding small subunit ofRR |
| *UL41* | N | vhc (virion4nduced host shutoff) | Degrading host mRNA |
| *UL44* | N | gC | Adsorption to cells |
| *UL48* | E | VPI6 | Promoting most early gene expression |
| *UL54** | E | ICP27 | Regulating DNA synthesis and transcription, suppressing expression of host proteins by suppressing splicing |
| *RS1** | E | ICP4 | Regulating β and γ genes. suppressing apoptosis |
| *US1** | N | ICP22 | Regulating transcription |
| *US3* | N | | Suppressing apoptosis, transferring capsid from nucleus to cytoplasm, regulating expression of gB |
| *US11* | N | | Inhibiting activation of protein kinase R under control by early promoter |
| *US12** | N | ICP47 | Inhibiting antigen presentation in host |
| * immed diate early genes | | | |

If the recombinant HSV is an HSV-1 mutant, it may have one or more of the following characteristics.
- Acquirement of tumor cell-specific replication ability due to inactivation of an enzyme involved in viral DNA synthesis, such as thymidine kinase (TK), ribonucleotide reductase (RR), and uracil-N-glycosylase (UNG or UDG).
- Acquirement of tumor cell-specific replication ability due to deletion of the gene γ34.5, which encodes ICP34.5, a protein involved in HSV-1 pathogenicity.
- Acquirement of anti-tumor effect due to deletion of α47.
- Deletion or inactivation of a gene for prevention of reversion to wild type to increase safety (e.g., deletion or inactivation of endogenous γ34.5 gene, α47 gene (ICP47 gene), α0 gene (ICP0 gene), U_{L}41 gene (vhs gene), and U_{L}56 gene).
- Enhancement of antitumor immunity and prolongation of survival by expression of an immune-stimulating gene (genes for IL-4, IL-10, GM-CSF, IL-12, soluble B7-1, etc.).
- Enhancement of antiangiogenic and antitumor effects by expression of a gene that expresses an angiogenesis inhibitory factor such as platelet factor 4, thrombospondin, endostatin, dominant-negative FGF, angiostatin, etc.
- Enhancement of antitumor effect by expression of an inhibitor for metalloproteinase involved in local invasion of tumors.
- Promotion of spread of viral infection by overexpression of metalloproteinases.
- Enhancement of tumor cell-specific viral replication ability by regulating a viral gene with a tumor or tissue-specific promoter (calponin promoter, E2F-responsive cell cycle-dependent promoter B-myb, Nestin promoter, carcinoembryonic antigen (CEA), α-fetoprotein (AFP), MUC-1, Musashi enhancer/promoter, etc.).
- Suppression of development of swelling by expression of a vascular endothelial growth factor (VEGF) antagonist.

In one embodiment, the recombinant HSV is preferably HSV-1 having one or more of the following characteristics (a) to (c), more preferably all of the following characteristics (a) to (c). HSV-1 mutants having these characteristics can be produced with reference to WO2011/101912, Patent document 2, etc.
(a) The ICP6 gene (UL39 gene) is deleted or inactivated, or expressed under the control of a tumor-specific or tissue-specific promoter
(b) The γ34.5 gene is deleted or inactivated.
(c) The α47 gene is deleted or inactivated.

With respect to the characteristics (a) and (b) mentioned above, deletion of ICP6, the large subunit of RR, which is the key enzyme for nucleotide metabolism and viral DNA synthesis in non-dividing cells, and/or γ34.5, which antagonizes the function of phosphorylated PKR that occurs during viral infection, results in a virus that cannot multiply in normal cells, but can multiply only in tumor cells that actively divide and show increased RR activity and suppressed phosphorylation of PKR. With respect to the characteristic (c) mentioned above, the protein encoded by the α47 gene has an ability to escape from immunosurveillance of the host by inhibiting TAP to suppress expression of MHC class I on the host cell surface, and therefore α47 gene-deleted HSV-1 is expected to give a stronger stimulation to immune cells due to maintenance of the MHC class I expression of the host cells. Further, in HSV-1 having both of the above characteristics (b) and (c), the US11 late promoter, which overlaps with the α47 gene on the genome, is simultaneously deleted with the deletion of α47, so that the expression of the US11 gene is placed under the control of the ICP47 immediate-early promoter, resulting in earlier expression of the gene, and therefore it comes to have an ability to restore the viral replication ability, which has been weakened by the deletion of γ34.5, only in tumor cells.

The term deletion or inactivation of a gene means deletion of all or a part of the gene, substitution or modification of some nucleotides of the gene, insertion of an unnecessary sequence into the gene, or the like to suppress expression of the gene, which can be carried out by known methods.

The term tumor-specific promoter or tissue-specific promoter means a promoter that enables expression of a gene that is under the control thereof specifically in a desired tumor cell or tissue, and known promoter can be used according to the gene. For example, the telomerase reverse transcriptase promoter (hTERT promoter) and E2F promoter can be used.

Examples of HSV of which γ34.5 gene, ICP6 gene and/or α47 gene are deleted or inactivated as described in the characteristics (a) to (c) mentioned above include G207 in which two copies of the γ34.5 gene are both deleted and the ICP6 gene is inactivated, and G47Δ in which two copies of the γ34.5 gene are deleted, the ICP6 gene is inactivated, and the α47 gene is deleted. Therefore, the recombinant HSV of one embodiment can also be produced by further modifying G207 or G47Δ.

In particular, G47Δ is an oncolytic HSV-1 having markedly improved tumor cell-selective viral replication ability and anti-tumor immunity induction ability in addition to the improved safety, and can also be safely intracerebrally administered to humans at high doses because of the broad therapeutic spectrum thereof. In June 2021, it was approved in Japan as a product for regenerative medicine (generic name, teserpaturev; product name, Delytact Injection) for the treatment of malignant glioma.

In one embodiment, the gene encoding the fusion polypeptide is inserted into the deleted ICP6 locus of G47Δ. That is, the gene is inserted at the site where the IPC6 gene has been deleted or inactivated.

The expression that the gene encoding the fusion polypeptide is inserted at the site where the IPC6 gene has been deleted or inactivated means, for example, that the virus has a sequence having a high identity to the sequence of SEQ ID NO: 7 mentioned in Sequence Listing.

As SEQ ID NO: 7 and in Fig. 14, the nucleotide sequence of the fusion polypeptide-encoding region insertion site and the upstream and downstream thereof in the virus produced in the experiments described in the section of Examples. In Fig. 14, the dotted line (1..226) indicates the sequence upstream of the U_{L}39 gene coding region reported to contain the U_{L}39 gene promoter, the underline (227..1544) indicates the amino acid coding region of the U_{L}39 gene, the wavy line (1546..1606) indicates the sequence resulting from the genetic recombination (Note 1: this sequence contains the loxP sequence (1546..1606)), the broken line (1607..4659) indicates the amino acid coding region of the lacZ gene, the following underline (660..4719) indicates the sequence derived from the plasmid vector (Note 2: sequence on the 3' side of lacZ derived from pcDNA6-E/Uni-lacZ), the dotted line (4726..4845) also indicates a sequence derived the plasmid vector (Note 2: sequence of SV40 polyA and sequence around BGH polyA derived from pVP22/myc-His2), TGA (4799..4801) is a termination codon of the ICP6-lacZ fusion protein, the double wavy line (4846..5010) indicates the SV40 polyA sequence derived from pVP22/myc-His2, the two-dot chain line (5011..5235) indicates the complementary sequence of BGH polyA derived from pVP22/myc-His2, the dotted line (5236..5330) indicates sequences derived from the plasmid vector (sequence of SV40 polyA and sequence around BGH polyA derived from pVP22/myc-His2), the following one-dot chain line (5342..5431) indicates 3' side complementary sequence of hIL12 cDNA, the following underline (5432..7042) indicates the amino acid coding region of the hIL12 gene (derived from pORF-hIL12 G2), the double line TTA (5432..5434) indicates the complementary sequence of the termination codon, complementary sequence of p35, complementary sequence of the hinge portion (bold dotted line, Location 6029..6058), complementary sequence of p40, and double line CAT (7040..7042) is complementary sequence of the start codon), the double line (7190..7697) indicates the complementary sequence of the CMV promoter, the wavy lines of the upstream and downstream thereof (7057..7189, 7698..7747) are sequences derived from the plasmid used (MCS), the two-dot chain line (7774..7799) indicates a sequence adjacent to the CMV promoter sequence of pVP22/myc-His2, the broken line (7800..7851) indicates a sequence generated at the time of the genetic recombination (synthetic oligomer sequence containing MCS incorporated in the production process of pLLZMF2 from pLLZF2), the wavy line (7852..7892) indicates the complementary sequences of the adapter sequence and FRT sequence (7857..7859) used in the genetic recombination, and the dotted line indicates the sequences of ICP6 on the 3' side downstream of the deletion site of 894 bp. *E*. *coli* β-galactosidase is expressed as a fusion protein with the N-terminus of ICP6.

The expression that the virus has the characteristic (b) mentioned above means that, for example, the virus has a sequence derived from the sequence of SEQ ID NO: 8 mentioned in Sequence Listing by deletion of all or a part of the region of the γ34.5 gene, substitution or modification of some nucleotides thereof, or insertion of an unnecessary sequence into the same, which results in suppression of the expression of the γ34.5 gene, or that the virus has a sequence derived from the sequence of SEQ ID NO: 8 by deletion of all or a part of the nucleotide sequence corresponding to the γ34.5 gene in IR_{L}, substitution or modification of some nucleotides thereof, or insertion of an unnecessary sequence into the same, which results in suppression of the expression of the γ34.5 gene. Ii is preferred that for the both γ34.5 gene sequences, all or parts of them are deleted, some nucleotides thereof are substituted or modified in them, or an unnecessary sequence is inserted into them, resulting in suppression of the expression of both genes.

As SEQ ID NO: 8 and in Fig. 15, a nucleotide sequence of the γ34.5 gene of a strain from which G47Δ is derived including upstream and downstream nucleotide sequences thereof (within the TR_{L} region) is shown. The sequence from the start codon to the termination codon shown with shading in Fig. 15 is the coding region of the γ34.5 gene. The sequence of the γ34.5 gene deletion site is underlined. For the downstream of the deletion site, the non-coding region on the 3' side of the γ34.5 gene is translated as a nonsense amino acid sequence until the termination codon (double underline) that occurs 130 bp downstream. The aforementioned sequence of TR_{L} (nucleotide numbers 487 to 1233 in GU734771.1) and the complementary sequence of the part of IR_{L} (nucleotide numbers 124965 to 125711 in GU734771.1) are completely identical.

The expression that the virus has the characteristic (c) mentioned above means that, for example, the virus has a sequence derived from the sequence of SEQ ID NO: 9 mentioned in Sequence Listing by deletion of all or a part of the region of the α47 gene, substitution or modification of some nucleotides thereof, or insertion of an unnecessary sequence into the same, which results in suppression of the expression of the α47 gene.

As SEQ ID NO: 9 and in Fig. 16, a nucleotide sequence of the α47 gene of a strain from which G47Δ is derived, including upstream and downstream sequences thereof, is shown. The α47 gene is encoded on the complementary strand side. The sequence of Location 218..484 in the sequence of SEQ ID NO: 9, or the sequence from the start codon to the termination codon indicated with shading in Fig. 16 is the coding region of the α47 gene. The sequence of the site deleted in the basic backbone of G47Δ is underlined in Fig. 16. It is deleted from the translation start site, and no new ORF is generated.

The recombinant HSV can be produced by referring to, for example, Patents documents 1 and 2, Non-patent document 1, and International Publication WO2005/103237.

### (Pharmaceutical composition)

In an embodiment, the recombinant HSV having a gene encoding a fusion polypeptide of the embodiment described above and having been subjected to genetic recombination for use in treating a tumor is an active ingredient of a pharmaceutical composition for treating a tumor. When such a pharmaceutical composition is administered to a subject, the recombinant HSV proliferates specifically in tumor cells and locally expresses IL-12 in the tumor whenever the virus replicates in the tumor.

Administration of the pharmaceutical composition according to one embodiment can be expected to suppress tumor growth with the immune effects of IL-12, more specifically, by promoting lymphocyte infiltration into the tumor, by inducing IFNγ production, or both, and to significantly increase the therapeutic effect of virus therapy. It can also be expected to dramatically increase the therapeutic effect for tumors for which increased immune cell infiltration is necessary or desirable, or for tumors with low immunogenicity or reduced immunogenicity, or tumors that are not easily recognized by the host immune system (immunologically cold tumors). Furthermore, the synergistic effect with the immune checkpoint inhibition therapy can be expected to significantly increase the therapeutic effect against both primary and metastatic tumors.

The administration of the pharmaceutical composition according to one embodiment is also expected to provide such statuses as mentioned below.
- IFNγ production is induced through IL-12 expression, but the activity of the virus, the active ingredient, is not suppressed and the replication ability thereof is not reduced.
- Systemic anti-tumor immunity is more favorably elicited through IL-12 expression and the effect is also obtained even in tumors not directly administered with the virus. It also more significantly reduces metastasis and the effect can also be obtained in metastasis lesions. The activity of cellular immunity to antigenic stimulation is enhanced.

It is also suggested that T cells are involved in the anti-tumor immune response induced by the pharmaceutical composition of one embodiment.

Examples of tumor for which application of the pharmaceutical composition of one embodiment is expected include, but are not limited to, brain tumor, glioma, ocular tumor, neuroblastoma, retinoblastoma, primary malignant lymphoma of CNS, meningioma, pituitary adenoma, schwannoma, craniopharyngioma, intraocular tumor, retinoblastoma, choroidal malignant melanoma, intraocular malignant lymphoma, tumor of ocular appendage, eyelid tumor, lacrimal gland cancer, ocular appendage lymphoma, orbital sarcoma, conjunctival tumor, optic nerve tumor, glioma, oral cavity cancer, tongue cancer, pharyngeal cancer, thyroid cancer, auricular cancer, adenoid cystic carcinoma, olfactory neuroblastoma, sarcoma of head and neck, lung cancer, breast cancer, thymoma, thymic carcinoma, malignant pleural mesothelioma, sarcoma of trunk, sarcoma of heart, pulmonary neuroendocrine tumor, SMARCA4-deficient thoracic tumor, non-small cell lung cancer, small cell lung cancer, esophageal cancer, stomach cancer, colorectal cancer (colon cancer and rectum cancer), small intestine cancer (duodenum cancer, jejunum cancer, and ileum cancer), GIST (gastrointestinal stromal tumor), pancreatic and gastrointestinal neuroendocrine tumors, anal cancer, gastrointestinal neuroendocrine tumor, hepatocellular cancer, biliary tract cancer (bile duct cancer (including intrahepatic bile duct cancer), gall bladder cancer, and papillary duodenal cancer), pancreatic cancer, pancreatic and gastrointestinal neuroendocrine tumors, liver tumor, pancreatic neuroendocrine tumor, renal cell carcinoma, renal pelvis and ureter cancers, bladder cancer, urachal carcinoma, renal tumor, prostate cancer, breast cancer, cervical cancer, uterine body cancer (endometrial cancer), uterine sarcoma, ovarian and oviduct cancers, vaginal cancer, vulvar cancer, malignant melanoma (skin), basal cell carcinoma, spinous cell carcinoma, lymphoma of skin, soft tissue sarcoma (adults), desmoid tumor, osteosarcoma (pediatric), Ewing's sarcoma (pediatric), soft tissue sarcoma (pediatric), rhabdomyosarcoma (pediatric), undifferentiated/unclassifiable sarcomas, osteosarcoma, chondrosarcoma, chordoma, Ewing's sarcoma, liposarcoma, fibrosarcoma, myxofibrosarcoma, undifferentiated pleomorphic sarcoma, leiomyosarcoma, rhabdomyosarcoma, hemangiosarcoma, malignant peripheral nerve sheath tumor, synovial sarcoma, epithelioid sarcoma, alveolar soft part sarcoma, clear cell sarcoma, extraskeletal Ewing's sarcoma, acute myeloid leukemia, acute lymphocytic leukemia/lymphoblastic lymphoma, chronic myeloid leukemia, myelodysplastic syndrome, malignant lymphoma, B cell lymphoma, T/NK cell lymphoma, Hodgkin lymphoma, lymphoma of skin, chronic lymphocytic leukemia/small lymphocytic lymphoma, adult T-cell leukemia lymphoma, multiple myeloma, acute promyelocytic leukemia, follicular lymphoma, MALT lymphoma, lymphoplasmacytic lymphoma, mantle cell lymphoma, diffuse large B-cell lymphoma, peripheral T-cell lymphoma, Burkitt's lymphoma, extranodal NK/T-cell lymphoma, cancer of unknown primary origin, hereditary and familial tumors, neuroendocrine cancer, germ cell tumor, paraganglioma, germ cell tumor, testicular germ cell tumor, ovarian germ cell tumor, extragonadal germ cell tumor, and neuroendocrine tumor. Examples of tumor for which the effect can be especially expected include neuroblastoma, prostate cancer, renal cancer, metastatic renal cancer, bladder cancer, metastatic bladder cancer, malignant melanoma, and non-seminomatous testicular tumor. For these, animal studies have confirmed that administration of the recombinant HSV with IL-12 expression exerts a remarkable anti-tumor effect.

From another aspect, examples of diseases for which the pharmaceutical composition of one embodiment is effective include cancers for which application of IL-12 has been approved or being considered, or immunotherapy is applied. Examples of such cancers include, but are not limited to, malignant melanoma, non-small cell lung cancer, renal cell carcinoma, Hodgkin lymphoma, head and neck cancer, stomach cancer, malignant pleural mesothelioma, esophageal cancer, MSI-High colorectal cancer, gastroesophageal junction cancer/esophageal cancer, small cell lung cancer, hepatocellular cancer, glioblastoma, urothelial carcinoma, ovarian cancer, bladder cancer, prostate cancer, cervical cancer, uterine body cancer, soft tissue sarcoma, primary lymphoma of CNS, primary testicular lymphoma, pancreatic cancer, biliary tract cancer,
radically unresectable malignant melanoma, unresectable advanced or recurrent non-small cell lung cancer, unresectable locally advanced non-small cell lung cancer (for maintenance therapy after radical chemoradiation), radically unresectable or metastatic renal cell carcinoma, curatively unresectable or metastatic renal cell carcinoma, recurrent or refractory classic Hodgkin lymphoma, recurrent head and neck cancer or head and neck cancer with distant metastasis, curatively unresectable advanced or recurrent stomach cancer exacerbated after cancer chemotherapy, unresectable advanced or recurrent malignant pleural mesothelioma exacerbated after cancer chemotherapy, curatively unresectable advanced or recurrent colorectal cancer with high frequency of microsatellite instability (MSI-High) exacerbated after cancer chemotherapy, radically unresectable advanced or recurrent esophageal cancer exacerbated after cancer chemotherapy, radically unresectable urothelial carcinoma exacerbated after cancer chemotherapy, advanced or recurrent solid tumors with high frequency of microsatellite instability (MSI-High) exacerbated after cancer chemotherapy (only when standard treatments are not feasible), radically unresectable or metastatic renal cell carcinoma, advanced small cell lung cancer, inoperable or recurrent breast cancer that is PD-L1-positive, hormone receptor-negative and HER2-negative, and radically unresectable Merkel cell carcinoma,

From another aspect, examples of tumors for which the pharmaceutical composition of one embodiment is effective include, for example, nerve system type tumors (e.g., astrocytoma, oligodendroglioma, meningioma, neurofibroma, glioblastoma, ependymoma, schwannoma, neurofibrosarcoma, and neuroblastoma), pituitary gland tumor (e.g., pituitary adenoma), medulloblastoma, melanoma, brain tumor, prostate cancer, head and neck cancer, esophageal cancer, renal cell cancer, pancreatic cancer, breast cancer, lung cancer, colon cancer, large bowel cancer, stomach cancer, skin cancer, ovarian cancer, bladder cancer, sarcoma (e.g., osteosarcoma, chondrosarcoma, rhabdomyosarcoma, leiomyosarcoma, fibrosarcoma, liposarcoma, and angiosarcoma), squamous cell carcinoma, neuroectoderm tumor, thyroid tumor, lymphoma, hepatocellular cancer, mesothelioma, epidermoid carcinoma, benign tumor (e.g., benign tumor of the thyroid gland or benign prostatic hyperplasia), etc., for which virus therapies are considered to be especially effective.

For the present invention and embodiments thereof, the term treatment referred to in relation to a disease or condition, it includes prevention, reduction of the risk of developing the disease, therapeutic treatment, inhibition of progression, and prevention of recurrence, unless especially noted.

The administration method of the pharmaceutical composition of one embodiment is not particularly limited and can be administered, for example, intravenously, intraarterially, intracerebroventricularly, intraperitoneally, intrathoracically, into the spinal cavity, subcutaneously, intradermally, intraepidermally, intramuscularly, or on mucosal surfaces (for example, eye, nasal cavity, lung, oral cavity, bowel, rectum, vagina, and urinary tract surfaces). It is preferably administered locally and directly to tumor tissues by injection, or endoscopic or surgical technique. If the pharmaceutical composition is locally administered, the risk of side effects is reduces compared with systemic administration of IL-12 because it does not make tissues other than the locally administered tissue virally positive and IL-12 is locally expressed in the tumor.

In another preferred embodiment, the pharmaceutical composition is intravenously administered. Intravenous administration can also be expected to deliver the virus to the tumor and exert the antitumor effect, with less obvious systemic toxicity and neurotoxicity due to intravenous administration. Furthermore, intravenous administration can be expected to exhibit the antitumor effect on systemic metastatic tumors.

The pharmaceutical composition of one embodiment can be formulated by known formulation methods for administering viruses into the body of mammals including humans. For example, the pharmaceutical composition may contain an adjuvant or an arbitrary carrier, or it may simply be diluted in a physiologically acceptable solution such as sterile physiological saline or sterile buffered physiological saline without adding any adjuvant or carrier. It may also be made as, for example, a frozen, dried, or lyophilized formulation suitable for long-term storage. It may also contain pharmaceutically acceptable additives.

The pharmaceutical composition of one embodiment contains a therapeutically effective amount of the recombinant HSV of one embodiment. The term therapeutically effective amount means an amount of the active substance with which one or more symptoms of the tumor to be treated are alleviated to some extent, more specifically, an amount that provides at least one of reduction in tumor size, inhibition (delay or arrest) of tumor metastasis, inhibition (delay or arrest) of tumor growth, and alleviation of one or more symptoms associated with the tumor. Specific doses may be appropriately determined by those skilled in the art according to the degree of symptoms, age, sex, weight, sensitivity difference of the target of administration, administration method, timing of administration, administration interval, nature of the preparation, strength of the promoter, etc.

The pharmaceutical composition of one embodiment can be administered in an amount of, for example, about 10¹ to 10¹² plaque-forming units (pfu), preferably about 10⁷ to 10¹⁰ pfu, more preferably about 10⁸ to 5 x 10⁹ pfu, which can be administered by one or several times of injection. The number of times of the administration of the pharmaceutical composition of one embodiment can be appropriately set according to the target patient and the target disease. For example, the first two doses can be administered within two weeks, and then the following doses can be administered at intervals of three to five weeks.

The pharmaceutical composition of one embodiment may further contain one or more other active ingredients or may be used in combination with a pharmaceutical composition comprising one or more other active ingredients. To use in combination means to administer multiple active ingredients or pharmaceutical compositions simultaneously, sequentially, or at intervals. Examples of those that can be combined include BCG (bovine attenuated tuberculosis), and IL-2/anti-IL-2 antibody complex. Such a combination therapy can provide a synergistic anti-tumor effect that cannot be obtained by administration of each alone. In addition, the combination therapy can infiltrate more CD4⁺ T cells and CD8⁺ T cells in the peritumoral area.

The pharmaceutical composition of one embodiment can also be used in combination with one or more other tumor treatment methods, such as surgery (tumor resection etc.), chemotherapy, radiation therapy, immunotherapy, hormone therapy, and combinations thereof. Since the viral therapy with the pharmaceutical composition of one embodiment is based on a mechanism different from those of existing tumor treatment methods, the tumor treatment effect can be enhanced by combining it with other treatment methods, such as radiation therapy and chemotherapy.

The pharmaceutical composition of one embodiment can be administered in combination as a plurality of HSVs, each expressing a different immune checkpoint inhibitory antibody. It can also be administered in combination with oncolytic HSV-1 with soluble B7-1 expression. When administered in combination, they may be administered as a mixture thereof or sequentially.

As another aspect, the present invention also relates to a method for treating a tumor using a virus having an additional function of expressing IL-12. The present invention also relates to use of a function-added recombinant HSV in manufacture of a medicament for treating a tumor. The method and use can be implemented with reference to the above descriptions for the pharmaceutical composition.

In a preferred embodiment, the pharmaceutical composition is used to enhance the effect of IL-12 in treatments of tumors. Administration of IL-12 to be virally expressed may act more effectively on local and systemic anti-tumor immunity compared with administration of IL-12 as a protein.

The present invention also provides a method for enhancing the effect of IL-12 in a treatment of a tumor, which comprises the step of administering a pharmaceutical composition comprising an oncolytic HSV transfected with an IL-12 gene to a target in need thereof.

### Examples

### [Example 1]

### (Methods)

### Cells

TRAMP-C2 cells were obtained from the American Type Culture Collection (ATCC, Rockville, USA). The cells were cultured in DMEM supplemented with 5% (v/v) fetal calf serum (FCS), 5% (v/v) Nu-serum IV and 10 nM dihydrotestosterone. Pr14-2 cells were kindly provided by Dr. Jeffery Green (NCI, Bethesda, USA) and were cultured in DMEM supplemented with 10% (v/v) FCS. Vero (African green monkey kidney) cells and Neuro2a cells were obtained from ATCC and maintained in DMEM supplemented with 10% (v/v) FCS and 2 mM glutamine.

### Virus construction

Conventionally, recombinant HSV-1s have been constructed by using homologous recombination techniques, but due to the large genome size of HSV-1, a time-consuming and laborious process of selecting and confirming the structure of the desired recombinant, which occurs with extremely low probability, was necessary. To eliminate this element of chance as much as possible, the inventors of the present invention previously constructed the G47Δ-BAC system using a bacterial artificial chromosome (BAC) and two recombinases to enable rapid and precise development of multiple armed oncolytic HSV-1 viruses in parallel, using G47Δ as the backbone (Non-patent document 31). However, the G47Δ-BAC product was later found to have lower replication ability than G47Δ. Presumably, this was due to an unknown mutation in the G47Δ-BAC plasmid. Therefore, the entire system was reconstructed with the same concept and improved by, for example, adding several useful cloning sites. This T-BAC system enables production of G47Δ-derived armed oncolytic HSV-1 with replication ability equivalent to that of G47Δ and a desired transgene.

First, the parent T-BAC virus was created by homologous recombination of G47Δ DNA with pBAC-ICP6EF, a plasmid containing the insertion sequence of the ICP6 coding region (see Non-patent document 31). Transfection was performed for Vero cells by using Lipofectamine (11668027, Thermo Fisher Scientific, USA) with 0.9 µg of DNA consisting of a 1:1:1 mixture of G47ΔDNA purified by the Na/I method, pBAC-ICP6EF (undigested), and pBAC-ICP6EF linearized by Asc I digestion according to the instructions of the manufacturer. Recombinant viruses forming GFP-positive plaques with 30 to 50% cytotoxic effect were selected and further subcultured in Vero cells. After selection of GFP-positive and lacZ-negative cells was repeated three times, the parent T-BAC virus was selected by screening several candidates that favorably replicated. This step is important because modified viruses have unexpected mutations that reduce viral yield. Circular viral DNA was separated from the infected Vero cells by the Hirt method and electroporated into *E. coli* DH10B (18290015, Thermo Fisher Scientific, USA). Antibiotic-resistant colonies were isolated, T-BAC plasmid DNA was purified, and the structure thereof was confirmed by endonuclease digestion.

Second, two kinds of plasmids were prepared to fill a mouse IL-12 expression cassette into a shuttle vector (pVec9). The fusion mouse IL-12 gene of T-mfIL12 (SEQ ID NO: 1) is derived from the pORF-mIL-12 plasmid (porf-mil12, InvivoGen, SanDiego, USA), in which the p35 and p40 subunits are linked with a bovine elastin motif. The fusion mouse IL-12 (SEQ ID NO: 2) is expressed as a single peptide comprising the p35 subunit having a signal sequence. The pVec9-fused-mIL12 plasmid was developed by using a plasmid having the mouse IL-12 p35 and p40 genes as one gene in the same reading frame. A 1.7 kb PCR fragment of pORF-mIL-12 (primers: 5'- GCTAGCCTGAGATCACCGCG-3' (SEQ ID NO: 5) and 5'-GCTAGCATCCGTTGCATCCTA-3' (SEQ ID NO: 6)) was digested with Nhe I and inserted into pVec9 at the Avr II site to create pVec9-fused-mIL12. Similarly, a single expression cassette separated with the IRES sequence from the 5' untranslated region of encephalomyocarditis virus (EMCV) was used to construct the pVec9-IRES-mIL12 plasmid. The IRES-type mouse IL-12 gene of T-mfIL12-IRES is derived from the plasmid pHCIL12-tk (Non-patent document 32). The constructs were confirmed by sequencing. A2.3 kb SpeI-StuI fragment containing the pIL-12-p40-IRES-p35 plasmid was inserted into pVec9 at the Avr II-Stu I site to create pVec9-IRES-mIL12.

Third, a mixture of T-BAC plasmid (1.5 µg) and pVec9-fused-mIL12 or pVec9-IRES-mIL12 (150 ng each) was incubated with Crerecombinase (M0298, NEB, USA) in a volume of 10 µl at 37°C for 30 minutes, and electroporated into *E. coli* DH10B. To select those containing the mutant BAC plasmid, the bacteria were streaked onto LB plates containing Cm (15 µg/ml) and Kan (10 µg/ml) and incubated overnight at 37°C. The DNA structure of the recombinant T-BAC/Vec9 plasmid was confirmed by gel analysis after endonuclease digestion. Transfection was performed on Vero cells using 2 µg of T-BAC/Vec9 DNA and 0.5 µg of pOG44 plasmid that expresses Flp (V600520, Thermo Fisher Scientific, USA) with 15 µl of Lipofectamine according to the manufacturer's instructions. The transfected cells were incubated overnight at 37°C in DMEM containing 10% (v/v) FCS, and on the next day the medium was replaced with DMEM containing 1% heat-inactivated fetal bovine serum (IFCS). Incubation was continued for several days until plaques appeared. Plaques GFP-negative under inverted fluorescence microscopy and lacZ-positive in X-gal staining were selected as progeny viruses. Limiting dilution was performed three times and one clone was selected. Recombinant viruses were collected and viral DNA structures were confirmed by endonuclease digestion. The nucleotide sequences of the transgene cassettes of the recombinant viruses were determined (primers: 5'-CGCAAATGGGCGTAGCGTG-3' (SEQ ID NO: 10), 5'-TAGAGGCACAGTCGAGG-3' (SEQ ID NO: 11), 5'-ACCCGCCCAAGAACTTGCAG-3' (SEQ ID NO: 12), and 5'-GGATCGACCCTGCAGGGAAC-3' (SEQ ID NO: 13)). The primers were designed to sequence the nucleotides between the CMV promoter and polyA. These viruses were individually titrated on Vero cells by plaque assay.

### Replication assay and cytotoxic effect test

For replication assay, Vero cells were seeded on a 6-well plate at a density of 3 x 10⁵ cells/well. The cells in the wells were infected with G47Δ, T-01, 4 clones of T-mflL12 or 4 clones of T-mIL12-IRES were infected at an MOI of 0.01 in 2 wells for each. Forty-eight hours after the infection, the cells were lysed by three cycles of freezing and thawing. Progeny viruses were measured on Vero cells by plaque assay as previously described (Non-patent document 6). In another experiment, Vero cells were infected with G47Δ, T-01, T-mfIL12 or T-mIL12-IRES at an MOI of 0.01 in two wells for each, and progeny viruses were collected at 0, 6, 24 and 48 hours after the infection and titrated by plaque assay. The results are shown as arithmetic means of the results of experiments performed in duplicate. The cytotoxic effect test was performed as described previously (Non-patent document 6). The cells were seeded on a 6-well plate and incubated overnight at 37°C. The cells were then inoculated with virus for 1 hour. The inoculum was removed and the cells were incubated in DMEM. The number of viable cells was counted daily on a Coulter counter (Z1 Single, Beckman Coulter, USA) and expressed as a percentage based on that observed in the mock-infected control.

### In vitro measurement of IL-12 expression

Vero, Neuro2a, PR14-2 or TRAMP-C2 cells were plated on a 24-well plate (1 x 10⁵ cells/well) and incubated at 37°C for 24 hours. The cells were infected with each virus at MOI=1 in two wells for each and further incubated at 39.5°C for 48 hours. All the viruses used in this study were derived from HSV-1 strain F and are therefore temperature-sensitive and do not replicate at 39.5°C (Non-patent document 5). The supernatants were collected and IL-12 concentrations were measured by using the Mouse IL12 p70 Immunoassay (M1270, R&D Systems Inc., IL). The detection limit was 7.8 pg/ml. The results are shown as arithmetic means of the results of experiments performed in duplicate. To separately detect the p35 and p40 subunits, ELISA kit for mouse IL12A (IL12 p35) (SEA059Mu, Cloud-Clone, TX) and ELISA kit for mouse IL12B (IL12 p40) (SEA058Mu, Cloud-Clone, TX) were used. The expression ratio of the p35 and p40 subunits was adjusted on the basis of that of the control (recombinant mouse IL-12, 095-05331, Wako, Japan).

### Interferon-γ release assay

Supernatants of Vero cells infected with T-mfIL12 or T-mIL12-IRES at an MOI = 1 were collected 48 hours after the infection. The VP-SFM medium (11681020, Thermo Fisher Scientific) was used for dilution. Spleens of A/J mice were collected and cell suspensions were prepared. The splenocytes were treated with the supernatants or recombinant mouse IL-12 (rIL12, 095-05331, Wako, Japan) for 48 hours, and interferon-γ (IFNγ) levels were measured in duplicate using Mouse IFNγ Uncoated ELISA Kit (88-7314, Thermo Fisher Scientific).

### Animal experiments

Six-week-old male C57BL/6 mice, female A/J mice, and female NOG (NOD/shi-scid.IL-2RγKO) mice were purchased from CLEA Japan (Tokyo, Japan). All the animals were caged in groups of no more than 5 animals each. Subcutaneous tumor therapy was performed as described previously (Non-patent document 31). Subcutaneous tumors were created by transplanting TRAMP-C2 prostate cancer cells (5 x 10⁶) into the left flanks of male C57BL/6 mice and Neuro2a neuroblastoma cells (5 x 10⁶) into the both flanks of female A/J mice or NOG mice. When the diameters of the tumors reached approximately 5 mm, 5 to 7 days after the transplantation, the animals were randomly divided into groups, blinded, and administered with 20 µl of mock, viral suspension or recombinant mouse IL-12p70 (#577004, BioLegend; rIL-12) into the left tumors. The administration was repeated after 3 or 4 days. Tumor growth was determined by measuring tumor volume (length x width x height x 0.52) twice per week. Mice were euthanized when the maximum tumor diameter reached 22 mm.

### Immunohistochemistry

Bilateral Neuro2a subcutaneous tumors were generated in A/J mice, and only the left tumor was inoculated with T-mfIL12, T-mIL12-IRES, T-01 (2 x 10⁵ pfu) or mock on days 0 and 3, and the tumors were harvested on day 6 (n=3 per group). The tumors were fixed overnight in 20% formaldehyde, embedded in paraffin and sectioned in a thickness of 4 µm. The sections were immunostained with anti-CD8 antibody (98941, CST, MA, USA), anti-CD4 antibody (25229, CST, MA, USA) or anti-HSV-1 antibody (ab9533, Abcam, Cambridge, UK) and developed with diaminobenzidine.

### In vivo IL-12 and IFNγ levels

In the unilateral subcutaneous Neuro2a model, T-01, T-mfIL12, T-mIL12-IRES (2 x 10⁶ pfu) or mock was inoculated into established tumors on day 0. Serum and tumor samples were collected from 3 mice per group on days 1, 3, and 6 and mouse IL12 and mouse IFNγ levels were measured in duplicate by using Mouse IL12 p70 Immunoassay (M1270, R&D Systems Inc., IL) and mouse IFNγ Uncoated ELISA (88-7314, Thermo Fisher Scientific). Tumor samples were also collected on day 0 immediately after the virus injection for the T-mfIL12 and T-mIL12-IRES-inoculated groups (n=4). The base IL-12 levels in mock group and virus suspensions were measured by ELISA. IL-12 was not detected in the mock and T-01-inoculated groups, and the amounts of IL-12 in 20 µl (2 x 10⁶ pfu) of T-mfIL12 and T-mIL12-IRES samples were calculated to be 157.3 pg and 5.2 pg, respectively.

### Enzyme-linked immunospot (ELISpot) assay

In a unilateral subcutaneous Neuro2a model, T-01, T-mfIL12, T-mIL12-IRES (5 x 10⁴ pfu) or mock was injected into tumors on days 0 and 3. On day 6, spleens were aseptically collected from 3 mice per group and cell suspensions were prepared. The splenocytes were co-cultured with Neuro2a cells or Sal/N cells derived from A/J mice (negative control) and used in the ELISpot assay using Mouse Interferon-γ ELISpot^{PLUS} (3321-4HPW-2, MabtechAB, Sweden) and Mouse interleukin-4 (mIL-4) ELISpot^{PLUS} (3311-4HPW-2, MabtechAB, Sweden). The assay was performed in triplicate.

### Statistics and reproducibility

Data comparisons among the treatment groups were performed by using Student's t-test, Tukey's one-way analysis of variance with multiple comparison tests, or Bonferroni's two-way analysis of variance with multiple comparison tests. The results of the survival test were analyzed by Kaplan-Meier analysis. Values of P<0.05 were considered statistically significant. All the statistical analyses were performed by using JMP Proversion 11.0.0 (SAS Institute, USA). The experiments were performed in duplicate for replication assay, cytotoxic effect study, and in vitro expression measurement. For in vivo IL-12 and IFNγ level measurements, tumor samples were collected from 3 mice per group. For the in vivo animal experiments, 9 to 12 mice per group were used. For IFNγ and IL-4 ELISpot assays, three mice per group were treated and the assays were performed in triplicate.

### (Results)

### Construction of T-mfIL12 and T-mIL12-IRES

The inventors of the present invention previously constructed oncolytic HSV-1 armed with mouse interleukin 18 and soluble mouse B7-1 using a bacterial artificial chromosome (BAC) and two recombinase systems (Cre/loxP and Flp/FRT) with G47Δ as the backbone (Non-patent document 29). Later, they reconstructed this system with improvements (T-BAC system) to produce G47Δ-derived armed oncolytic HSV-1 with improved replication ability and a desired gene. By using this T-BAC system with two different mouse IL-12 expression cassettes, two IL-12-expressing oncolytic HSV-1s, T-mfIL12 and T-mIL12-IRES, were created. Each virus has deletion of 894 bp in the ICP6 gene, where the expression cassette is inserted, as well as deletions of both copies of the γ34.5 gene and deletion of the α47 gene (Fig. 1). Downstream of the ICP6 promoter, the lacZ gene was placed and the expression cassette controlled by the CMV promoter in the opposite direction. The size of the mouse IL-12 gene in T-mflL12 from the ATG to the stop codon is 1,620 bp, and since the upstream p35 gene is linked to the downstream p40 gene, mouse IL-12 is expressed as a fusion protein. The mouse IL-12 gene in T-mIL12-IRES contains the 1,008 bp p40 subunit and the 648 bp p35 subunit downstream and upstream of the IRES sequence, respectively, and the two subunits are expressed simultaneously but separately. The control virus, T-01, was constructed by using a shuttle vector for an empty cassette. When these three viruses were constructed in parallel, 99% or more of the viral plaques formed after the recombination were lacZ expression-positive. Four clones of each recombinant HSV-1 were isolated by three times of limiting dilution, and the constructs were confirmed by restriction enzyme digestion (Fig. 7). It was confirmed that, in the transgene cassettes of T-mfIL12 and T-mIL12-IRES, the p35 and p40 subunit sequences of T-mflL12 were identical to those of T-mIL12-IRES (Fig. 12).

### In vitro characterization of T-mfIL12 and T-mIL12-IRES

To evaluate the replication ability of the two kinds of viruses expressing IL-12, the number of progeny viruses recovered from Vero cells (3 x 10⁵ cells per well) was measured 48 hours after infection at an MOI of 0.01 (Fig. 2, a). The arithmetic means of the viral yields (± SD) obtained from the 4 clones of T-mfIL12 and 4 clones of T-mIL12-IRES were 6.8 x 10⁶ (± 0.59 x 10⁶) and 7.0 x 10⁶ (± 0.94 x 10⁶) pfu, respectively (p= 0.736, t-test). These yields were comparable to that of the control virus, T-01 (7.6 x 10⁶ pfu), but the parent virus, G47Δ, showed a higher yield (2.0 x 10⁷ pfu). The expression level of mouse IL-12 was assessed by measuring its concentration in the supernatant of Vero cells 48 hours after infection at an MOI of 1 using ELISA (Fig. 2, b). This ELISA specifically detects the functional form of mouse IL-12 (p70). The arithmetic means (± SD) of IL-12 concentrations for 4 clones of T-mfIL12 and 4 clones of T-mIL12-IRES were 10.0 ± 0.18 and 1.5 ± 0.39 ng/mL (p<0.001, t-test), respectively, that is, the concentration obtained with the clones of T-mfIL12 expressing the p70 form of IL-12 was almost 7-fold higher than that obtained with the clones of T-mIL12-IRES. The first clones identified from the respective recombinant G47Δ were used for further analysis. The corrected expression ratios of the p35 and p40 subunits were 1.8:1 and 10:1 for T-mfIL12 and T-mIL12-IRES, respectively. The time course (0, 6, 24 and 48 hours) of viral yield of T-mfIL12 in Vero cells was comparable to that of T-mIL12-IRES (Fig. 2, c). Both IL-12 expressed from T-mfIL12 and T-mIL12-IRES stimulated splenocytes to induce release of IFNγ, confirming the biological activities thereof (Fig. 8).

The in vitro cytotoxic effects of T-mfIL12 and T-mIL12-IRES were evaluated by using the mouse neuroblastoma cell line Neuro2a and the mouse prostate cancer cell lines Pr14-2 and TRAMP-C2. In all the three mouse cancer cell lines, both T-mflL12 and T-mIL12-IRES showed cytotoxic effects comparable to that of the control virus T-01 at both MOIs of 0.1 and 1 (Fig. 9). Expression of mouse IL-12 from T-mflL12 and T-mIL12-IRES was further evaluated in the three cell lines. Concentrations in the supernatants were measured by ELISA 48 hour after infection at an MOI of 1 (Fig. 2, d). In all the three cell lines, T-mfIL12 showed higher expression of mouse IL-12 than T-mIL12-IRES.

### In vivo efficacy of T-mfIL12 and T-mIL12-IRES

The in vivo efficacy of the two kinds of IL-12 expressing viruses was evaluated in two types of immunodeficient mouse tumor models, TRAMP-C2 tumors in allogeneic C57BL/6 mice and Neuro2a tumors in allogeneic A/J mice (Figs. 4, a and b, Fig. 9). When the established subcutaneous TRAMP-C2 tumors reached approximately 5 mm in diameter, T-01, T-mfIL12, T-mIL12-IRES (5 x 10⁶ pfu) or mock was intratumorally inoculated twice on days 0 and 3. C57BL/6 mice and the TRAMP-C2 cells derived from C57BL/6 are relatively resistant to HSV-1 infection (Non-patent document 33). The dose (5 x 10⁶ pfu) was determined on the basis of the results of the previous study of the inventors of the present invention (Non-patent document 9). In this TRAMP-C2 model, T-mflL12 treatment showed the highest efficacy, with significantly smaller tumors than those obtained with the T-01 and T-mIL12-IRES treatments on day 25 (p=0.003 and p=0.029 vs T-01 and T-mIL12-IRES treatments, respectively, t-test; Fig. 4, a). Among the groups, all the three viruses were significantly more effective compared with the mock (p<0.001 for all the three viruses, two-way ANOVA), but only T-mfIL12 was significantly more effective compared with T-01 (p=0.027, two-way ANOVA; Fig. 4, a) and T-mIL12- IRES was not significantly different from T-01.

Neuro2a cells with low immunogenicity were transplanted into the bilateral flanks of A/J mice. When the established Neuro2a subcutaneous tumors reached approximately 5 mm in diameter, T-01, T-mfIL12, T-mIL12-IRES (5 x 10⁴ pfu) or mock was inoculated twice on days 0 and 3 into only the left tumor. A/J mice and the A/J-derived Neuro2a cells are susceptible to HSV-1 infection (Non-patent document 33). The dose (5 x 10⁴ pfu) was determined on the basis of experience with this model (Non-patent document 20). Both T-mfIL12 and T-mIL12-IRES caused significant tumor growth inhibition compared with T-01 on the treated side on day 11 (p<0.001 and p=0.016 for T-mfIL12 and T-mIL12-IRES, respectively, t test) and untreated side on day 11 (p<0.001 and p=0.018 for T-mfIL12 and T-mIL12-IRES, respectively, t-test; Fig. 4, b). Among the groups, all three viruses were significantly more effective compared with the mock on the treated side (p=0.001 for T-01 and p<0.001 for both T-mflL12 and T-mIL12-IRES; two-way analysis of variance), and on the untreated side, T-mfIL12 and T-mIL12-IRES were more effective compared with the mock (p<0.001 for both viruses, two-way analysis of variance, Fig. 4, B). Both T-mflL12 and T-mIL12-IRES were significantly more effective also compared with T-01 on the treated side (p<0.001 and p=0.006 for T-mfIL12 and T-mIL12-IRES, respectively, two-way ANOVA), and on the untreated side, they were similarly significantly more effective compared with T-01 (p<0.001 and p=0.011 for T-mfIL12 and T-mIL12-IRES, respectively, two-way ANOVA). In addition, T-mfIL12 caused more significant tumor growth inhibition compared with T-mIL12-IRES on both treated and untreated sides on day 13 (p=0.043 and p=0.040 for treated and untreated sides, respectively, t-test, Fig. 4, b). Between the groups, T-mfIL12 was more effective compared with T-mIL12-IRES on the untreated side (p=0.018, two-way ANOVA; Fig. 4, b). The data on tumor growth in individual animals are shown in Fig. 9.

To confirm that the enhanced efficacy of T-mflL 12 and T-mIL12-IRES compared with T-01 was due to the immune effects of virally expressed IL-12, efficacy was further evaluated in NOG mice, in which the effects of adaptive immunity as well as NK activity were excluded (Fig. 4, c). When the established bilateral Neuro2a subcutaneous tumors reached approximately 5 mm in diameter, T-01, T-mfIL12, T-mIL12-IRES (1 x 10⁶ pfu) or mock was inoculated into only the left tumors on days 0 and 3. On the treated side, all the three viruses showed more significant efficacy compared with the mock (p<0.001 vs mock for all the viruses, two-way analysis of variance). However, the effect of IL-12 expression was completely abolished in NOG mice for both T-mfIL12 and T-mIL12-IRES, and there was no difference in efficacy among the three viruses T-01, T-mfIL12 and T-mIL12-IRES. All the three viruses had no effect on the contralateral untreated tumors (Fig. 4, c).

The efficacy was further evaluated on the basis of the survival rate of A/J mice with bilateral Neuro2a subcutaneous tumors. When established Neuro2a subcutaneous tumors reached approximately 5 mm in diameter, T-01, T-mfIL12, T-mIL12-IRES or mock was inoculated into the left tumors on days 0 and 3, and mice were euthanized when one tumor reached 22 mm in diameter. In separate experiments, two doses, 5 x 10⁵ pfu and 5 x 10⁴ pfu, were used. When the dose was 5 x 10⁵ pfu, both T-mfIL12 and T-mIL12-IRES significantly prolonged the survival time of the tumor-bearing mice compared with T-01 (p=0.023 and p=0.002 for T-mfIL12 and T-mIL12-IRES, respectively, log-rank test; Fig. 4, d). However, survival was confirmed on day 60 only for the T-mfIL 12-treated group (2/11). When the dose was 5 x 10⁴ pfu, both T-mfIL12 and T-mIL12-IRES significantly prolonged survival time of the tumor-bearing mice compared with T-01 (p=0.015 and p=0.046 for T-mfIL12 and T-mIL12-IRES, respectively, log-rank test, Fig. 4, e, Supplementary Data 4). There were 1 or 2 T-mfIL12-treated mice that showed long-term survival with the both doses, but no significant difference was observed between T-mflL12 and T-mIL12-IRES (p=0.974 for 5x 10⁵ pfu [Fig. 4, d] and p=0.265 for 5 x 10⁴ pfu [Fig. 4, e], log-rank test). In total, the experimental results show that T-mfIL12 exhibits higher in vivo efficacy than T-mIL12-IRES.

Further, whether viruses that express IL-12 promote lymphocyte infiltration into tumors was immunohistochemically evaluated. Bilateral Neuro2a subcutaneous tumors were created in A/J mice, T-mfIL12, T-mIL12-IRES, T-01 (2 x 10⁵ pfu) or mock was inoculated into only the left tumors on days 0 and 3, and tumors were collected on day 6. Within the tumors, increased infiltration of CD4⁺ and CD8⁺ lymphocytes was observed for all the three viruses on both the treated and untreated sides, more pronounced for T-mfIL12 and T-mIL12-IRES than for T-01 (Fig. 5, a). While HSV-1-positive cells were observed within the tumors for all the viruses on the treated side, none of the viruses made the cells HSV-1 positive on the untreated side (Fig. 5, a).

### In vivo levels of IL-12 and IFNγ induced by T-mfIL12 and T-mIL12-IRES

To evaluate in vivo gene expression and function induced by T-mfIL12 and T-mIL12-IRES, in vivo IL-12 and IFNγ levels were measured in a unilateral Neuro2a subcutaneous tumor model. Established tumors were inoculated with T-01, T-mfIL12, T-mIL12-IRES (2 x 10⁶ pfu) or mock, and serum and tumor samples were collected on days 1, 3 and 6 (n=3 per group). In the tumors, the IL-12 levels peaked on day 1, measured to be 2590 ± 1450 pg/ml and 228 ± 115 pg/ml for T-mfIL12 and T-mIL12-IRES, respectively (mean ± SD), and both IL-12 levels gradually decreased by days 3 and 6 (Fig. 5, b). The IL-12 levels in the tumors on day 0, immediately after the viral injection, were 7.7 ± 9.9 pg/ml for T-mfIL12 and undetectable for T-mIL12-IRES, indicating that IL-12 detected on day 1 and thereafter was due to viral expression of IL-12. The IL12 levels in the tumors of the T-mflL12-inoculated group were significantly higher than those of the T-mIL12-IRES-inoculated group at all time points (p=0.018, p=0.016 and p=0.046 on days 1, 3 and 6, respectively, one-way analysis of variance). The IL-12 levels detected in sera were significantly lower (approximately 2 log lower) than the levels in the tumors. Correlating with intratumoral IL-12, serum IL-12 levels of the T-mfIL 12-inoculated group were higher than those of the T-mIL12-IRES-inoculated group on day 1 (p=0.047, one-way analysis of variance). IFNγ was detected in the tumors from day 1 for all the three viruses and increased by day 3 (Fig. 5, b). IFNγ was detected in serum only with the viruses expressing IL-12 and peaked on day 1. IFNγ levels of the T-mflL12-inoculated group were significantly higher than those of the T-mIL12-IRES-inoculated group in both tumor and serum on day 1 (p=0.014 and p=0.027 for tumor and serum, respectively, one-way analysis of variance)

### Specific anti-tumor immune responses to T-mfIL12 and T-mIL12-IRES

To evaluate the specific anti-tumor immune responses elicited by T-mfIL12 and T-mIL12-IRES, the ELISpot assay was performed in a unilateral Neuro2a subcutaneous tumor model. Established tumors were inoculated with T-01, T-mfIL12, T-mIL12-IRES (5 x 10⁴ pfu) or mock on days 0 and 3, and spleens were extracted on day 6. ELISpot assay revealed that splenocytes from the T-mfIL12-treated mice showed release of IFNγ stimulated by the Neuro2a cells in a more significantly larger number compared with those of the T-01- and T-mIL12-IRES-treated mice (p=0.005 and p=0.004 vs T-01 and T-mIL12-IRES, respectively, one-way analysis of variance; Fig. 5, c). Such a response was not observed in SaI/N cells, control cells derived from the A/J mouse strain (Fig. 10). No significant differences were observed in the number of IL-4-releasing splenocytes among the three virus-treated groups (Fig. 5, c, and Fig. 11).

### Comparison of in vivo efficacies of T-mfIL12 and intratumoral recombinant IL-12 administration

To evaluate the benefit of IL-12 expression from oncolytic HSV-1, the efficacy of T-mfIL12 was compared with that of direct intratumoral injection of recombinant mouse IL-12 (rIL-12) in A/J mice with bilateral Neuro2a subcutaneous tumors (Fig. 6). It has been reported that intratumoral injection of 500 ng of rIL-12 in immunodeficient mice with subcutaneous tumors of low immunogenicity caused tumor growth inhibition (Non-patent document 34). From the above experiment shown in Fig. 5, b, the highest intratumoral IL-12 level attained by intratumoral injection of 2 x 10⁶ pfu of T-mfIL12 was calculated to be 45.7 ng/tumor (30.1 ± 9.6, mean ± SEM). Furthermore, when Neuro2a subcutaneous tumors were intratumorally injected with 5 x 10⁴ pfu of T-mfIL12, the highest intratumoral IL-12 level was measured to be 0.668 ng/tumor (0.238 ± 0.147, n=4). Therefore, three different doses of rIL-12, 500 ng, 50 ng and 1 ng, were tested. When established tumors reached approximately 5 mm in diameter, rIL-12, T-01 (5 x 10⁴ pfu), T-01 (5 x 10⁴ pfu) plus rIL-12, T-mflL12 (5 x 10⁴ pfu) or mock was inoculated on days 0 and 4 into only the left tumors (n=10 per group). When the dose of rIL-12 was 500 ng, rIL-12, T-01, T-01+rIL-12 and T-mflL12 were all significantly more effective compared with the mock on the treated side (p<0.001 vs mock for all, two-way analysis of variance, Fig. 6, a). On the untreated side, rIL-12 alone did not show significant antitumor effect compared with the mock, while T-01+rIL-12 and T-mfIL12 were significantly more effective compared with the mock (p=0.039 and p<0.001 vs mock, respectively, two-way ANOVA). Furthermore, on the untreated side, T-mfIL12 was significantly more effective compared with rIL-12 alone (p=0.003, two-way analysis of variance), while T-01+rIL-12 was not significantly different from rIL-12 alone. Similar results were obtained when the dose of 50 ng was used for rIL-12 (Fig. 6, b). When the dose of rIL-12 was 1 ng, which is the dose representing the IL-12 level in the tumor treated with T-mfIL12 used in this experiment, rIL-12 alone was not significantly effective on both the treated and untreated sides, and T-mflL12 was significantly more effective compared with rIL-12 alone on the treated side (p<0.001, two-way analysis of variance, Fig. 6, c), but T-01+rIL-12 did not show significant difference compared with rIL-12 alone. These results indicate that virally expressed IL-12 is more effective compared with recombinant IL-12 on local and systemic antitumor immunity.

### (Discussion)

In this study, the efficacies of two types of armed oncolytic HSV-1s that express the same mouse IL-12 molecule in different ways but are controlled by the same very early CMV promoter were compared. T-mfIL12, which expresses mouse IL-12 as a single active fusion peptide in which the p35 and p40 subunits are linked via bovine elastin motif, while T-mIL12-IRES co-expresses the p35 and p40 subunits, with the IRES sequence inserted between the two subunit genes. The inventors of the present invention showed that fusion type expression produces IL-12 significantly more efficiently than co-expression of the two subunits in various cell lines, while the expression method does not affect the replication ability and cytotoxic effect of armed oncolytic HSV-1. T-mfIL12 and T-mIL12-IRES both show significantly increased efficacy compared with the control virus, T-01, but T-mfIL12 shows significantly higher efficacy than T-mfIL12-IRES in vivo in different two allogenic mouse tumor models, reflecting that T-mfIL12 expresses a higher amount of active IL-12 compared with T-mIL12-IRES. The function of virus-expressed IL-12 is confirmed to be immune-mediated, as the enhanced efficacy disappears in NOG mice. As evidenced by IFNγ measurement, ELISpot assay, and immunohistochemistry, in the less immunogenic Neuro2a tumor model of HSV-1 susceptible A/J mice, a significantly higher amount of IL-12 is detected in the T-mfIL12-treated tumors than in the T-mIL12-IRES-treated tumors, leading to an enhanced anti-tumor immune response. This tumor model was used in this study because it appears to be one of the best models for assessing the function of immunomodulatory payload of the oncolytic HSV-1.

In addition to direct cell killing, oncolytic HSV-1 elicits a tumor-specific immune response (Non-patent documents 35 to 37). One approach to increase the efficacy of oncolytic HSV-1 is to arm the virus with immunostimulatory molecules. Several studies evaluated different immunostimulatory transgenes carried on the same oncolytic HSV-1 backbone. In mice with subcutaneous squamous cell carcinoma, oncolytic HSV-1 that expresses mouse IL-12 (NV1042) showed higher effect compared with one that expresses mouse GM-CSF (NV1034) (Non-patent document 19). The mice administered with NV1042 rejected a higher percentage of rechallenged tumor cells compared with mice administered with NV1034 (Non-patent document 19). In two mouse prostate cancer models, NV1042 was more effective compared with control HSV-1 (Non-patent document 38), whereas NV1034 did not show significant enhancement of the effect. The inventors of the present invention prepared three types of oncolytic HSV-1s armed with mouse soluble B7-1, mouse IL-12, or mouse IL-18 (referred to as vHsv-B7-1-Ig, vHsv-IL-12 and vHsv-IL-18, respectively) by using the same HSV-1 backbone in which the γ34.5 and ICP6 genes were deleted (Non-patent document 20). Combination of the three types of HSV-1s, tvHsv-B7.1-Ig, vHsv-IL-12, and vHsv-IL-18, was more effective in A/J mice with Neuro2a subcutaneous tumors than the single virus or combination of two viruses, but vHsv-IL-12 alone was the most effective among the three types of viruses. These studies indicate that IL-12 is so far a good choice as a transgene when arming oncolytic HSV-1 with a single immunostimulatory inserted gene (transgene). Systemic administration of IL-12 has been shown to cause severe toxicity, including death, in clinical trials (Non-patent documents 39 and 40). Therefore, local expression of IL-12 using oncolytic HSV-1 as a distribution tool is a reasonable approach from the aspect of safety (Non-patent document 41). Several clinical trials of IL-12 gene therapy using adenovirus vectors have recently been conducted (Non-patent documents 42 and 43).

In addition to the above, IL-12 is being tested as a transgene to arm other oncolytic HSV-1 for preclinical as well as clinical purposes. G47Δ-mIL12, which has the mouse IL-12 gene in the G47Δ backbone, like T-mfIL12, was created by using the G47Δ-BAC system previously described by the inventors of the resent invention (Non-patent documents 21 and 31). In an immunodeficient mouse glioblastoma stem cell model, G47Δ-mIL12 attacked mouse glioblastoma cells to increase IFNγ release, resulting in inhibition of angiogenesis, and reduced number of regulatory T cells in the tumor (Non-patent document 21). The anti-tumor effect of the combination of G47Δ-mIL12 with anti-PD-1 and anti-CTLA-4 antibodies was dependent on CD4⁺ and CD8⁺ T cells and macrophages (Non-patent documents 44, 45, and 46). While temozolomide chemotherapy antagonizes G47Δ-mIL12, the combination of G47Δ-mIL12 with systemic VEGFR tyrosine kinase inhibitor (Non-patent document 47) or G47Δ that expresses angiostatin (Non-patent document 48) increased the efficacy in a mouse glioblastoma model. In addition, M002, a first-generation γ34.5-deleted oncolytic HSV-1 armed with mouse IL-12, showed higher efficacy than the parent virus and G207 in preclinical brain tumor models (Non-patent documents 22 and 49). Subsequently, for the purpose of clinical trials in patients with high-grade glioma, M032 that expresses human IL-12, not mouse IL-12, was constructed by using the same backbone and strategy as those of M002 (Non-patent document 50). Safety of intracerebral administration of M032 (up to 10⁸ pfu) was confirmed in a study using non-human primates (Non-patent document 51). M032 has been used in a phase I study in patients with recurrent malignant glioma (NCT02062827). There have also been reported a method of inserting the human IL-12 gene into the HSV-1 backbone lacking ICP47 and ICP34.5 (Δ47/Δ34.5/IL12, Non-patent document 52) and a method of allowing mouse IL-12 (R-115) to act on fully-virulent HER2-retargeted oncolytic HSV-1 (Non-patent documents 53 and 54).

NV1042 and G47Δ-mIL12 use mouse IL-12 genes that are expressed as a single fusion protein (Non-patent documents 19 and 21), while M002, M032, Δ47/Δ34.5/IL12 and R-115 use mouse or human IL2 genes of which p35 and p40 subunit genes are separated with IRES, and co-expressed as two proteins under the control of the early growth response-1 promoter or CMV promoter. Because IL-12 is species-specific, in order to initiate clinical trials after obtaining preclinical data with oncolytic HSV-1 armed with mouse IL-12, it is necessary to newly construct a similar oncolytic HSV-1 armed with human IL-12. Since clinical development is time, labor, and money-consuming, it is difficult to backtrack or change course once it progresses. Therefore, it is important to consider whether the oncolytic virus to be clinically developed is optimal in all aspects, including those of safety, efficacy, manufacturing, stability, clinical utility, regulatory hurdles, environmental impact, etc. In this study, as one of such aspects, the optimal way to express IL-12 as a payload of oncolytic HSV-1 was evaluated. As a result, it was found that expressing the IL-12 gene as a single fusion peptide is significantly better than co-expressing the two subunits. As a result of this study, by using the T-BAC system, an improved version of the G47Δ-BAC system (Non-patent document 31) that allows rapid and accurate insertion of a desired transgene into the deleted ICP6 locus of G47Δ, G47Δ-derived oncolytic HSV-1 that expresses human IL-12 as a functional fusion peptide (T-hfIL12) has been successfully created. The fusion type human IL-12 expressed by T-hfIL12 (the nucleotide sequence is shown as SEQ ID NO: 3, and the amino acid sequence is shown as SEQ ID NO: 4) comprises the p40 subunit and p35 subunit linked via an elastin motif. T-hIL12 has been fully characterized, including the functions of expressed human IL-12, and is currently being used in an ongoing physician-initiated phase 1/2 clinical trials (jRCT2033190086) in Japan for patients with malignant melanoma.

### (Other cited references)

[Non-patent document 31] Fukuhara, H., Ino, Y., Kuroda, T., Martuza, R.L. & Todo, T. Triple gene-deleted oncolytic herpes simplex virus vector double-armed with interleukin 18 and soluble B7-1 constructed by bacterial artificial chromosomemediated system. Cancer Res. 65, 10663-10668 (2005).
[Non-patent document 32] Toda, M., Martuza, R.L., Kojima, H. & Rabkin, S.D. In situ cancer vaccination: an IL-12 defective vector/replication-competent herpes simplex virus combination induces local and systemic antitumor activity. J. Immunol. 160, 4457-4464 (1998).
[Non-patent document 33] Lopez, C. Genetics of natural resistance to herpesvirus infections in mice. Nature 258, 152-153 (1975).
[Non-patent document 34] Caminschi, I., et al. Interleukin-12 induces an effective antitumor response in malignant mesothelioma. Am. J. Respir. Cell Mol. Biol. 19, 738-746 (1998).
[Non-patent document 35] Toda, M., Rabkin, S.D., Kojima, H. & Martuza, R.L. Herpes simplex virus as an in situ cancer vaccine for the induction of specific antitumor immunity. Hum. Gene. Ther. 10, 385-393 (1999).
[Non-patent document 36] Liu, B.L., et al. ICP34.5 deleted herpes simplex virus with enhanced oncolytic, immune stimulating, and anti-tumour properties. Gene Ther. 10, 292-303 (2003).
[Non-patent document 37] Thomas, D.L. & Fraser, N.W. HSV-1 therapy of primary tumors reduces the number of metastases in an immune-competent model of metastatic breast cancer. Mol. Ther. 8, 543-551 (2003).
[Non-patent document 38] Varghese, S., et al. Enhanced therapeutic efficacy of IL-12, but not GM-CSF, expressing oncolytic herpes simplex virus for transgenic mouse derived prostate cancers. Cancer Gene Ther. 13, 253-265 (2006).
[Non-patent document 39] Atkins, M.B., et al. Phase I evaluation of intravenous recombinant human interleukin 12 in patients with advanced malignancies. Clin. Cancer Res. 3, 409-417 (1997).
[Non-patent document 40] Cohen, J. IL-12 deaths: explanation and a puzzle. Science. 270, 908 (1995).
[Non-patent document 41] Alatrash, G., et al. Clinical and immunologic effects of subcutaneously administered interleukin-12 and interferon alfa-2b: phase I trial of patients with metastatic renal cell carcinoma or malignant melanoma. J. Clin. Oncol. 22, 2891-2900 (2004).
[Non-patent document 42] Chiocca, E.A., et al. Regulatable interleukin-12 gene therapy in patients with recurrent high-grade glioma: Results of a phase 1 trial. Sci. Transl. Med. 11(505), eaaw5680 (2019).
[Non-patent document 43] Chiocca, E.A., et al. Combined immunotherapy with controlled interleukin-12 gene therapy and immune checkpoint blockade in recurrent glioblastoma: An open-label, multi-institutional phase I trial. Neuro. Oncol. 24, 951-963 (2022).
[Non-patent document 44] Saha, D., Martuza, R.L. & Rabkin, S.D. Macrophage polarization contributes to glioblastoma eradication by combination immunovirotherapy and immune checkpoint blockade. Cancer Cell. 32, 253-267.e5 (2017).
[Non-patent document 45] Saha, D., Martuza, R.L. & Rabkin, S.D. Curing glioblastoma: oncolytic HSV-IL12 and checkpoint blockade. Oncoscience. 4, 67-69 (2017).
[Non-patent document 46] Saha, D., Martuza, R.L. & Rabkin, S.D. Temozolomide antagonizes oncolytic immunovirotherapy in glioblastoma. J. Immunother. Cancer. 8, e000345 (2020).
[Non-patent document 47] Saha, D., et al. Combinatorial effects of VEGFR kinase inhibitor axitinib and oncolytic virotherapy in mouse and human glioblastoma stemlike cell models. Clin. Cancer Res. 24, 3409-3422 (2018).
[Non-patent document 48] Zhang, W., et al. Combination of oncolytic herpes simplex viruses armed with angiostatin and IL-12 enhances antitumor efficacy in human glioblastoma models. Neoplasia. 15, 591-599 (2013).
[Non-patent document 49] Markert, J.M., et al. Preclinical evaluation of a genetically engineered herpes simplex virus expressing interleukin-12. J. Viol. 86, 5304-5313 (2012).
[Non-patent document 50] Patel, D.M., et al. Design of a Phase I Clinical Trial to Evaluate M032, a Genetically Engineered HSV-1 Expressing IL-12, in Patients with Recurrent/Progressive Glioblastoma Multiforme, Anaplastic Astrocytoma, or Gliosarcoma. Hum. Gene Ther. Clin. Dev. 27 69-78 (2016).
[Non-patent document 51] Roth, J.C., et al. Evaluation of the safety and biodistribution of M032, an attenuated herpes simplex virus type 1 expressing hIL-12, after intracerebral administration to aotus nonhuman primates. Hum. Gene Ther. Clin. Dev. 25 16-27 (2014).
[Non-patent document 52] Haghighi-Najafabadi, N., et al. Oncolytic herpes simplex virus type-1 expressing IL-12 efficiently replicates and kills human colorectal cancer cells. Microb. Pathog. 160, 105164 (2021).
[Non-patent document 53] Menotti, L., et al. HSV as A Platform for the Generation of Retargeted, Armed, and Reporter-Expressing Oncolytic Viruses. Viruses 10, 352 (2018).
[Non-patent document 54] Lioni, V., et al. A fully-virulent retargeted oncolytic HSV armed with IL-12 elicits local immunity and vaccine therapy towards distant tumors. PLos Pathog. 14, e1007209 (2018).

### [Example 2: In vivo viral replication assay]

To investigate whether T-mfIL12 with an inserted exogenous gene can have equivalent in vivo replication ability compared with T-01, T-01 or T-mfIL12 was administered to Neuro2a unilateral subcutaneous tumors of A/J mice, and the viral titers in the tumors were measured on the day of the administration and 1, 4, 7 and 11 days after the administration.

In vivo, T-01 and T-mfIL 12 showed nearly equal replication ability within Neuro2a subcutaneous tumors (Fig. 17). Both viruses showed slight increase in titer on day 1 (T-01, 1.18 x 10⁶ pfu/tumor, SEM = 5.38 x 10⁵; T-mfIL12: 3.22 x 10⁵ pfu/tumor, SEM = 1.63 x 10⁵, p=0.2020) compared with day 0 (T-01, 2.94 x 10⁴ pfu/tumor, SEM = 5.80 x 10³; T-mfIL12, 1.14 x 10⁵ pfu/tumor, SEM = 2.17 x 10⁴, p=0.0194), but then it gradually decreased as observed on day 4 (T-01, 1.83 x 10⁴ pfu/tumor, SEM = 1.77 x 10⁴; T-mfIL12, 2.30 x 10⁴ pfu/tumor, SEM = 1.17 x 10⁴, p=0.8331), day 7 (T-01, 7.51 x 10³ pfu/tumor, SEM = 5.20 x 10³; T-mfIL12, 1.26 x 10⁴ pfu/tumor, SEM = 1.14 x 10⁴, p = 0.7062), and day 11 (T-01, 0 pfu/tumor, SEM = 0.00; T-mfIL12, 1.69 x 10² pfu/tumor, SEM = 1.69 x 10², p = 0.3739). Although there was concern that IFNγ provided through IL-12 expression might suppress the activity of the virus itself and reduce its replication ability, this was not the case.

### [Example 3: Renal cancer]

### 1. In vitro cell-killing and antitumor effects of T-mfIL12 virus

In the previous study, the T-01 virus was found to have an antitumor effect against renal cancer in vivo. Therefore, T-mfIL12 virus that expresses mIL-12, which was created for the purpose of obtaining stronger anti-tumor effect by activating T cells and NK cells in vivo, was compared with the T-01 virus to examine whether the antitumor effect is enhanced. First, the cell-killing effects of the two viruses were compared in vitro using RenCa cells. As a result, in the studies of infection with either virus, most cells were killed by day 2 at an MOI of 1.0, indicating that the two viruses have comparable cell-killing effects in vitro (Fig. 18, A). Then, to RenCa subcutaneous tumor models (1.0 x 10⁵ cells/50 µl/mouse of mouse renal carcinoma RenCa cells were subcutaneously injected into both legs of BALB/c mice, the day when the maximum tumor diameter became 5 mm or greater was defined as day 0), 2 x 10⁵ pfu of the T-01 virus or T-mfIL 12 virus was intratumorally administered, and the anti-tumor effects were compared. The tumor volumes on day 9 in the mock-administered group averaged 992 mm³, and the tumor volumes on the same day 9 in the T-01 virus or T-mfIL12 virus-administered group averaged 609 mm³ and 342 mm³, respectively. There was a significant difference in tumor volume in the T-mfIL12 virus-administered group compared with the mock-administered group. In the mock-administered group, some mice died on day 11 due to hemorrhage from the tumors and wasting, but none of the mice in the T-01 virus or T-mfIL12 virus-administered groups died until day 15. On day 13, the tumor volumes in the T-01 virus and T-mfIL12 virus-administered groups averaged 974 mm³ and 445 mm³, respectively, and thus in the subcutaneous tumor model, the T-mfIL 12 virus significantly suppressed tumor growth compared with the T-01 virus (Fig. 18, B and C).

### 2. Antitumor effect of T-mfIL12 virus in distant tumors

Since it is said that, in the efficacy of HSV-1-based viral therapy of cancer, induction of systemic anti-tumor immunity is also involved in addition to the direct cell-killing effect of the virus, it was examined whether the efficacy can be obtained even in cancer lesions where the virus has not been directly administered. It was also examined whether the T-mfIL 12 virus, which is thought to more favorably utilize antitumor immunity by expressing IL-12, would enhance anti-tumor effects also in distant tumors not directly administered with the virus, compared with the T-01 virus. In the Neuro2a subcutaneous tumor models, subcutaneous tumors were created bilaterally in the mice, and 5 x 10⁵ pfu of the T-01 virus or T-mfIL12 virus was intratumorally administered into only the left tumors. In this Neuro2a bilateral subcutaneous tumor model, the mean tumor volumes of the mock, T-01 virus, and T-mfIL12 virus-administered groups on day 15 were 2838 mm³, 526 mm³, and 30 mm³, respectively, for the tumors on the treated side that directly received the virus. Significant difference tests showed that in both the T-01 virus and T-mfIL12 virus-administered groups, tumor growth was significantly suppressed compared with the mock-administered group. In the T-mfIL12 virus-administered group, tumor growth was significantly suppressed also compared with the T-01 virus-administered group (Fig. 18, D, a and c). On the other hand, the mean tumor volumes on day 15 of the contralateral tumors not directly administered with the viruses were 4037 mm³, 2443 mm³, and 1377 mm³ in the mock, T-01 virus, and T-mfIL12 virus-administered groups, respectively, indicating that tumor growth was attenuated in theT-01 virus and T-mfIL12 virus-administered group, and a significant difference was observed especially in the T-mfIL12 virus-administered group compared with the mock-administered group (Fig. 18, D, b and c).

### 3. Antitumor effect of T-mfIL12 virus in RenCa lung metastasis model

Then, the anti-tumor effects of intravenous administration of the T-01 virus and T-mfIL12 virus were compared in a RenCa lung metastasis model (5.0 x 10⁵ RenCa cells/200 µl/mouse were intravenously administered to BALB/c mice via the tail vein, the day when the cells were intravenously administered was defined as day 0). The T-01 virus or T-mfIL12 virus (5 x 10⁵ or 5 x 10⁶ pfu) was intravenously administered and the number of lung metastases was evaluated (n=10 to 12/group). Survival time was also separately evaluated by the same protocol. At the virus dose of 5 x 10⁵ pfu, the numbers of lung metastases on day 14 averaged 205, 42, and 1.9 in the mock, T-01, and T-mfIL12 virus-administered groups, respectively (Fig. 19-1, A and B). In the T-01 and T-mfIL12 virus-administered groups, the numbers of metastases significantly decreased compared with the mock-administered group. The T-mfIL 12 virus significantly reduced the number of metastases also compared with the T-01 virus. In the survival time observation experiment, the mean survival days of the mock, T-01, and T-mfIL12 virus-administered groups were 31, 39, and 46 days, respectively, at the virus dose of 5 x 10⁵ pfu, and the results of the significant difference test showed that the T-01 virus and T-mfIL12 virus significantly prolonged the survival time of mice compared with the mock, and the T-mfIL 12 virus significantly prolonged the survival time of mice also compared with the T-01 virus (Fig. 19-1, C). Further, at the virus dose of 5 x 10⁶ pfu (actual T-01 virus or T-mfIL12 virus doses were 3.4 x 10⁶ and 2.7x 10⁶ pfu), the numbers of lung metastases on day 14 averaged 326, 7.1, and 0.4 in the mock, T-01 virus and T-mfIL12 virus-administered groups, respectively. The number of metastases significantly decreased in the T-01 and T-mfIL 12 virus-administered groups compared with the mock-administered group (p=0.0045 and p=0.0024, respectively), and the T-mfIL12 virus significantly reduced the number of metastases also compared with the T-01 virus (p=0.0023). In the survival time observation experiment, the mean survival days of the mock, T-01 virus, and T-mfIL 12 virus-administered groups were 24, 42, and 55 days, respectively, indicating that the T-01 virus and T-mfIL12 virus significantly prolonged the survival time of mice compared with the mock, and the T-mfIL12 virus significantly prolonged mouse survival time compared with the T-01 virus (Fig. 19-2, A and B).

### 4. Anti-tumor effect of T-mfIL12 virus in mice after establishment of RenCa lung metastasis

In order to further examine the therapeutic effects of intravenous administrations of the viruses after lung metastasis had been established, the start of the treatment was delayed and the survival time of the mice was observed. In clinical practice, lung metastases are confirmed by X-ray examination, but it was considered difficult to create a model with exactly the same degree of progression as such metastases. Therefore, it was decided to start the treatment when at least a tumor was observed with naked eyes, and a preliminary experiment was conducted to determine the starting date of the treatment. On day 0, 2.0 x 10⁵ cells/200 µl/mouse of RenCa cells were intravenously administered, and the lungs of mice were extracted on days 3, 5, 7, and 9, stained with black ink, and examined with naked eyes whether metastasis lesions were observed on the lung surfaces (n=3/day). In this preliminary experiment, no lung metastasis lesion was observed on the surfaces of mice lungs extracted on days 3, 5, and 7. On the other hand, 16, 54, and 20 lung metastasis lesions were observed on the surfaces of mice lungs extracted on day 9, respectively. Therefore, to examine the effect of treatment in the presence of established lung metastasis, the start date of the treatment was decided to be day 9, 5 x 10⁶ pfu of the T-01 virus or T-mfIL12 virus was intravenously administered on that day, and the survival time was evaluated. When the start of the treatment was delayed to day 9, the mean survival times in the mock, T-01 virus, and T-mfIL12 virus-administered groups were 24, 26, and 28 days, respectively, and thus only the T-mfIL12 virus significantly prolonged the survival time (Fig. 19-2, C).

### 5. Evaluation of cytokine secretory activity in mouse splenocytes after intravenous administration of T-mfIL12 virus by ELISpot assay

To evaluate the in vivo immune activity provided by intravenous administration of the T-01 virus or T-mfIL12 virus in the RenCa lung metastasis model, cytokine secretory activity in the spleen was evaluated on day 14. To the RenCa lung metastasis models, the T-01 virus or T-mfIL 12 virus was intravenously administered at 5 x 10⁶ pfu on days 1, 3, and 5 as in usual treatment models, the mice were euthanized on day 14, and the spleens were aseptically extracted. The interferon γ (IFNγ) and interleukin 4 (IL-4) secretory activities of the splenocytes were evaluated by the enzyme-linked immunospot (ELISpot) method. The spleens were placed in a dish containing 5% heat-deactivated FBS-supplemented DMEM (DMEM-5), crushed and passed through a 70-µm mesh twice. The cell suspension was centrifuged at 300 RCF and 4°C for 5 minutes, the supernatant was removed, 5 ml/spleen of ACK Lysing Buffer (Lonza, MD, USA) was added, and the cells were allowed to stand at room temperature for 10 minutes. The cell suspension was centrifuged at 300 RCF and 4°C for 5 minutes, the supernatant was removed, the cells were washed twice with HL-1 supplemented with 2 mM L-glutamine and 1% penicillin/streptomycin solution (P0781, Sigma-Aldrich, MO, USA), and counted, and the cell density was adjusted to 2 x 10⁶/ml. To each well of a 96-well plate, 100 µl (2 x 10⁵ cells) of the splenocyte suspension was added, 100 µl of an activity stimulation solution was added, and incubation was performed at 37°C for 24 hours. For each group consisting of 3 animals, the measurement was independently performed 3 times by using the ELISpot kit (BD ELISPOT Mouse IFNγ ELISPT Kit, BD Biosciences, CA, USA) for mouse IFNγ (mIFNγ) and corresponding antibody for mouse IL-4 (mIL-4) (BD ELISPOT Mouse IL-4 ELISPOT Set, BD Biosciences, CA, USA). For IL-4, the incubation time was 48 hours. For antigen stimulation, there were used the RenCa cells incubated with RPMI 1640 supplemented with 0.05 mg/ml of mitomycin C (Sigma-Aldrich, MO, USA) at 37°C for 30 minutes, washed three times with DMEM-5, and diluted to 5 x 10⁶ cells/ml with HL-1. Concanavalin A (Wako Pure Chemical Industries, Osaka) was used at 2 µg/ml for positive control, while only medium was used for negative control, with which the experiments were performed simultaneously. The numbers of spots showing secretion of IFNγ under stimulation with RenCa antigen averaged 187, 211, and 234 per well in the mock, T-01 virus, and T-mfIL 12 virus-administered groups, respectively, indicating enhanced IFNγ secretion in the T-01 virus and T-mfIL 12 virus-administered groups and a significant enhancement in the T-mfIL12 virus-administered group. Significant enhancement was observed in the T-mfIL12 virus-administered group also compared with the T-01 virus-administered group. There was no significant difference in the IL-4 secretory activity in either group (Fig. 20, A). These results suggest enhancement of the activity of tumor-specific cellular immunity against RenCa cell antigen stimulation in the T-mfIL12 virus-administered group.

### 6. Evaluation of cytokine levels in mouse serum and lung tissue after intravenous administration of T-mfIL12 virus by ELISA

To evaluate cytokine levels in vivo in the RenCa lung metastasis model after intravenous administration of the T-01 virus or T-mfIL12 virus, each virus was intravenously administered once and cytokines in the serum and lung tissue were measured over time. The T-01 virus or T-mfIL12 virus was administered to the RenCa lung metastasis model once at a dose of 5 x 10⁶ pfu on day 1, and mIL-12 and mIFNγ in the serum and lung tissue were measured on days 2, 4 and 6. The serum was obtained by centrifuging collected blood at 2000 RCF for 20 minutes and collecting the supernatant. The lungs were weighed after extraction, homogenized in Eppendorf tubes containing PBS for 3 minutes using a homogenization stick, and disrupted by sonication for 1 minute, the suspension was centrifuged at 2000 RCF for 20 minutes, and the supernatant was collected and used for the evaluation. The measurement was independently performed twice for 3 animals per each group by using the mIL-12 ELISA kit (Quantikine Mouse IL-12 p70 Immunoassay R&D, MN, USA) and mIFNγ ELISA kit (Endogen Mouse IFNγ ELISA Kit, Pierce Biotechnology, IL, USA). The IL-12 level was highest in both serum and lung tissue on day 2 in the T-mfIL12 virus-administered group. On day 2, the serum IL-12 level was approximately 2.0- and 1.5-fold higher in the T-mfIL12 virus-administered group compared with the mock and T-01 virus-administered groups, respectively, and significant differences were observed. On day 4, a significant difference was observed compared with only the mock-administered group, and the level was higher by approximately 1.3-fold (Fig. 13, B, a). In the lung tissue, IL-12 level was above the sensitivity level and measured only on day 2 and day 4 in the T-mfIL12 virus-administered group (Fig. 20, B, b). As for IFNγ, the serum IFNγ level was highest on day 2 in the T-mfIL12 virus-administered group and could not be measured thereafter. In the other two groups, IFNγ was not detected at any of the measurement points (Fig. 20, B, c). The IFNγ level in the lung tissue was highest on day 2 in all the groups, and no significant difference was observed among the groups on days 2 and 4. On day 6, the IFNγ level was approximately 1.2-fold higher in the T-mfIL12 virus-administered group compared with the mock-administered group, and a significant difference was observed (Fig. 20, B, d). It was considered that, in the lung tissues, the increase in IFNγ level was caused subsequent to the increase in IL-12 level, suggesting that these cytokines are also involved in the enhancement of the in vivo anti-cancer effect of the T-mfIL12 virus.

### [Example 4-1: Bladder cancer]

### 1. T-mfIL 12 prolonged survival time of mice with orthotopic bladder cancer.

The ability of T-mfIL12 to regulate tumor cell growth in vivo was examined in an orthotopic bladder cancer model (5.0 x 10⁵ mouse bladder cancer cells, MB49, were intravesically inoculated to female C57BL/6 mice). Kaplan-Meier survival curves were generated, and log-rank tests were performed to compare the survival time distributions of animals treated with each regimen. Almost all of the mock-treated control mice died within 24 days after the tumor cell inoculation. All of the mice that died had macroscopic bladder tumors. In contrast, in the medium and high-dose (1.0 x 10⁶ and 5.0 x 10⁶ pfu) T-mfIL12-administered groups, 50% of the mice survived for 38 days or longer (Fig. 21, A, P=0.02 and P<0.01, log-rank test). However, there was no effect in the low-dose administration group (P=0.625, log-rank test). There was no statistical difference between the medium and high-dose administration groups (P=0.261, log-rank test).

### 2. T-mfIL12 prolonged survival time of mice with orthotopic bladder cancer after BCG treatment.

Further, by using the same animal model and treatment protocol, it was investigated whether the combination of BCG (1.35 mg) and T-mfIL12 (1.0 x 10⁶ pfu) is more effective compared with administration of BCG or T-mfIL12 alone. To the bladder cancer model mice, the mock was intravesically inoculated, or each or combination of BCG and T-mfIL12 was consecutively administered on days 1, 4, and 7. In all the treatment groups (BCG+mock, PBS+T-mfIL12, BCG+T-mfIL12), significant and strong antitumor effects were observed (P<0.05, log-rank test). However, Bonferroni correction revealed no anti-tumor effect in the BCG+mock group. On the other hand, the combination treatment (BCG+mock group) showed a significantly greater efficacy compared with BCG alone (BCG+mock group) or T-mfIL12 alone (PBS+T-mfIL12 group). In the combination treatment group, about 30% of the mice survived for 50 days (Fig. 21, B; P<0.01, log-rank test).

### 3. T-01 or T-mfIL12 prolonged survival time of mice with lung metastases of bladder cancer.

Since suppression of metastatic tumor growth is a major challenge in cancer therapy, the inventors of the present invention then examined whether T-01 and T-mfIL12 can show such suppression in established lung metastasis models of bladder cancer. The T-01-treated animals (P=0.04, log-rank test) or T-mfIL12-treated animals (P< 0.01, log-rank test) showed significantly prolonged survival times compared with the mock-treated animals (Fig. 21, C). The median survival times were 50, 39, and 26 days, respectively. In addition, the animals administered with T-mfIL12 showed prolonged survival time also compared with the animals administered with T-01 (P<0.05, log-rank test).

### [Example 4-2: Renal cancer, simultaneous administration of IL-2/anti-IL-2 mAb]

### 4. Simultaneous administration of IL-2/anti-IL-2 mAb complex after intratumoral injection of T-mfIL12 produces an effective anti-tumor immune response.

The efficacy of IL-2/anti-IL-2 mAb complex was tested in a mouse renal cancer model. An IL-2/anti-IL-2 mAb complex was prepared by incubating 2 µg of mouse IL-2 with 1 mg of anti-IL-2 mAb (S4B6-1) at room temperature for 10 minutes. Tumor growth was more inhibited by the IL-2/anti-IL-2 mAb complex compared with anti-IL2 mAb (P<0.05, days 18 to 23) or control treatment (P<0.01, days 12 to 23) (Fig. 22, A). It was decided to examine the effect of combination treatment of T-mfIL12 and IL-2/anti-IL-2 mAb complex by using the same model. The treatment with T-mfIL12 alone or IL-2/anti-IL-2 mAb complex alone was superior to mock+IgG, but the combination treatment with T-mfIL 12 and IL-2/anti-IL-2 mAb complex resulted in significant improvement over T-mfIL 12 alone or IL-2/anti-IL-2 mAb complex alone in tumor control (Fig. 22, B). In subcutaneous tumors treated with simultaneous intratumoral injection of T-mfIL12 and intraperitoneal injection of IL-2/anti-IL-2 mAb complex, infiltration of many CD4⁺ and CD8⁺ T lymphocytes was observed. The combination treatment group showed reduced cellularity and areas of necrosis. Immunohistochemistry of tumors treated with the combination therapy also showed increased CD4⁺ (blue dots) and CD8⁺ (blue dots) T lymphocyte infiltration (Fig. 22, C).

### 5. Combination treatment with T-mfIL12 and IL-2/anti-IL-2 mAb complex reduces tumor metastasis.

It was investigated whether T-mfIL 12 prevents tumor progression in the mouse RenCa lung metastasis model. As a result, the mean number of lung surface nodules in mock-treated control was significantly greater than that of 2.0 x 10⁵ or 1.0 x 10⁶ pfu T-mfIL12-treated animals (Figs. 23, A and B, P<0.05 and P<0.01, respectively). In a part of 1.0 x 10⁶ pfu T-mfIL12-treated animals, surface nodules were not seen.

Simultaneous administration of T-mfIL12 and IL-2/anti-IL-2 mAb complex produced an additive or synergistic effect. Therefore, the inventors of the present invention investigated whether this combination treatment produces additive or synergistic effect to arrest tumor progression in a mouse RenCa lung metastasis model. The results showed that the combination of T-mfIL12 and IL-2/anti-IL-2 mAb complex was effective and significantly reduced the number of models of BALB/c mice bearing RenCa cells in the lung, while T-mfIL 12 alone or IL-2/anti-IL-2 mAb complex alone slightly reduced nodule numbers (Figs. 23, C and D). These results clearly demonstrate that treatment consisting of the concurrent administration of T-mfIL12 and IL-2/anti-IL-2 mAb complex has an effective protective effect in the lung RenCa cell tumor burden model.

Further, survival experiments were performed in a RenCa cell mouse model. Survival rate was significantly improved in the combination-treated animals. That is, the median survival time of the control animals treated with mock and IgG was 18 days, with 100% death by day 24. In contrast, with the combination treatment, T-mfIL12 alone or IL-2/anti-IL-2 mAb complex alone, the median survival times were 49, 38 and 25 days, respectively (log-rank test, P< 0.001) (Fig. 23, E). Analysis of liver and kidney tissue specimens showed no histological damage in all the treatment groups.

### [Example 5: Intravenous administration]

### 1. Intravenous administration of HSV-1 inhibited the growth of Neuro2a subcutaneous tumors.

To evaluate the therapeutic effect of intravenous administration of oncolytic viruses, the antitumor effects of T-01 and T-mfIL 12 were tested in A/J mice harboring Neuro2a subcutaneous tumors. When subcutaneous tumors reached 5 mm in diameter (4 days after tumor cell inoculation), the mice were administered with extract for mock-infection, T-01 or T-mfIL12 (5 x 10⁶ pfu) by tail vein injection on days 0, 2, and 4. Intravenous administration of T-01 on day 14 significantly inhibited tumor growth (Aver. 4000 mm³) compared with the mock treatment (6000 mm³, p<0.001, SNK). Furthermore, intravenous administration of T-mfIL 12 enhanced the antitumor effect (Aver. 2100 mm³) compared with T-01 (p<0.001, SNK). The antitumor effect was observed from the 7th day, and the p values were less than 0.001 among all the three groups. These results demonstrated that intravenous administration of the oncolytic HSV-1 has a significant antitumor effect, which is further enhanced by IL-12 (Fig. 24, A). The mice were weighed three times a week. Except for temporary slight weight loss in the T-mfIL12-treated group compared with the mock-treated group on day 2 (p<0.05), no clear weight loss was induced by the intravenous administration therapy (Fig. 24, B). There were no clinical side effects such as apparent systemic or neurological toxicity that may be attributable to the intravenous administration of the virus.

### 2. HSV-1 was delivered to subcutaneous tumors by intravenous administration.

In order to investigate distribution of the virus after tail vein injection, viral DNA was quantified in subcutaneous tumor and normal tissues. The mice received administration three times on days 0, 2 and 4. On day 5, mice were sacrificed, the tumor, liver, spleen, lung, kidney and brain were collected (n=5), and the amounts of the virus were quantified by real-time PCR. The results are presented in Fig. 24, C. The amounts of viral DNA detected in the lung, liver, spleen, kidney and brain were 307 pfu/mg, 111 pfu/mg, 297 pfu/mg, 9 pfu/mg and 7 pfu/mg, respectively. The average amount of viral DNA detected in the subcutaneous tumor was 1140 pfu/mg, much larger than in normal tissues. This indicated that the oncolytic virus could be delivered into subcutaneous tumors by intravenous administration.

### 3. Intravenous administration of T-mfIL 12 prolonged survival time of mice with intracranial tumors.

To determine whether intravenous administration of oncolytic virus has a therapeutic effect on brain tumors, A/J mice bearing intracranial (i.c.) Neuro2a tumors were administered with oncolytic virus by tail vein injection on days 5, 7, and 9 (Fig. 25, A). For the intracranial tumor model animal, A/J mice and Neuro2a (neuroblastoma) cells were used. No significant differences were observed between the T-01-administered group (average, 18 days) and T-mfIL12-administered group (average, 19 days), but the mice treated with intravenous administration of T-mfIL12 showed significantly prolonged survival time (p<0.05, Breslow-Gehan-Wilcoxon) compared with mice administered with the mock (average 14 days, p<0.05).

### 4. Virus therapy by intravenous administration showed antitumor effect on systemic metastatic tumors.

The antitumor effect of intravenous administration on Neuro2a metastatic tumors was investigated. The viruses were injected into the tail vein on days 1, 4, and 7 (Fig. 25, B). The mock-administered control animals showed a median survival time of 27.1 days and 100% mortality on day 33. In contrast, the mice administered with T-01 showed prolonged survival time compared with the mock-administered group (p<0.05, Breslow-Gehan-Wilcoxon), and significantly longer survival times were observed in the T-mfIL12-administered group compared with the T-01 and mock-administered groups (p<0.05 and p<0.001, respectively, Breslow-Gehan-Wilcoxon).

The mice were treated with the same doses on days 1, 3, and 5 (treatment schedule 2). As shown in Fig. 25, C, the median survival times of the mock, T-01, and T-mfIL12-treated groups were 35.9, 69.8, and 123.9 days, respectively. A significant increase in average survival time was observed in the mice treated with T-mfIL12 compared with the mock-administered mice (p>0.05, Breslow-Gehan-Wilcoxon). Despite that no significant difference was observed in mice treated with T-01 compared with the mock or T-mfIL12-treated groups (p>0.05, Breslow-Gehan-Wilcoxon), 20% (2/10) of the T-01-administered mice and 60% (6/10) of the T-mfIL12-administered mice showed remarkable survival times of more than 180 days. All the long-term survivors remained completely disease-free, indicating that oncolytic virus by intravenous administration for these animals was effective for potential tumor dissemination.

In the study of the inventors of the present invention, mice that died of Neuro2a metastatic tumors were autopsied, and metastases were found in various organs, such as the lung, liver, kidney, ovary, lymph node, intestine and muscle. The number and distribution of metastases significantly varied among animals. As a result, it was thought that survival time was the best evaluation criterion for therapeutic effect in this experiment.

### 5. T cells play an important role in the anti-tumor immune response induced by T-mfIL12.

To investigate the involvement of T cells in the anti-tumor immune response induced by T-mfIL12, treatment experiments were performed by using athymic mice, in which T lymphocytes were absent. The athymic mice were treated by tail vein injection on days 1, 3, 5 after intravenous Neuro2a cell inoculation. As shown in Fig. 25, D, there was no difference in survival time between the T-01 and T-mfIL12-administered mice (p>0.05, Breslow-Gehan-Wilcoxon); however, there was a significant difference in survival time between the T-mlIL12-administered mice (average, 48.8 days) and mock-administered mice (average, 33.8 days, p<0.005, Breslow-Gehan-Wilcoxon). Prolonged survival times were also observed in the mice administered with T-01 (average, 53.3 days) compared with the mock-administered mice (p<0.05, Breslow-Gehan-Wilcoxon). One mouse (1/10) administered with T-01 survived for more than 180 days. Enhanced antitumor effect was not found in T-mfIL12-administered mice in comparison with T-01-administered mice. The data suggest that T cells are involved in the antitumor immune response induced by T-mfIL12.

### 6. IL-12 induced significant IFNγ secretion by intravenous administration.

Serum IL-12 and IFNγ levels in A/J mice Neuro2a metastatic tumor models were examined (Fig. 26, A). The mice were treated three times on days 1, 3, 5 by intravenous administration after tumor cell intravenous inoculation. On days 2, 4, 6, blood was collected and assayed for IL-12 and IFNγ concentrations (n=3 for each time point). On day 2, the day after the first intravenous administration, the level of IL-12 detected was 518 pg/ml in the T-mfIL12-administered mice, followed by a rapid decline. On days 4 and 6, IL-12 expression in serum was undetectable (0 pg/ml). In contrast, the mock and T-01 administrations resulted in undetectable levels of IL-12 in serum at all time points.

As shown in Fig. 26B, in the T-mfIL12-administered group, the IFNγ level was 3989 pg/ml on day 2, followed by a decline. On day 4, the level of IFNγ was 868 pg/ml and on day 5, it became 711 pg/ml. In the T-01-administered mice, it was 174 pg/ml on day 1, 300 pg/ml on day 4 and 262 pg/ml on day 6. In the mock-administered mice, IFNγ level was 115 pg/ml, 58 pg/ml, and 148 pg/ml at those time points, respectively. There was significant difference in the IFN-γ expression on day 6 among the three groups (T-mfIl12 vs T-01, p=0.019; T-01 vs mock, p=0.030; T-mfIL12 vs mock, p=0.007; t-test).

### [Example 6: Malignant melanoma]

### 1. Subcutaneous tumor treatment experiment using DBA/2 mouse bilateral subcutaneous tumor model

A bilateral subcutaneous tumor model was created (1.0 x 10⁶ cells of mouse malignant melanoma cell clone M3/50 µL DMEM/animal were subcutaneously injected into the right and left lateral flanks of congenic DBA/2 mice), and treatment experiments were conducted with mock, T-01 or T-mfIL12 (4 x 10⁴ pfu/20 µL). In these experiments, viral treatment was given only to subcutaneous tumor of one side (left side) of the bilateral subcutaneous tumors in the hope that IL-12 produced by T-mfIL12 would enhance anti-tumor immunity and thus exhibit an anti-tumor effect on the distant tumor. Statistical analysis of the results was performed by Student's t-test.

On the administered side, T-mfIL12 significantly inhibited the increase of subcutaneous tumors on the treated side compared with the mock (day 21, p<0.05; day 25, p<0.05, Fig. 27, A). T-mfIL12 significantly inhibited subcutaneous tumor growth on the treated side also compared with T-01 (day 17, p<0.01; day 21, p<0.05). Furthermore, on the untreated side, T-mfIL12 provided a significant difference compared with the mock (day 17, p<0.05; day 21, p<0.01, Fig. 27, B). Significant differences were also observed between T-mfIL12 and T-01 (day 21, p<0.05). No significant difference was observed between the mock and T-01 in either the administered or non-administered side. These results indicate that T-mfIL12 has a significant antitumor effect on Clone M3 subcutaneous tumors compared with the mock not only on the administered side but also on the non-administered side. Furthermore, T-mfIL 12 showed a significant antitumor effect in vivo on the administered side compared with T-01, despite the comparable in vitro cell killing effect and viral replication ability, suggesting that the enhanced antitumor effect may involve immune activation by IL-12 expression.

### 2. Observation of tumor-infiltrating lymphocytes by immunohistochemical staining

To evaluate the activation of anti-tumor immunity within tumors, tumor-infiltrating lymphocyte CD4⁺ and CD8⁺ cells in the subcutaneous tumor on the administered side of the DBA/2 mouse Clone M3 bilateral subcutaneous tumor model were observed by immunohistochemical staining. A subcutaneous tumor removed on day 14 after the virus administration was divided into three pieces and one of them was used in the experiment. Cross-sections of the subcutaneous tumor were observed at three positions, on the skin side (outer side), at the center, and on the body side (inner side). The negative control was a section treated with 2% BSA/PBS for the primary antibody reaction, and it was confirmed that there was no nonspecific reaction with the secondary antibody (Fig. 28, A).

CD4⁺ and CD8⁺ cells were found mainly along the tumor-skin borders and vascular contours (Figs. 28, B and C), and an increase in CD4⁺ cells was observed in the T-mfIl12-administered group compared with the mock or T-01-administered groups at all the positions, on the skin side, at the center and on the body side, of the subcutaneous tumor.

### 3. Treatment experiment using a nude mouse bilateral subcutaneous tumor model

To investigate whether the enhanced anti-tumor effect observed in the T-mfIL12-administered group of the DBA/2 mouse subcutaneous tumor model shown above was due to the immunity enhancing effect of mouse IL-12 produced by T-mfIL12, nude mice bilateral subcutaneous tumor models (Clone M3 1.0 x 10⁶ cells/50 µ L DMEM/animal were subcutaneously injected into the right and left lateral flanks of nude mice) were used to conduct a subcutaneous tumor treatment experiment.

On the administered side, T-mfIL12 significantly inhibited subcutaneous tumor growth compared with the mock (day 15, p<0.01; day 18, p<0.01; Fig. 29, A). T-mfIL 12 significantly inhibited subcutaneous tumor growth also compared with T-01 (day 15, p<0.01; day 18, p<0.01). However, there was no significant difference in subcutaneous tumors on the non-administered side between the groups, which results were different from those of the experiment using DBA/2 mice, where tumor-shrinking effect was observed on the non-administered side (Fig. 29, B).

These results indicate that T cells are required for enhancing the anti-tumor effect of mouse IL-12 expression by T-mfIL12, on distant tumors.

### 4. Analysis of IFNγ- or IL-4-producing lymphocytes by ELISpot method

On the basis of the results of the aforementioned immunohistochemical staining and treatment experiment with a nude mouse subcutaneous tumor model, it was found that T cells are important for enhancing the anti-tumor effect of T-mfIL 12. Therefore, to evaluate the enhancement of anti-tumor immune activity by T-mfIL12 in the DBA/2 mouse Clone M3 subcutaneous tumor model, IFNγ-producing and IL-4-producing lymphocytes were analyzed by ELISpot method.

Since the results for the administered side of the subcutaneous tumor treatment experiment (Fig. 10, A) showed tumor-shrinking effect in the T-mfIL12-administered group on days 13 to 17, it was considered that anti-tumor immunity might be enhanced during this period, and day 14 was chosen as the measurement date. Statistical analysis of the results was performed by Student's t-test.

The T-mfIL12-administered group showed a significant increase in IFNγ-producing lymphocytes that responded to stimulation by Clone M3 cells compared with the mock and T-01-administered groups (T-mfIL12-mock, p<0.01; T-mfIL12-T-01, p<0.05; Fig. 30, A). On the other hand, there was no significant difference in the number of IL-4-producing lymphocyte spots between the T-mfIL12 and T-01-administered groups (Fig. 30, B).

### [Example 7: Non-seminomatous testicular germ cell tumor (NSGCT)]

### Anti-tumor effects of T-01 and T-mfIL12 in F9 model

The F9 model mice (5.0 x 10⁵ of mouse NSGCT cells F9 in 50 µL DMEM medium and 50 µL BD Matrigel, total 100 µL, were subcutaneously injected to 129 male mice on the dorsal side) were selected and divided into two groups of 4.0 x 10⁴ pfu T-01-administration group and 4.0 x 10⁴ pfu T-mfIL12-administration group, and mock group not treated with T-01, total three groups (8 animals for each group). Intratumoral administration of 20 µL of solution containing T-01, T-mfIL12, or mock was performed twice, on day 0 and day 3. The tumor diameter in each individual was measured twice a week, and this system was terminated on day 14, when the maximum diameter of the tumors of the individuals in the mock group exceeded 23 mm. On day 9 and thereafter, both the T-01 and T-mfIL12-administered groups showed significant anti-tumor effects compared with the mock-administered group, and the T-mfIL12-administered group showed significant tumor-suppressing effect also compared with the T-01-administered group. In the T-mfIL12-administered group, tumors were suppressed to the same sizes as those observed at the start of the treatment in all the individuals, showing a strong tumor-suppressing effect (Fig. 31; *, P<0.01; †, P<0.05).

### [Sequences listed in Sequence Listing]

SEQ ID NO: 1, Nucleotide sequence of the insertion nucleotides of T-mfIL 12
SEQ ID NO: 2, Amino acid sequence encoded by the nucleotide sequence of the insertion nucleotides of T-mfIL12
SEQ ID NO: 3, Nucleotide sequence of the insertion nucleotides of T-hIL12
SEQ ID NO: 4, Amino acid sequence encoded by the nucleotide sequence of the insertion nucleotides of T-hIL12
SEQ ID NO: 5, Primer
SEQ ID NO: 6, Primer
SEQ ID NO: 7, Nucleotide sequence of the fusion polypeptide insertion site and upstream and downstream thereof
SEQ ID NO: 8, Nucleotide sequence of the γ34.5 gene deletion site and upstream and downstream thereof (in the TR_{L} region)
SEQ ID NO: 9, Nucleotide sequence of the α47 gene deletion site and upstream and downstream thereof
SEQ ID NO: 10, Primer
SEQ ID NO: 11, Primer
SEQ ID NO: 12, Primer
SEQ ID NO: 13, Primer

## Claims

1. A pharmaceutical composition comprising a recombinant herpes simplex virus (HSV) with interleukin 12 (IL-12) expression, the recombinant HSV comprising a gene encoding a fusion polypeptide in which the 35 kDa light chain (p35) and the 40 kDa heavy chain (p40) of IL-12 are linked via two or more elastin motifs, and having the following characteristics (a) to (c):
(a) the ICP6 gene is deleted or inactivated,
(b) the γ34.5 gene is deleted or inactivated, and
(c) the α47 gene is deleted or inactivated.

2. The pharmaceutical composition according to claim 1, wherein the fusion polypeptide comprises p40, two or more elastin motifs, and p35 linked in this order from the N-terminal side.

3. The pharmaceutical composition according to claim 1, wherein the gene encoding the fusion polypeptide is inserted into the ICP6 locus.

4. The pharmaceutical composition according to claim 1, wherein the gene encoding the fusion peptide is any of the following polynucleotides:
(i) a polynucleotide consisting of the sequence of SEQ ID NO: 1;
(ii) a polynucleotide consisting of a sequence having a sequence identity of 90% or higher to the sequence of SEQ ID NO: 1 and encoding a polypeptide capable of forming an active IL-12;
(iii) a polynucleotide encoding a polypeptide consisting of the sequence of SEQ ID NO: 2;
(iv) a polynucleotide encoding a polypeptide consisting of a sequence having a sequence identity of 90% or higher to the sequence of SEQ ID NO: 2 and capable of forming an active IL-12;
(v) a polynucleotide consisting of the sequence of SEQ ID NO: 3;
(vi) a polynucleotide consisting of a sequence having a sequence identity of 90% or higher to the sequence of SEQ ID NO: 3 and encoding a polypeptide capable of forming an active IL-12;
(vii) a polynucleotide encoding a polypeptide consisting of the sequence of SEQ ID NO: 4; and
(viii) a polynucleotide encoding a polypeptide consisting of a sequence having a sequence identity of 90% or higher to the sequence of SEQ ID NO: 4 and capable of forming an active IL-12.

5. The pharmaceutical composition according to claim 1, wherein the recombinant HSV is derived from G47Δ.

6. The pharmaceutical composition according to any one of claims 1 to 5, which is used for enhancing the effect of IL-12 in a treatment of a tumor.

7. The pharmaceutical composition according to claim 5, which is for local injection.

8. A method for enhancing the effect of IL-12 in a treatment of a tumor, the method comprising
administering the pharmaceutical composition comprising a recombinant HSV with IL-12 expression according to any one of claims 1 to 5 to a subject in need of the enhancement.

9. A pharmaceutical composition comprising a recombinant HSV with IL-12 expression, the recombinant HSV comprising a gene encoding IL-12, and having the following characteristics (a) to (c), which induces IFNγ production through IL-12 expression without decreasing the replication ability of the recombinant HSV:
(a) the ICP6 gene is deleted or inactivated,
(b) the γ34.5 gene is deleted or inactivated, and
(c) the α47 gene is deleted or inactivated.

10. A pharmaceutical composition for intravenous administration, comprising a recombinant HSV with IL-12 expression, the recombinant HSV comprising a gene encoding IL-12, and having the following characteristics (a) to (c):
(a) the ICP6 gene is deleted or inactivated,
(b) the γ34.5 gene is deleted or inactivated, and
(c) the α47 gene is deleted or inactivated.

11. A pharmaceutical composition for intratumoral administration, comprising a recombinant HSV with IL-12 expression, the recombinant HSV comprising a gene encoding IL-12, and the following characteristics (a) to (c), which elicits systemic antitumor immunity, and thereby attains either the suppression of metastasis or the suppression of growth of a tumor not receiving direct intratumoral administration, or both:
(a) the ICP6 gene is deleted or inactivated,
(b) the γ34.5 gene is deleted or inactivated, and
(c) the α47 gene is deleted or inactivated.

12. A pharmaceutical composition for treating a tumor requiring increased infiltration of immune cells comprising a recombinant HSV with IL-12 expression, the recombinant HSV comprising a gene encoding IL-12, and having the following characteristics (a) to (c):
(a) the ICP6 gene is deleted or inactivated,
(b) the γ34.5 gene is deleted or inactivated, and
(c) the α47 gene is deleted or inactivated.

13. The pharmaceutical composition according to any one of claims 9 to 12,
wherein the recombinant HSV is the recombinant HSV defined in any one of claims 1 to 5.
